(19) Europäisches Patentamt
European Patent Office
Office européen des brevets

(11) EP 1 445 250 A1

(12) **EUROPEAN PATENT APPLICATION**
published in accordance with Art. 158(3) EPC

(43) Date of publication:
**11.08.2004 Bulletin 2004/33**

(21) Application number: **02755745.3**

(22) Date of filing: **01.08.2002**

(51) Int Cl.7: **C07C 211/30**

(86) International application number:
**PCT/JP2002/007843**

(87) International publication number:
**WO 2003/011812 (13.02.2003 Gazette 2003/07)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **01.08.2001 JP 2001233818**

(71) Applicant: **MOCHIDA PHARMACEUTICAL CO., LTD.**
**Shinjuku-ku Tokyo 160-8515 (JP)**

(72) Inventors:
- **KATO, Yutaka,**
**c/o MOCHIDA PHARMACEUTICAL CO., LTD.**
**Tokyo 160-8515 (JP)**
- **MIYAZAKI, Yutaka,**
**MOCHIDA PHARMACEUTICAL CO., LTD.**
**Tokyo 160-8515 (JP)**
- **SHIMADA, Hiroyasu,**
**MOCHIDA PHARMACEUTICAL CO. LTD.**
**Tokyo 160-8515 (JP)**
- **MANABE, Tadashi,**
**MOCHIDA PHARMACEUTICAL CO., LTD.**
**Tokyo 160-8515 (JP)**
- **SHIROMIZU, Ikuya,**
**MOCHIDA PHARMACEUTICAL CO., LTD.**
**tokyo 160-8515 (JP)**
- **OKAMOTO, Atsushi**
**Takatsuki-shi, Osaka 569-1126 (JP)**

(74) Representative: **Grünecker, Kinkeldey,**
**Stockmair & Schwanhäusser Anwaltssozietät**
**Maximilianstrasse 58**
**80538 München (DE)**

(54) **NOVEL AMINE DERIVATIVE HAVING HUMAN BETA-TRYPTASE INHIBITORY ACTIVITY AND DRUGS CONTAINING THE SAME**

(57) It is intended to provide a novel low-molecular weight amine derivative, that is absorbed well, has low toxicity, and has an excellent human β-tryptase inhibitory activity with extremely high selectivity, a pharmaceutically acceptable salt thereof, and a medicine containing the same as an active ingredient. The medicine of the present invention is efficacious as a prophylactic/therapeutic agent for diseases in the crisis and evolution of which are considered to be attributed to β-tryptase, for example, respiratory diseases, allergic diseases, inflammatory bowel diseases, hyperprolliferative skin diseases, vascular edema and rheumatoid arthritis.

EP 1 445 250 A1

## Description

Technical Field

The present invention relates to a nonpeptidic and low-molecular amine derivative having a human mast cell β-tryptase selective inhibiting function and a medicine containing the derivative as an active ingredient.

Background Art

Tryptase (EC 3. 4. 21. 59) is a proteinase similarly to trypsin, thrombin, Factor Xa (FXa), and kalliklein. Heretofore, tryptase c DNAs' such as: α I and II; β I, II, and III; γ I and II; δ I; and ε have been known. Of those, α-tryptase and β-tryptase specifically localize in granules of mast cells or of basophils. Although α-tryptase and β-tryptase have an amino acid sequence homology of 93%, there is a point of difference between α-tryptase and β-tryptase in that α-tryptase is constantly secreted from a mast cell, whereas β-tryptase is specifically secreted outside the cell by degranulation of the mast cell (Schwartz et al: Journal of Clinical Investigation (J. Clin. Invest.), vol. 96, pp. 2702 to 2710 (1995)).

β-tryptase has characteristics largely different from those of other serine proteinases. For example, the sensitivity to various serine protease inhibitors of β-tryptase is remarkably different from those of other serine proteinases (Schwartz et al: Journal of Immunology (J. Immunol.), vol. 126, pp. 1290 to 1294 (1981)). Furthermore, for example, the enzyme activity reveals by the formation of a tetramer (Schwartz et al: Journal of Biological Chemistry (J. Biol. Chem.), vol. 261, pp. 7372 to 7379 (1986); Pereira et al: Nature, vol. 392, pp. 306 to 311 (1998)).

The relationship of tryptase with many pathological states has also been suggested. For example, in a patient suffering from a systematic anaphylactic shock such as an allergy to bee venoms, the β-tryptase content in the blood increases continuously (Schwartz et al: New England Journal of Medicine (N. Engl. J. Med.), vol. 316, pp. 1622 to 1626 (1987)). Furthermore, for example, the inhalation of allergen in patients suffering from atopic asthma increases the content of β-tryptase in their blonchoalveolar fluid (Bousquet et al: Journal of Allergy and Clinical Immunology (J. Allergy Clin. Immunol.), vol. 88, pp. 649 to 660 (1991)). That is, there have been many reports showing that β-tryptase level increase at the site of allergen exposure in such allergic diseases. Proteinase activated receptor-2 (PAR-2), complement C3, Vasoactive intestinal peptide (VIP), calcitonin gene-related peptide (CGRP), and the like have been reported as natural substrates of tryptase. In addition, it has been suggested that protein processing action of those substrates leads to airway contraction, increase of vascular permiability, activation of airway epithelial cells or fibroblasts, or the like. Furthermore, β-tryptase is specifically secreted outside a cell by the degranulation of mast cells, and, in the granule of the mast cells, β-tryptase localizes with trypstatin that is a natural inhibitor of β-tryptase and that is a basic protein (Katsunuma et al: Biochemistry, vol. 62, pp. 18 to 31 (1990)). Therefore, it appears that β-tryptase specifically induces airway contraction and airway inflammation owing to the degranulation of mast cells, and does not act on a living body unless the mast cells are degranulated. From the above, it is conjectured that a substance inhibiting human β-tryptase is useful in a treatment for the disease due to the mast cell activation, in particular, an allergic disease such as bronchial asthma.

As examples of a currently known inhibitor for human β-tryptase, trypstatin (Kido et al: Archives of Biochemistry and Biophysics (Arch. Biochem. Biophys), vol. 239, pp. 436 to 443 (1985)) and leech derived tryptase inhibitor (LDTI) (Sommerhoff et al: Biological Chemistry Hoppe-Seyler (Biol. Chem. Hoppe-Seyler), vol. 375, pp. 685 to 694 (1994)) can be given as natural inhibitors, and several of peptide derivatives, nonpeptidic compounds having amidino groups (aminoiminomethyl groups) and guanidino groups (aminoiminomethylamino groups), and the like have been reported as synthetic compounds.

For instance, US 5,525,623 and EP 504,064 B each disclose a peptide analog having a guanidino group, WO 99/24407 discloses a compound having an amidino group, and WO 99/55661 discloses a compound having an aminomethyl group. However, each of those compounds involves a problem to be solved in that: the selectivity of β-tryptase inhibition is not high; or the presence of an amidino group or a guanidino group is likely to cause such a compound to be poorly absorbed or to exhibit toxicity.

Disclosure of the Invention

It is an object of the present invention to provide a novel low-molecular amine derivative that is absorbed well, has low toxicity, and has an excellent β-tryptase inhibitory activity with extremely high selectivity.

It is another object of the present invention to provide a medicine containing the derivative as an active ingredient.

The inventors of the present invention have made extensive studies to solve the above problems. As a result, the inventors of the present invention have found a novel low-molecular amine derivative having an excellent β-tryptase

inhibitory activity with extremely high selectivity, thereby achieving the present invention.

Hereinafter, the present invention is described in detail. However, the present invention is not limited to the statement below.

According to a first aspect of the present invention, there is provided an amine derivative represented by the following formula (I) or a pharmaceutically acceptable salt thereof:

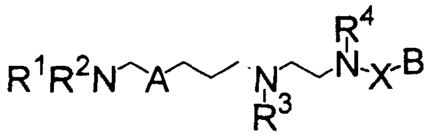

(I)

In the formula (I), $R^1$, $R^2$, and $R^3$ each independently is hydrogen atom, a lower alkyl group, a lower alkenyl group, a lower alkynyl group, or a lower acyl group; $R^1$ and $R^2$ may be bonded to each other to form one of a 5-membered ring and a 6-membered ring each containing nitrogen atom to which $R^1$ and $R^2$ are bonded; and 1 to 5 hydrogen atoms in each of the lower alkyl group, the lower alkenyl group, the lower alkynyl group, and the lower acyl group may be substituted by a hydroxyl group, an amino group, a carboxyl group, a lower alkoxyl group, or a lower alkoxycarbonyl group.

$R^4$ is hydrogen atom or a lower alkyl group.

A is an aromatic ring which is a 5-membered aromatic ring or 6-membered aromatic ring, wherein the aromatic ring may contain 1 to 4 hetero atoms selected from the group consisting of nitrogen atom, sulfur atom, and oxygen atom, and A may be substituted;

B is a saturated or unsaturated hydrocarbon group which is a 5-to-7-membered monocyclic hydrocarbon group or 6-to-12-membered condensed bicyclic hydrocarbon group, wherein the hydrocarbon group may contain 1 to 3 hetero atoms selected from the group consisting of nitrogen atom, sulfur atom, and oxygen atom, and each of these groups may be substituted with any of substituents excluding an amino group, an amidino group, and a guanidino group, with the proviso that B is a benzene ring the substituent is any of substituents excluding a carboxyl group, an alkoxycarbonyl group, and a carbamoyl group; and

X is a carbonyl group (-CO-), a sulfonyl group ($-SO_2-$), a methylene group ($-CH_2-$), a vinylenecarbonyl group (-CO-CH=CH-), a vinylenesulfonyl group ($-SO_2-CH=CH-$), an oxymethylenecarbonyl group ($-CO-CH_2O-$), or an oxymethylenesulfonyl group ($-SO_2-CH_2O-$).

According to a second aspect of the present invention, there is provided a pharmaceutical composition characterized by containing the amine derivative represented by the formula (I) or the pharmaceutically acceptable salt thereof as an active ingredient.

According to a third aspect of the present invention, there is provided a β-tryptase inhibitor characterized by containing the amine derivative represented by the formula (I) or the pharmaceutically acceptable salt thereof as an active ingredient.

According to a fourth aspect of the present invention, there is provided an agent to prevent and/or treat for an allergic disease, characterized by containing the amine derivative represented by the formula (I) or the pharmaceutically acceptable salt thereof as an active ingredient.

According to a fifth aspect of the present invention, there is provided a method to prevent and/or treat for a disease the crisis and evolution of which are considered to be attributed to β-tryptase, particularly for an allergic disease using the pharmaceutical composition containing the amine derivative represented by the formula (I) or the pharmaceutically acceptable salt thereof as an active ingredient.

In the second to fifth aspects, a preferable aspect of an amine derivative to be contained is in conformance with a preferable aspect of the first aspect.

Best Mode for carrying out the Invention

Description is made of a compound of the present invention represented by the following formula (I):

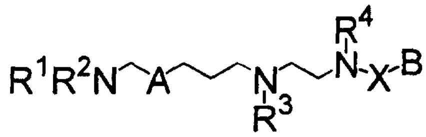

(I)

In the formula (I), $R^1$, $R^2$, and $R^3$ each independently is hydrogen atom, a lower alkyl group, a lower alkenyl group, a lower alkynyl group, or a lower acyl group; $R^1$ and $R^2$ may be bonded to each other to form one of a 5-membered ring and a 6-membered ring each containing nitrogen atom to which $R^1$ and $R^2$ are bonded; and 1 to 5 hydrogen atoms in each of the lower alkyl group, the lower alkenyl group, the lower alkynyl group, and the lower acyl group may be substituted by a hydroxyl group, an amino group, a carboxyl group, a lower alkoxyl group, or a lower alkoxycarbonyl group.

$R^4$ is hydrogen atom or a lower alkyl group.

A is an aromatic ring which is a 5-membered aromatic ring or 6-membered aromatic ring, wherein the aromatic ring may contain 1 to 4 hetero atoms selected from the group consisting of nitrogen atom, sulfur atom, and oxygen atom, and A may be substituted;

B is a saturated or unsaturated hydrocarbon group which is a 5-to-7-membered monocyclic hydrocarbon group or 6-to-12-membered condensed bicyclic hydrocarbon group, wherein the hydrocarbon group may contain 1 to 3 hetero atoms selected from the group consisting of nitrogen atom, sulfur atom, and oxygen atom, and each of these groups may be substituted with any of substituents excluding an amino group, an amidino group, and a guanidino group, with the proviso that B is a benzene ring the substituent is any of substituents excluding a carboxyl group, an alkoxycarbonyl group, and a carbamoyl group; and

X is a carbonyl group (-CO-), a sulfonyl group ($-SO_2-$), a methylene group ($-CH_2-$), a vinylenecarbonyl group (-CO-CH=CH-), a vinylenesulfonyl group ($-SO_2-CH=CH-$), an oxymethylenecarbonyl group ($-CO-CH_2O-$), or an oxymethylenesulfonyl group ($-SO_2-CH_2O-$).

The term "lower" in the substituent such as 'lower alkyl group' refers to a straight-chain or branched-chain compound having 1 to 4 carbon atoms.

$R^1$, and $R^2$ each independently is hydrogen atom, a lower alkyl group, a lower alkenyl group, a lower alkynyl group, or a lower acyl group. Preferably, each of $R^1$, and $R^2$ is hydrogen atom.

Examples of the lower alkyl group (having 1 to 4 carbon atoms) include methyl group, ethyl group, n-propyl group, isopropyl group, n-butyl group, isobutyl group, sec-butyl group, and tert-butyl group. Examples of the lower alkenyl group (having 2 to 4 carbon atoms) include a vinyl group, a 2-propenyl group, a 2-butenyl group, and a 3-butenyl group. Examples of the lower alkynyl group (having 2 to 4 carbon atoms) include a 2-propynyl group. Examples of the lower acyl group (having 1 to 4 carbon atoms) include a formyl group, an acetyl group, a propanoyl group, a butanoyl group, and an isobutanoyl group.

Furthermore, $R^1$ and $R^2$ may be bonded to each other to form a saturated 5- or 6-membered ring containing nitrogen atom to which $R^1$ and $R^2$ are bonded. Examples of such a ring include pyrrolidine, piperidine, 2-pyrrolidinone, 2-piperidine, and succinimide.

Furthermore, 1 to 5 hydrogen atoms in each of the lower alkyl group, the lower alkenyl group, the lower alkynyl group, and the lower acyl group may be substituted by hydroxyl group, an amino group, a carboxyl group, a lower alkoxyl group, or a lower alkoxycarbonyl group.

Examples of the lower alkoxyl group (having 1 to 4 carbon atoms) include a methoxy group, an ethoxy group, an n-propoxy group, an isopropoxy group, an allyloxy group, an n-butoxy group, an isobutoxy group, a sec-butoxy group, and a tert-butoxy group. Examples of the lower alkoxycarbonyl group (having 2 to 4 carbon atoms) include a methoxycarbonyl group, an ethoxycarbonyl group, an n-propoxycarbonyl group, an isopropoxycarbonyl group, and an allyloxycarbonyl group.

Any combination of the substituents exemplified in $R^1$ and $R^2$ may be used for an aminomethyl group $R^1R^2N-CH_2-$. Examples thereof include an aminomethyl group, an N-methylaminomethyl group, an N,N-dimethylaminomethyl group, an N,N-diethylaminomethyl group, an N-ethyl-N-methylaminomethyl group, an N,N-diisopropylaminomethyl group, a formylaminomethyl group, an acetylaminomethyl group, a propanoylaminomethyl group, a butanoylaminomethyl group, a 1-piperidylmethyl group, and a 1-pyrrolidinylmethyl group. Of those, an aminomethyl group is preferable.

The aromatic ring A is a 5- or 6-membered aromatic ring that may contain 1 to 4 hetero atoms selected from the group consisting of nitrogen atom, sulfur atom, and oxygen atom. Examples of the 5- or 6-membered ring include a benzene ring, a pyridine ring, a pyrazine ring, a pyrimidine ring, a pyridazine ring, a pyrrole ring, an imidazole ring, a pyrazole ring, a thiophene ring, a furan ring, a thiazole ring, an isothiazole ring, an oxazole ring, an isoxazole ring, and a cyclopentadiene ring. Of those, the benzene ring, the pyridine ring, and the thiophene ring are preferable, and

the benzene ring is more preferable.

Examples of the substituent of the 5- or 6-membered aromatic ring A which may be substituted include the lower alkyl group, the lower acyl group, the lower alkoxyl group, the lower alkoxycarbonyl group, a lower carboxyalkyl group, a carboxyl group, a hydroxyl group, and a halogen atom.

Examples of the halogen atom include a fluorine atom, a chlorine atom, a bromine atom, and an iodine atom.

A substitution position of the aminomethyl group $R^1R^2N-CH_2-$ of the 5- or 6-membered aromatic ring A may be any position of the aromatic ring A. However, the substitution position is preferably a substitution position of any of partial structures $R^1R^2N-CH_2-A$ each including the aromatic ring A represented by the following formulae (Ia), (Ib), (Ic), and (Id). The substitution position is more preferably the substitution position of any of the structures represented by the following formulae (Ia) and (Ib).

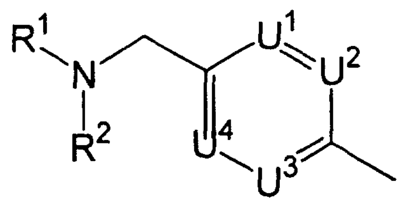

(Ia)

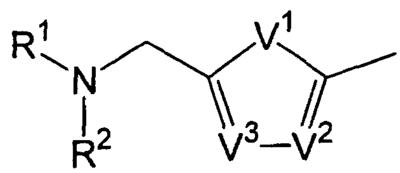

(Ib)

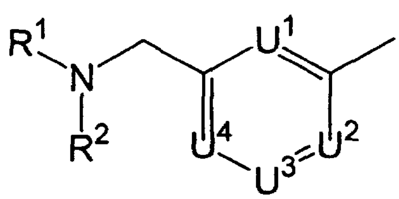

(Ic)

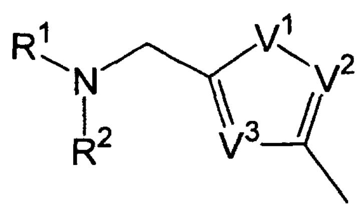

(Id)

Here, $U^1$, $U^2$, $U^3$, and $U^4$ in the above formulae each independently is CH or N. $V^1$ is $CH_2$, NH, O, or S. $V^2$ and $V^3$ each is CH or N.

$R^3$ is hydrogen atom, a lower alkyl group, a lower alkenyl group, a lower alkynyl group, or a lower acyl group.

Examples of the lower alkyl group, the lower alkenyl group, and the lower alkynyl group include the above-described groups. Of those, methyl group is preferable. Examples of the lower acyl group include the above-described groups. Of those, an acetyl group is preferable. $R^3$ is particularly preferably hydrogen atom or methyl group.

In addition, 1 to 5 hydrogen atoms in each of the lower alkyl group, the lower alkenyl group, the lower alkynyl group, and the lower acyl group may be substituted by a hydroxyl group, an amino group, a carboxyl group, a lower alkoxyl group, or a lower alkoxycarbonyl group.

$R^4$ is hydrogen atom or a lower alkyl group. Examples of the lower alkyl group include the above-described groups. Of those, hydrogen atom or methyl group is preferable.

B is a saturated or unsaturated hydrocarbon group which is a 5-to-7-membered monocyclic hydrocarbon group or 6-to-12-membered condensed bicyclic hydrocarbon group, wherein the hydrocarbon group may contain 1 to 3 hetero atoms selected from the group consisting of nitrogen atom, sulfur atom, and oxygen atom. Each of these groups may be substituted with any of substituents. However, the substituent is excluding an amino group, an amidino group, and a guanidino group. Furthermore, with the proviso that B is a benzene ring, the substituent is any of substituents excluding a carboxyl group, an alkoxycarbonyl group, and a carbamoyl group; and

Examples of a mother nucleus hydrocarbon of the monocyclic hydrocarbon group include benzene, pyridine, pyrazine, pyrimidine, pyridazine, triazine, pyrrole, imidazole, pyrazole, thiophene, furan, thiazole, isothiazole, oxazole, isoxazole, thiazoline, thiazine, thiadiazine, thiadiazole, triazole, and cyclopentane.

Furthermore, the monocyclic hydrocarbon group may be substituted with a substituent. However, the substituent is excluding an amino group, an amidino group, and a guanidino group. Furthermore, with the proviso that B is a

benzene ring, the substituent is any of substituents excluding a carboxyl group, an alkoxycarbonyl group, and a carbamoyl group. The substituent of the monocyclic hydrocarbon group is not particularly limited provided that the substituent is none of those substituents. Examples of the substituent include a lower alkyl group, a lower acyl group, a lower alkoxyl group, a lower alkoxycarbonyl group, a lower carboxyalkyl group, an aryloxy group, an aralkyloxy group, an aryl-or-aralkylacyl group, an alkylsulfone group, a carboxyl group, a hydroxyl group, a nitrile group, a carbamoyl group, and a halogen atom, each described above. The examples also include a substituent obtained by substituting a halogen atom (particularly a fluorine atom) for hydrogen atom in each of those substituents.

Examples of the aryloxy group include a phenoxy group. Examples of the aralkyloxy group include a benzyloxy group and a phenethyloxy group. Examples of the aryl-or-aralkylacyl group include a benzoyl group and a phenylacetyl group. Examples of the alkylsulfone group include a methanesulfonyl group, a trifluoromethanesulfonyl group, and an ethanesulfonyl group.

Furthermore, a group to be bonded to an amino nitrogen in a carbamoyl group is preferably hydrogen atom or a lower alkyl group.

Examples of a ring of the two rings of the saturated or unsaturated 6-to-12-membered condensed bicyclic hydrocarbon group which may contain 1 to 3 hetero atoms selected from the group consisting of nitrogen atom, sulfur atom, and oxygen atom, the ring being bonded to X, include the same cyclic hydrocarbons as the examples of the mother nucleus hydrocarbon of the monocyclic hydrocarbon group. In addition, the ring is preferably benzene, pyrrole, furan, or thiophene.

Examples of a ring of the two rings of the saturated or unsaturated 6-to-12-membered condensed bicyclic hydrocarbon group which may contain 1 to 3 hetero atoms selected from the group consisting of nitrogen atom, sulfur atom, and oxygen atom, the ring not being bonded to X, may include benzene, pyridine, pyrazine, pyrimidine, pyridazine, triazine, pyrane, pyrrole, imidazole, pyrazole, thiophene, furan, thiazole, isothiazole, oxazole, isoxazole, thiazoline, thiazine, thiadiazine, thiadiazole, triazole, cyclohexane, cyclopentane, piperidine, piperazine, morpholine, pyridone, 1,3-dioxolan, cyclohexene, 1,3-cyclohexadiene, 1,4-cyclohexadiene, cyclopentene, cyclopentadiene, pyrroline, imidazoline, and pyrazoline. Among those rings, preferred are benzene, pyridine, thiophene, pyrrole, cyclohexane, cyclopentane, piperidine, piperazine, and morpholine. The benzene ring and the pyrrole ring are more preferable.

Each of the two rings of the condensed bicyclic hydrocarbon group may have a substituent. The substituent is not particularly limited as long as the substituent is excluding an amino group, an amidino group, and a guanidino group. Examples of the substituent include a lower alkyl group, a lower acyl group, a lower alkoxyl group, a lower alkoxycarbonyl group, a lower carboxyalkyl group, an aryloxy group, the aralkyloxy group, an aryl-or-aralkylacyl group, an alkylsulfone group, a carboxyl group, a hydroxyl group, a nitrile group, a carbamoyl group, and a halogen atom, each described above. Furthermore, hydrogen atom in each of those substituents may be substituted with a halogen atom.

Any combination of the examples of the ring bonded to X and the examples of the ring not bonded to X may be used for a mother nucleus hydrocarbon of the saturated or unsaturated 6-to-12-membered condensed bicyclic hydrocarbon group which may contain 1 to 3 hetero atoms selected from the group consisting of nitrogen atom, sulfur atom, and oxygen atom. Preferable examples of the ring include a naphthalene ring, an indole ring, a benzofuran ring, a benzothiophene ring, a benzothiazole ring, an indan ring, a quinoline ring, an isoquinoline ring, and a quinolone ring. The ring is more preferably naphthalene, indole, benzo[b]furan, or benzo[b]thiophene. A bonding position of X is preferably 2-, 5-, or 6-position of the B.

X is a carbonyl group (-CO-), a sulfonyl group ($-SO_2-$), a methylene group ($-CH_2-$), a vinylenecarbonyl (-CO-CH=CH-), a vinylenesulfonyl group ($-SO_2-CH=CH-$), an oxymethylenecarbonyl group ($-CO-CH_2O-$), or an oxymethylenesulfonyl group ($-SO_2-CH_2O-$). Preferably, X is a carbonyl group (-CO-), a sulfonyl group ($-SO_2-$), or a methylene group ($-CH_2-$). More preferably, X is a carbonyl group (-CO-) or a sulfonyl group ($-SO_2-$).

As described above, the amine derivative of the present invention is an amine derivative represented by the formula (I) or the pharmaceutically acceptable salt thereof (Aspect 1-1).

In the formula (I), $R^1$, $R^2$, and $R^3$ each independently is hydrogen atom, a lower alkyl group, a lower alkenyl group, a lower alkynyl group, or a lower acyl group; $R^1$ and $R^2$ may be bonded to each other to form one of a 5-membered ring and a 6-membered ring each containing nitrogen atom to which $R^1$ and $R^2$ are bonded; and 1 to 5 hydrogen atoms in each of the lower alkyl group, the lower alkenyl group, the lower alkynyl group, and the lower acyl group may be substituted by a hydroxyl group, an amino group, a carboxyl group, a lower alkoxyl group, or a lower alkoxycarbonyl group.

$R^4$ is hydrogen atom or a lower alkyl group.

A is an aromatic ring which is a 5-membered aromatic ring or 6-membered aromatic ring, wherein the aromatic ring may contain 1 to 4 hetero atoms selected from the group consisting of nitrogen atom, sulfur atom, and oxygen atom, and A may be substituted;

B is a saturated or unsaturated hydrocarbon group which is a 5-to-7-membered monocyclic hydrocarbon group or 6-to-12-membered condensed bicyclic hydrocarbon group, wherein the hydrocarbon group may contain 1 to 3 hetero

atoms selected from the group consisting of nitrogen atom, sulfur atom, and oxygen atom. Each of these groups may be substituted with any of substituents. However, the substituent is excluding an amino group, an amidino group, and a guanidino group. Furthermore, with the proviso that B is a benzene ring, the substituent is any of substituents excluding a carboxyl group, an alkoxycarbonyl group, and a carbamoyl group; and

X is a carbonyl group (-CO-), a sulfonyl group ($-SO_2-$), a methylene group ($-CH_2-$), a vinylenecarbonyl group (-CO-CH=CH-), a vinylenesulfonyl group ($-SO_2-CH=CH-$), an oxymethylenecarbonyl group ($-CO-CH_2O-$), or an oxymethylenesulfonyl group ($-SO_2-CH_2O-$).

The term "lower" in the phrases such as 'lower alkyl group' refers to a straight-chain or branched-chain compound having 1 to 4 carbon atoms. Furthermore, the phrase 'pharmaceutically acceptable salt thereof' of the present invention refers to a salt that retains biological effectiveness and properties of its parent compound and that is not undesirable biologically and in other respects.

Here, each of $R^1$ and $R^2$ is preferably hydrogen atom (Aspect 1-2).

The 5- or 6-membered aromatic ring A is preferably a benzene ring (Aspect 1-3). Further, a partial structure $R^1R^2N-CH_2-A-$ containing the 5- or 6-membered aromatic ring A preferably has a structure represented by the following formulae (Aspect 1-4).

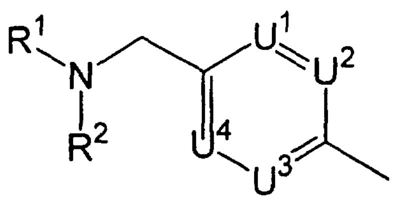

(Ia)

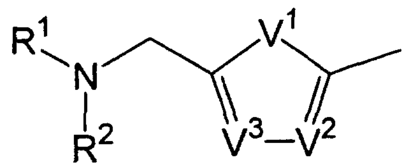

(Ib)

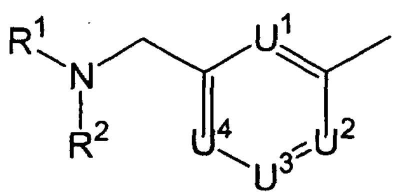

(Ic)

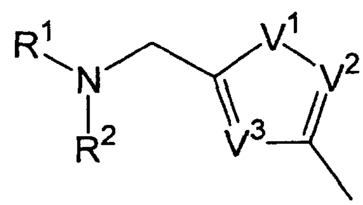

(Id)

Here, $U^1$, $U^2$, $U^3$, and $U^4$ in the formulae each independently is CH or N. $V^1$ is $CH_2$, NH, O, or S. $V^2$ and $V^3$ each is CH or N.

X is preferably one of a carbonyl group (-CO-), a sulfonyl group ($-SO_2-$), and a methylene group ($-CH_2-$) (Aspect 1-5).

Further, B is preferably a saturated or unsaturated 6-to-12-membered condensed bicyclic hydrocarbon group which may contain 1 to 3 hetero atoms selected from the group consisting of nitrogen atom, sulfur atom, and oxygen atom (Aspect 1-6).

A ring of the two rings of the condensed bicyclic hydrocarbon group, which is bonded to X, is preferably a benzene ring, a pyrrole ring, a furan ring, or a thiophene ring (Aspect 1-7).

A ring of the two rings of the condensed bicyclic hydrocarbon group, which is not bonded to X, is preferably a benzene ring or a pyrrole ring (Aspect 1-8).

In particular, B is preferably naphthalene, indole, benzo[b]furan, or benzo[b]thiophene. Preferably, a bonding position of X is 2-, 5-, or 6-position (Aspect 1-9).

The compound represented by the formula (I) of the present invention includes all of its isomers. For instance, in the case where the compound has an asymmetric carbon atom, the compound can be existent in the form of a mixture of different stereoisomers or of stereoisomers including a racemic form. In other words, the present invention also includes various forms defined as described above, and these forms can be similarly used for active ingredient compounds. Such a stereoisomer can be isolated and purified by one skilled in the art with an ordinary technique via

optical resolution using preferential crystallization or column chromatography, or via asymmetric synthesis.

Furthermore, intermediates as novel substances obtained in synthesizing amine derivatives of the present invention are also included in the present invention. Table 5 lists $^1$H-NMR data for a part of those intermediates. However, the intermediate of the present invention is not limited to those intermediates.

The phrase 'pharmaceutically acceptable salt thereof' of the present invention refers to a salt that retains biological effectiveness and properties of its parent compound and that is not undesirable biologically and in other respects. Specifically, the definition of such a salt also includes: salts of amine derivatives of the present invention and inorganic acids such as hydrochloric acid, hydrobromic acid, sulfuric acid, and phosphoric acid; salts (including a monosalt, a disalt, and a trisalt) of amine derivatives of the present invention and organic acids such as acetic acid, lactic acid, tartaric acid, malic acid, succinic acid, fumaric acid, maleic acid, citric acid, benzoic acid, trifluoroacetic acid, p-toluenesulfonic acid, and methanesulfonic acid; hydrates of amine derivatives of the present invention and of salts thereof; solvates acceptable as medicines of amine derivatives of the present invention and of salts thereof; and crystal polymorphs acceptable as medicines of amine derivatives of the present invention.

Further, the present invention provides a medicine characterized by containing as an active ingredient one of the amine derivatives described in Aspects 1-1 to 1-9 or the pharmaceutically acceptable salt thereof (Aspect 2).

Here, the medicine of the present invention is a medicine characterized by being a β-tryptase inhibitor, and is preferably a medicine characterized by being a human β-tryptase inhibitor (Aspect 3).

Further, the medicine of the present invention is a prophylactic/therapeutic agent for diseases the crisis and evolution of which are considered to be attributed to β-tryptase, such as: respiratory failures such as bronchial asthma, lung fibrosis, and chronic obstructive pulmonary disease (COPD); and allergic diseases such as anaphylaxis, allergic rhinitis, allergic conjunctivitis, atopic dermatitis, and urticaria. In addition, the medicine of the present invention is a prophylactic/therapeutic agent for diseases the crisis and evolution of which are considered to be attributed to β-tryptase such as: an inflammatory bowel disease; a hyperproliferative skin disease; vascularedema; and rheumatoid arthritis. Preferably, the medicine of the present invention is a prophylactic/therapeutic agent for bronchial asthma or an allergic disease such as atopic dermatitis (Aspect 4).

The present invention provides a prophylactic/therapeutic method for diseases the crisis and evolution of which are considered to be attributed to β-tryptase, such as: respiratory failures such as bronchial asthma, lung fibrosis, and chronic obstructive pulmonary disease (COPD); and allergic diseases such as anaphylaxis, allergic rhinitis, allergic conjunctivitis, atopic dermatitis, and urticaria, by using a pharmaceutical composition containing as an active ingredient the amine derivative described in Aspects 1-1 to 1-9 or the pharmaceutically acceptable salt thereof. In addition, the present invention provides a prophylactic/therapeutic method for diseases the crisis and evolution of which are considered to be attributed to β-tryptase, such as: an inflammatory bowel disease; a hyperproliferative skin disease; vascular edema; and rheumatoid arthritis, by using a medical composition containing as an active ingredient the amine derivative described in Aspects 1-1 to 1-9 or the pharmaceutically acceptable salt thereof. The present invention provides a prophylactic/therapeutic method particularly for an allergic disease (Aspect 5), by using a pharmaceutical composition containing as an active ingredient the amine derivative described in Aspects 1-1 to 1-9 or the pharmaceutically acceptable salt thereof.

Subsequently, methods of producing compounds of the present invention are shown, and a description is given of each reaction step. In the following <Production Method 1>, <Production Method 2>, <Production Method 3>, and <Production Method 4>, definitions of A, B, $R^1$, $R^2$, $R^3$, $R^4$, and X in the compounds represented by the formula (I) in the reaction schemes and in the descriptions are identical to those described above unless otherwise stated.

The compounds represented by the formula (I) and salts thereof of the present invention can be produced from compounds represented by the formula (III) (where A has the same meaning as that described above, and $R^{1'}$ and $R^{2'}$ have the same meanings as those of $R^1$ and $R^2$ described above or each represent a protective group of an amino group), the formula (IV) (where B, X, and $R^4$ have the same meanings as those described above, and $R^{3'}$ represents a lower alkyl group that may be substituted by hydrogen atom, a hydroxyl group, an amino group, a carboxyl group, an alkoxyl group, or an alkoxycarbonyl group; or a protective group of an amino group), the formula (V) (where $R^{3'}$ and $R^4$ have the same meanings as those in the formula (IV), and P represents a protective group of an amino group), the formula (VIII) (where B has the same meaning as that described above, Y represents -CO-, $-SO_2-$, -CO-CH=CH-, $-SO_2-CH=CH-$, $-CO-CH_2O-$, or $-SO_2-CH_2O-$, and Z represents a chlorine atom or represents a hydroxyl group or hydrogen atom when Y represents -CO-, -CO-CH=CH-, or $-CO-CH_2O-$), the formula (IX) (where A, $R^{1'}$, $R^{2'}$, and $R^{3'}$ have the same meanings as those described above), the formula (X) (where B, X, and $R^4$ have the same meanings as those described above), the formula (XI) (where $R^4$ and P have the same meanings as those described above), the formula (XII) (where A, $R^{1'}$ and $R^{2'}$ have the same meanings as those described above), the formula (XVI) (where B, X, and $R^4$ have the same meanings as those described above), the formula (XVII) (where $R^4$ and P have the same meanings as those described above), the formula (XVIII) (where A has the same meaning as that described above), the formula (XXII) (where A and $R^{3'}$ have the same meanings as those described above), and the formula

(XXIII) (where A has the same meaning as that described above), which are readily produced from compounds known in literatures or from commercially available compounds, by each of <Production Method 1>, <Production Method 2>, <Production Method 3>, and <Production Method 4> to be described below or by modification thereof. In each production method, starting materials, intermediates, or products can be treated as a salt as required.

Hereinafter, a detailed description is given of each production method.

Isolation and purification of products and intermediates described herein can be performed by any suitable isolation or purification such as filtration, extraction, crystallization, column chromatography, thin-layer chromatography, thick-layer chromatography, and high-performance liquid chromatography (HPLC) or by combinations of these.

All starting material compounds and reagents may be prepared by well-known methods. Alternatively, commercially available products may be used for the starting material compounds and reagents.

<Production Method 1>

A compound represented by the formula (I) or a salt thereof can be produced from a compound represented by the formula (III) and a compound represented by the formula (IV), or a compound represented by the formula(III) and a compound represented by the formula (V) according to each production step of <<Reaction Scheme 1>> shown below.

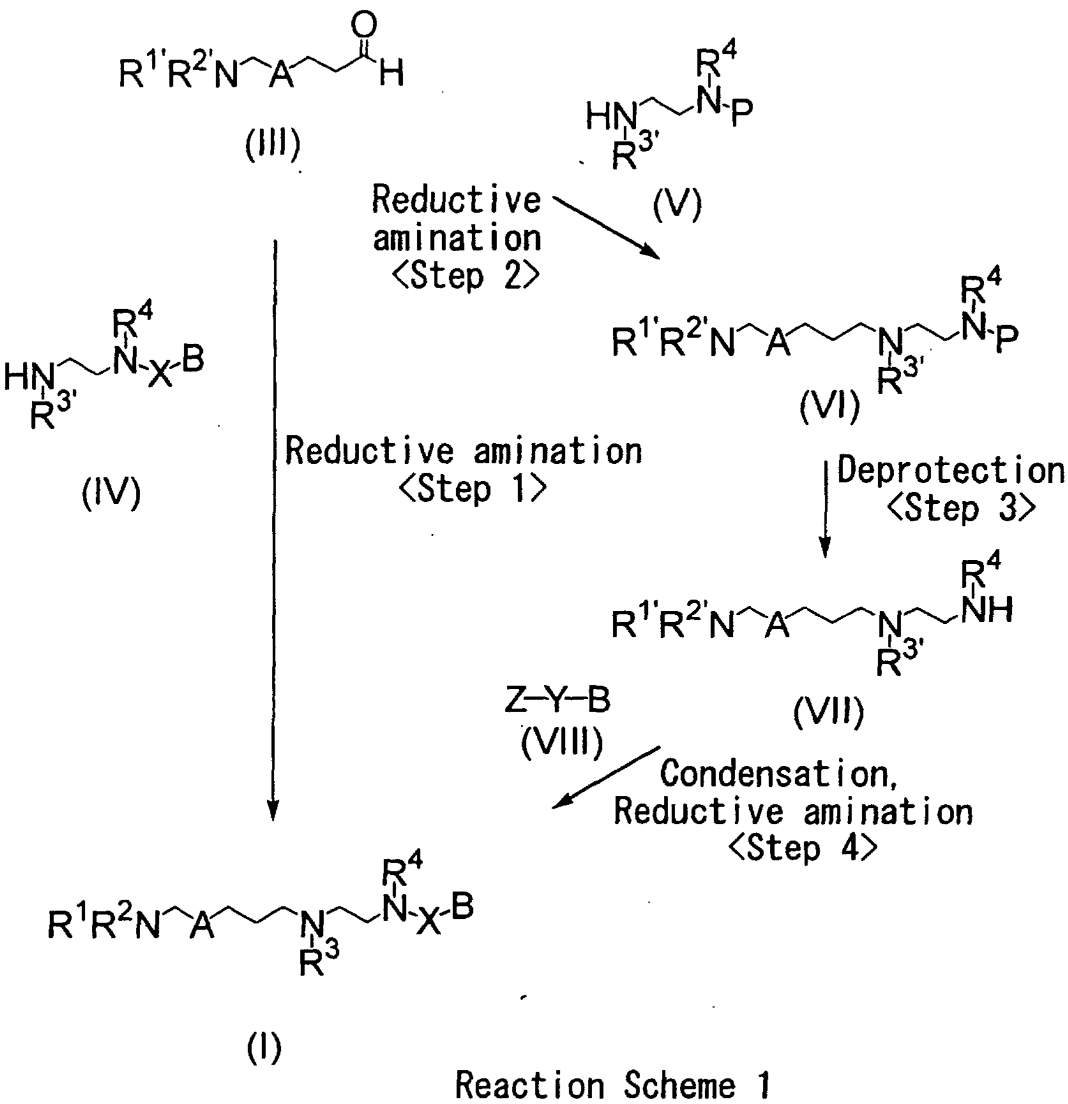


Reaction Scheme 1

<Step 1>

A compound represented by the formula (I) or a salt thereof can be produced from a compound represented by the formula (III) and a compound represented by the formula (IV) according to the following method for reductive amination.

The compound represented by the formula (I) and the salt thereof can be produced by reacting a compound represented by the formula (III) with a compound represented by the formula (IV) in a solvent that is not involved in the reaction such as an aromatic hydrocarbon solvent such as toluene or benzene, a halogenated hydrocarbon solvent such as methylene chloride or chloroform, or an alcoholic solvent such as methanol or ethanol in the presence or absence of an acid catalyst such as acetic acid by using a suitable reducing agent. In general, every reducing agent that can reduce an imino group to an amino group can be used. Among them, preferable are reducing agents such as sodium triacetoxyborohydride, sodium borohydride, lithium borohydride, diisobutylaluminum hydride, and sodium cyanoborohydride. The reaction can be performed at a temperature between -78°C and room temperature, preferably at room temperature, until the reaction progresses sufficiently, specifically for 3 to 12 hours.

In the case where any of $R^{1'}$, $R^{2'}$, and $R^{3'}$ of the compound represented by the formula (III) and the compound represented by the formula (IV) is a protective group, the compound represented by the formula (I) or the salt thereof can be produced by deprotection of the protective group.

Examples of the protective group include suitable protective groups described in the topic of "Protective Groups in Organic Synthesis" second edition, 1991, edited by T. W. Green and P. G. M. Wuts, published from John Wiley and Sons, such as: alkyl protective groups including a benzyl group, a trityl group, and a methoxymethyl group; and carbamate protective groups including a tert-butoxycarbonyl group and a benzyloxycarbonyl group. The deprotection can be performed in conformance with a method described in the topic of "Protective Groups in Organic Synthesis", second edition, 1991. For example, in the case where the protective group is a benzyl group, a benzyloxycarbonyl group, or the like, the deprotection can be performed by using palladium-carbon, platinum oxide, or the like as a catalyst in a solvent such as: an alcoholic solvent such as methanol or ethanol; ethyl acetate; acetic acid; or water under hydrogen atmosphere or in the presence of ammonium formate between 0°C and a temperature at which a reaction mixture is refluxed. Moreover, in the case where the protective group is a tert-butoxycarbonyl group, the deprotection can be performed in the presence or absence of anisole by using an acid such as trifluoroacetic acid or hydrochloric acid between 0°C and a temperature at which the solvent is refluxed.

A compound represented by the formula (I) or a salt thereof can be also produced from a compound represented by the formula (III) and a compound represented by the formula (V) according to <Step 2>, <Step 3>, and <Step 4> to be described below.

<Step 2>

A compound represented by the formula (VI) (where A, $R^{1'}$, $R^{2'}$, $R^{3'}$, $R^4$, and P have the same meanings as those described above) can be produced from a compound represented by the formula (III) and a compound represented by the formula (V) according to the method for reductive amination described in <Step 1>.

<Step 3>

A compound represented by the formula (VII) (where A, $R^{1'}$, $R^{2'}$, $R^{3'}$, and $R^4$ have the same meanings as those described above) can be produced from a compound represented by the formula (VI) according to the deprotection method described in <Step 1>.

<Step 4>

The compound represented by the formula (I) or the salt thereof can be produced from the compound represented by the formula (VII) and a compound represented by the formula (VIII) by the following method depending on the kind of Z-Y- in the formula (VIII).

(Method A): In the case where Z-Y- represents sulfonyl chloride or an acid chloride, the compound represented by the formula (I) or the salt thereof can be produced by reacting the compound represented by the formula (VII) with the compound represented by the formula (VIII) in the presence of an organic base such as triethylamine or pyridine, or an inorganic base such as potassium carbonate in a solvent such as: a halogenated hydrocarbon solvent such as methylene chloride or chloroform; an ether solvent such as diethyl ether or tetrahydrofuran; or a hydrocarbon solvent such as benzene or hexane; or in a basic solvent such as pyridine or triethylamine between -20°C and a temperature at which a reaction mixture is refluxed.

(Method B): In the case where Z-Y- represents a carboxylic acid (carboxyl group), the compound represented by the formula (I) or the salt thereof can be produced by reacting the compound represented by the formula (VII) with the compound represented by the formula (VIII) by using a condensation agent such as 1-ethyl-3-(3-dimethylaminopropyl)carbodiimide hydrochloride (water-soluble carbodiimide hydrochloride, WSC·HCl) or dicyclohexylcarbodiimide (DCC) in a solvent that is not involved in the reaction such as: a halogenated hydrocarbon solvent such as methylene chloride or chloroform; an ether solvent such as diethyl ether or tetrahydrofuran; a hydrocarbon solvent such as benzene or hexane; or a polar solvent such as dimethylformamide or dimethyl sulfoxide between 0°C and a temperature at which a reaction mixture is refluxed.

The reaction can be also performed by using a dehydrating agent such as phosphorous oxychloride in the presence of a base such as pyridine or triethylamine in a solvent that is not involved in the reaction such as: a halogenated hydrocarbon solvent such as methylene chloride or chloroform; an ether solvent such as diethyl ether or tetrahydrofuran; or a hydrocarbon solvent such as benzene or hexane between -20°C and a temperature at which a reaction mixture is refluxed.

Furthermore, the compound represented by the formula (I) or the salt thereof can be produced by converting the compound represented by the formula (VIII) into an acid chloride by using thionyl chloride or the like, and then by reacting the acid chloride with the compound represented by the formula (VII) in the presence of an organic base such as triethylamine or pyridine, or an inorganic base such as potassium carbonate in a solvent that is not involved in the reaction such as: a halogenated hydrocarbon solvent such as methylene chloride or chloroform; an ether solvent such as diethyl ether or tetrahydrofuran; or a hydrocarbon solvent such as benzene or hexane; or in a basic solvent such as pyridine or triethylamine between -20°C and a temperature at which a reaction mixture is refluxed.

(Method C): In the case where Z-Y- represents an aldehyde (formyl group), the compound represented by the formula (I) or the salt thereof can be produced from the compound represented by the formula (VII) and the compound represented by the formula (VIII) according to the method for reductive amination described in <Step 1>.

In the case where any of $R^{1'}$, $R^{2'}$, and $R^{3'}$ of the compound represented by the formula (VII) is a protective group, the compound represented by the formula (I) or the salt thereof can be produced by performing the deprotection described in <Step 1>.

The compound represented by the formula (I) or the salt thereof can be also produced by using the compound represented by the formula (IX) and the compound represented by the formula (X) instead of using the compound represented by the formula (III) and the compound represented by the formula (IV) in <Step 1> of <Production Method 1>.

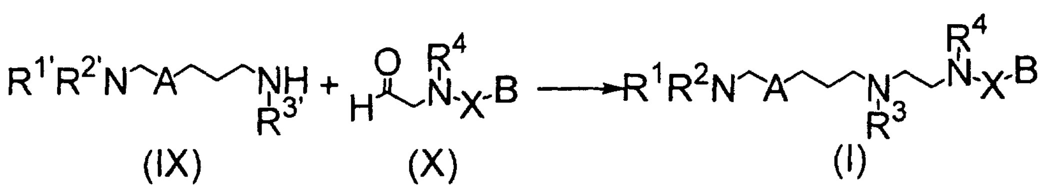

(IX) (X) (I)

Reaction Scheme 2

Furthermore, the compound represented by the formula (VI) can be also produced by using the compound represented by the formula (IX) and the compound represented by the formula (XI) instead of using the compound represented by the formula (III) and the compound represented by the formula (V) in <Step 2> of <Production Method 1>.

Subsequently, the compound represented by the formula (I) or the salt thereof can be also produced according to <Step 3> and <Step 4>.

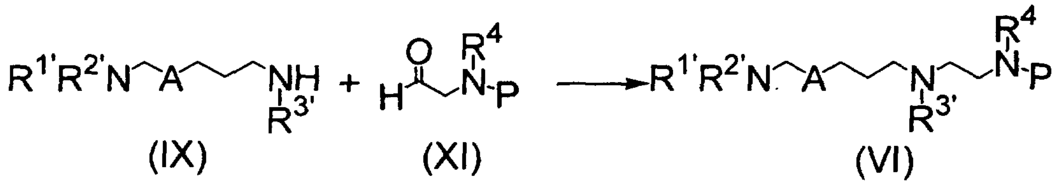

(IX) (XI) (VI)

Reaction Scheme 3

<Production Method 2>

The following formula (I') represents a compound in the case where X represents $-SO_2-$, $-CH_2-$, $-SO_2-CH=CH-$, or $-SO_2-CH_2O-$ in the formula (I).

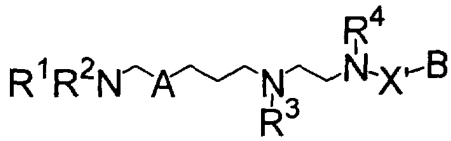

(I')

The compound represented by the formula (I) or the salt thereof or the compound represented by the formula (I') (where A, B, $R^1$, $R^2$, $R^3$, and $R^4$ have the same meanings as those described above, and X' represents $-SO_2-$, $-CH_2-$, $-SO_2-CH=CH-$, or $-SO_2-CH_2O-$) or a salt thereof can be produced from the compound represented by the formula (XII) and the compound represented by the formula (IV), the compound represented by the formula (XII), or the compound represented by the formula (V) according to each production step of <<Reaction Scheme 4>>.

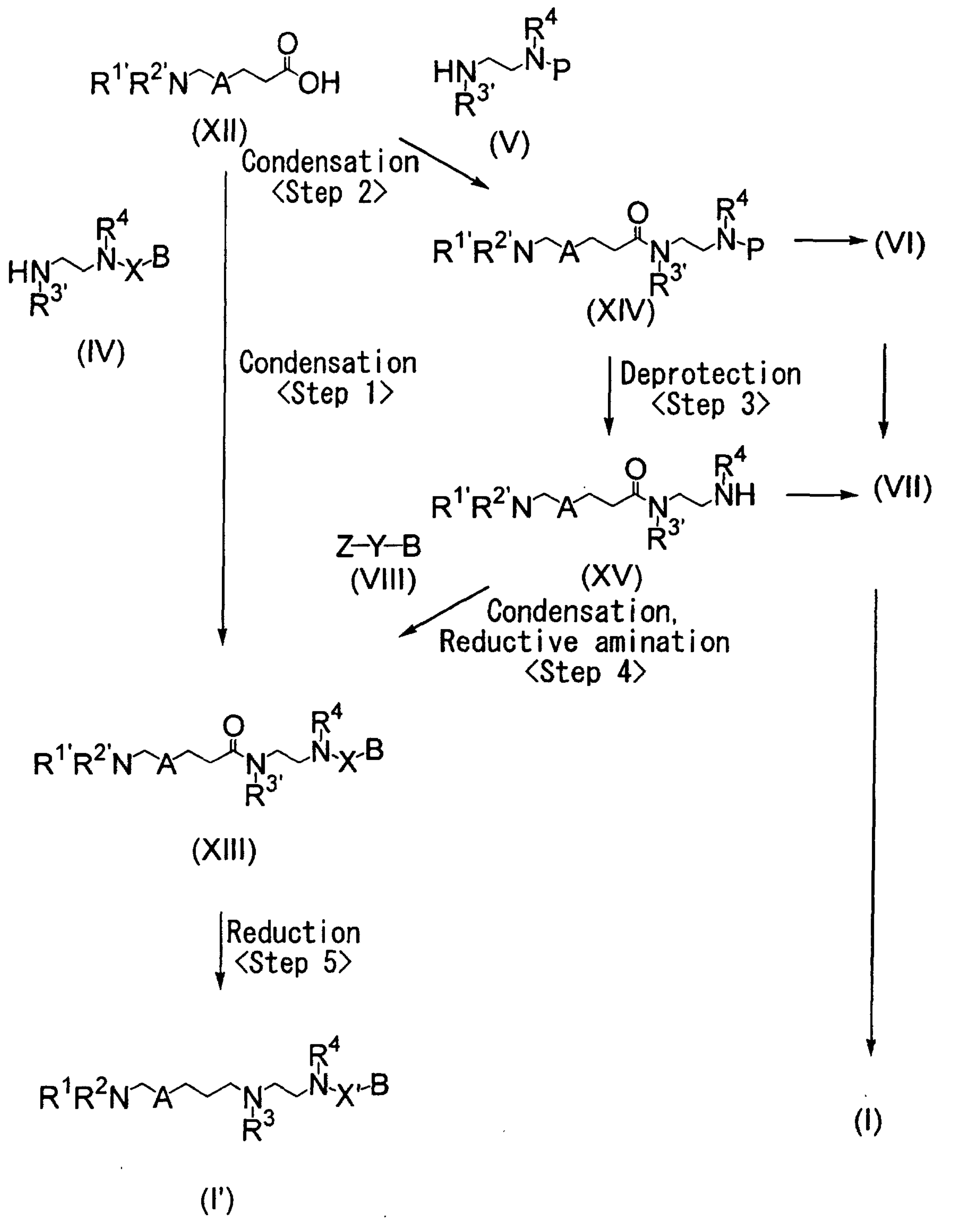


Reaction Scheme 4

<Step 1>

A compound represented by the formula (XIII) (where A, B, $R^{1'}$, $R^{2'}$, $R^{3'}$, $R^4$, and X have the same meanings as those described above) can be produced from the compound represented by the formula (IV) and the compound represented by the formula (XII) according to (Method B) of <Step 4> of <Production Method 1>.

Further, the compound represented by the formula (XIII) can also be produced from the compound represented by the formula (V) and the compound represented by the formula (XII) according to <Step 2>, <Step 3>, and <Step

4> described below.

<Step 2>

A compound represented by the formula (XIV) (where A, $R^{1'}$, $R^{2'}$, $R^{3'}$, $R^4$, and P have the same meanings as those described above) can be produced from the compound represented by the formula (V) and the compound represented by the formula (XII) according to (Method B) of <Step 4> of <Production Method 1>.

<Step 3>

A compound represented by the formula (XV) (where A, $R^{1'}$, $R^{2'}$, $R^{3'}$, and $R^4$ have the same meanings as those described above) can be produced from the compound represented by the formula (XIV) according to the deprotection method described in <Step 1> of <Production Method 1>.

<Step 4>

The compound represented by the formula (XIII) can be produced from the compound represented by the formula (VIII) and the compound represented by the formula (XV) according to <Step 4> of <Production Method 1>.

<Step 5>

The compound represented by the formula (I') or the salt thereof can be produced by reacting the compound represented by the formula (XIII) by using a reducing agent such as lithium aluminum hydride, diisobutylaluminum hydride, or a borane complex typified by boranetetrahydrofuran complex in a solvent that is not involved in the reaction such as: an ether solvent such as diethyl ether or tetrahydrofuran; or an aromatic hydrocarbon solvent such as toluene or benzene between 0°C and a temperature at which a reaction mixture is refluxed.

In the case where any of $R^{1'}$, $R^{2'}$, and $R^{3'}$ of the compound represented by the formula (XIII) is a protective group, the compound represented by the formula (I') or the salt thereof can be produced by performing the deprotection described in <Step 1> of <Production Method 1>.

The compound represented by the formula (VI) can be produced by reducing the compound represented by the formula (XIV) produced in <Step 2> according to the method of <Step 5>. Subsequently, the compound represented by the formula (I) or the salt thereof can be produced according to <Step 3> and <Step 4> of <Production Method 1>.

Furthermore, the compound represented by the formula (VII) can be produced by reducing the compound represented by the formula (XV) produced in <Step 3> according to the method of <Step 5>. Subsequently, the compound represented by the formula (I) or the salt thereof can be produced according to <Step 4> of <Production Method 1>.

The compound represented by the formula (XIII') (where A, B, $R^{1'}$, $R^{2'}$, $R^{3'}$, $R^4$, and X have the same meanings as those described above) can be produced by using the compound represented by the formula (IX) and the compound represented by the formula (XVI) instead of using the compound represented by the formula (IV) and the compound represented by the formula (XII) in <Step 1> of <Production Method 2>. Subsequently, the compound represented by the formula (I') or the salt thereof can also be produced according to <Step 5>.

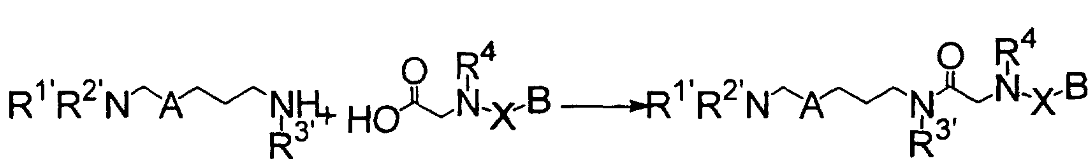

(IX) (XVI) (XIII')

Reaction Scheme 5

The compound represented by the formula (XIV') (where A, $R^{1'}$, $R^{2'}$, $R^{3'}$, $R^4$, and P have the same meanings as those described above) can be produced by using the compound represented by the formula (XVII) and the compound represented by the formula (IX) instead of using the compound represented by the formula (V) and the compound represented by the formula (XII) in <Step 2> of <Production Method 2>. Subsequently, the compound represented by the formula (I') or the salt thereof can also be produced according to <Step 3>, <Step 4>, and <Step 5>.

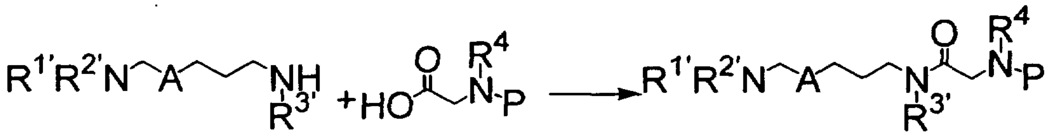

(IX) (XVII) (XIV')

Reaction Scheme 6

<Production Method 3>

The compound represented by the formula (I) or the salt thereof can be produced from the compound represented by the formula (XVIII) and the compound represented by the formula (IV), the compound represented by the formula (XVIII), or the compound represented by the formula (V) according to the respective production steps of <<Reaction Scheme 7>> .

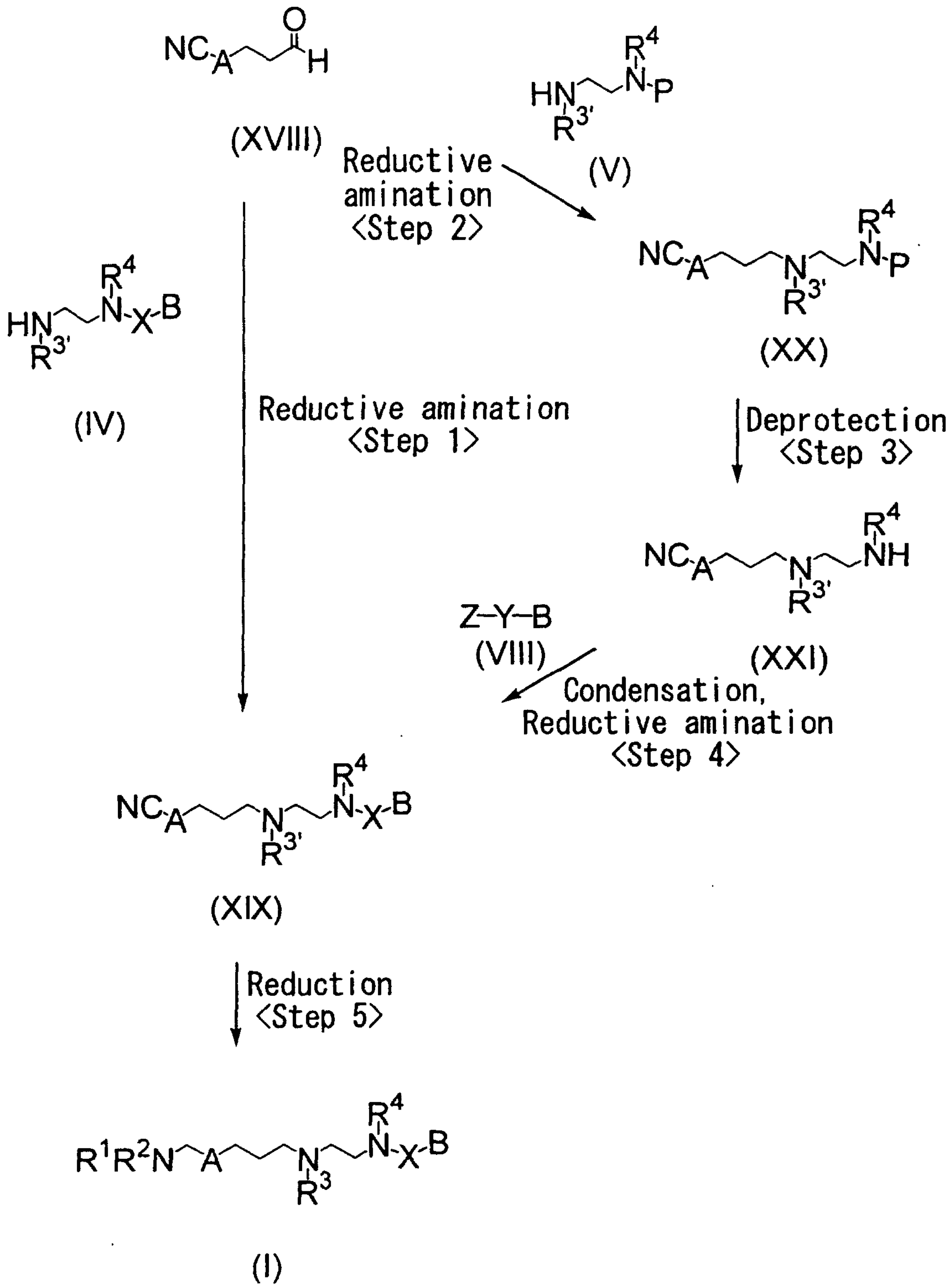


Reaction Scheme 7

<Step 1>

A compound represented by the formula (XIX) (where A, B, $R^{3'}$, $R^4$, and X have the same meanings as those described above) can be produced from the compound represented by the formula (IV) and the compound represented by the formula (XVIII) according to the method for reductive amination described in <Step 1> of <Production Method 1>. However, a reducing agent to be used for reducing an imino group to an amino group is a reducing agent that does not reduce a nitrile group such as sodium triacetoxyborohydride, sodium borohydride, lithium borohydride, or sodium

cyanoborohydride.

The compound represented by the formula (XIX) can also be produced from a compound represented by the formula (V) and the compound represented by the formula (XVIII) according to <Step 2>, <Step 3>, and <Step 4> described below.

<Step 2>

A compound represented by the formula (XX) (where A, $R^{3'}$, $R^4$, and P have the same meanings as those described above) can be produced from the compound represented by the formula (V) and the compound represented by the formula (XVIII) according to the method of <Step 1>.

<Step 3>

A compound represented by the formula (XXI) (where A, $R^{3'}$, and $R^4$ have the same meanings as those described above) can be produced from the compound represented by the formula (XX) according to the deprotection method described in <Step 1> of <Production Method 1>.

<Step 4>

A compound represented by the formula (XIX) can be produced from the compound represented by the formula (VIII) and the compound represented by the formula (XXI) according to <Method A> or <Method B> described in <Step 4> of <Production Method 1> or according to the method of <Step 1> when Z-Y- represents aldehyde.

<Step 5>

The compound represented by the formula (I) or the salt thereof can be produced by reacting the compound represented by the formula (XIX) by using palladium-carbon, platinum oxide, or the like as a catalyst in a solvent such as: an alcoholic solvent such as methanol or ethanol; ethyl acetate; acetic acid; or water under hydrogen atmosphere or in the presence of ammonium formate between 0°C and a temperature at which a reaction mixture is refluxed.

Furthermore, in the case where $R^3$ in the compound represented by the formula (I) or the salt thereof is anything but an acyl group, the compound represented by the formula (I) or the salt thereof can be also produced by reducing the compound represented by the formula (XIX) according to the method of <Step 5> of <Production Method 2>.

In the case where $R^{3'}$ of the compound represented by the formula (XIX) is a protective group, the compound represented by the formula (I) or the salt thereof can be produced by performing the deprotection described in <Step 1> of <Production Method 1>.

The compound represented by the formula (XIX) can be produced by using the compound represented by the formula (X) and the compound represented by the formula (XXII) instead of using the compound represented by the formula (IV) and the compound represented by the formula (XVIII) in <Step 1> of <Production Method 3>. Subsequently, the compound represented by the formula (I) or the salt thereof can also be produced according to <Step 5>.

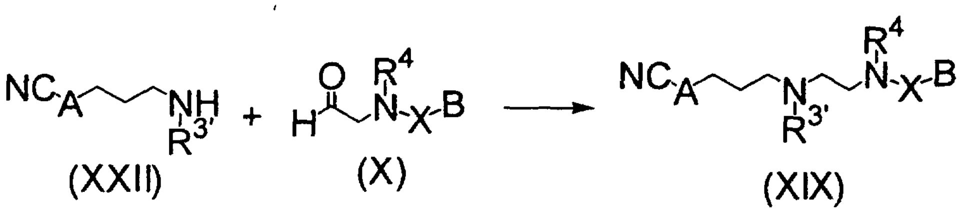

(XXII) (X) (XIX)

Reaction Scheme 8

Also, the compound represented by the formula (XX) can be produced by using the compound represented by the formula (XI) and the compound represented by the formula (XXII) instead of using the compound represented by the formula (V) and the compound represented by the formula (XVIII) in <Step 2> of <Production Method 3>. Subsequently, the compound represented by the formula (I) or the salt thereof can also be produced according to <Step 3>, <Step 4>, and <Step 5>.

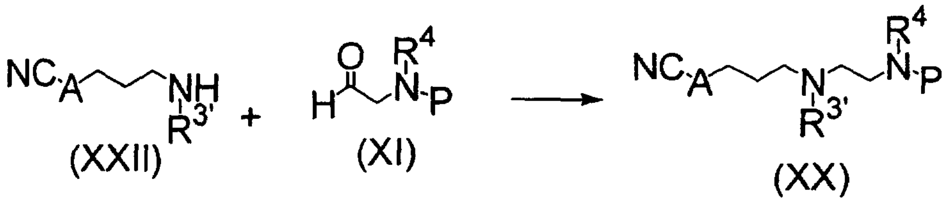

(XXII) (XI) (XX)

Reaction Scheme 9

<Production Method 4>

The compound represented by the formula (I') or the salt thereof can be produced from the compound represented by the formula (XXIII) and the compound represented by the formula (IV), the compound represented by the formula (XXIII), or the compound represented by the formula (V) according to the respective production steps of <<Reaction Scheme 10>> .

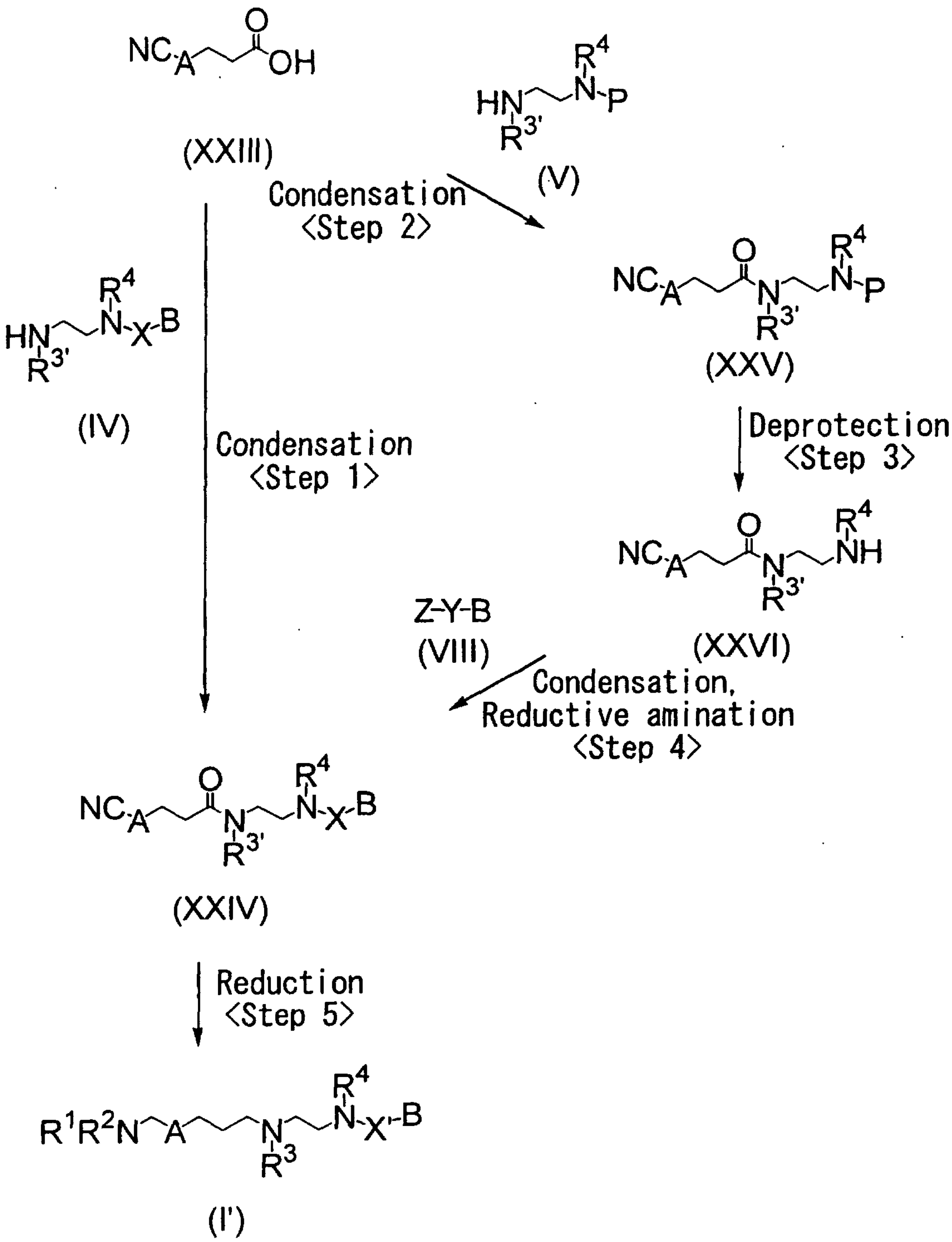


Reaction Scheme 10

<Step 1>

A compound represented by the formula (XXIV) (where A, B, $R^{3'}$, $R^4$, and X have the same meanings as those described above) can be produced from a compound represented by the formula (IV) and the compound represented by the formula (XXIII) according to (Method B) of <Step 4> of <Production Method 1>.

Further, the compound represented by the formula (XXIV) can also be produced from a compound represented

by the formula (V) and the compound represented by the formula (XXIII) according to <Step 2>, <Step 3>, and <Step 4> described below.

<Step 2>

A compound represented by the formula (XXV) (where A, $R^{3'}$, $R^4$, and P have the same meanings as those described above) can be produced from the compound represented by the formula (V) and the compound represented by the formula (XXIII) according to (Method B) of <Step 4> of <Production Method 1>.

<Step 3>

A compound represented by the formula (XXVI) (where A, $R^{3'}$, and $R^4$ have the same meanings as those described above) can be produced from the compound represented by the formula (XXV) according to the deprotection method described in <Step 1> of <Production Method 1>.

<Step 4>

The compound represented by the formula (XXIV) can be produced from a compound represented by the formula (VIII) and the compound represented by the formula (XXVI) according to the method of <Step 4> of <Production Method 3>.

<Step 5>

The compound represented by the formula (I') or the salt thereof can be also produced by reducing the compound represented by the formula (XXIV) according to the method of <Step 5> of <Production Method 2>.

Also, in the case where $R^{3'}$ of the compound represented by the formula (XXIV) is a protective group, the compound represented by the formula (I') or the salt thereof can be produced by performing the deprotection described in <Step 1> of <Production Method 1>.

The compound represented by the formula (XXIV') (where A, B, $R^{3'}$, $R^4$, and X have the same meanings as those described above) can be produced by using the compound represented by the formula (XVI) and the compound represented by the formula (XXII) instead of using the compound represented by the formula (IV) and the compound represented by the formula (XXIII) in <Step 1> of <Production Method 4>. Subsequently, the compound represented by the formula (I') or the salt thereof can also be produced according to <Step 5>.

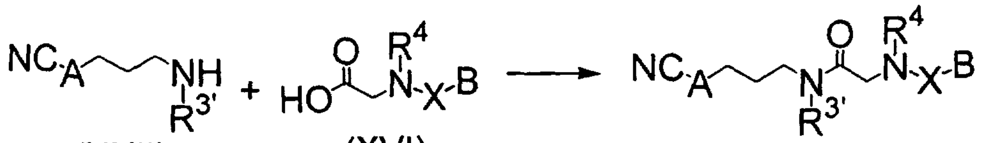

(XXII) (XVI) (XXIV')

Reaction Scheme 1 1

Also, the compound represented by the formula (XXV') (where A, $R^{3'}$, $R^4$, and P have the same meanings as those described above) can be produced by using the compound represented by the formula (XVII) and the compound represented by the formula (XXII) instead of using the compound represented by the formula (V) and the compound represented by the formula (XXIII) in <Step 2> of <Production Method 4>. Subsequently, the compound represented by the formula (I') or the salt thereof can also be produced according to <Step 3>, <Step 4>, and <Step 5>.

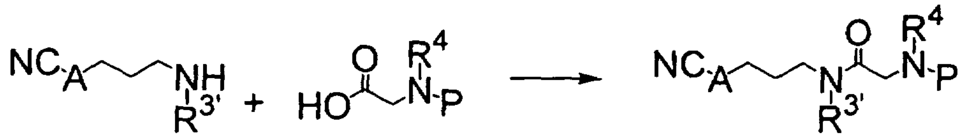

(XXII) (XVII) (XXV')

Reaction Scheme 1 2

In each of the above production methods, a compound in which any of $R^1$, $R^{1'}$, $R^2$, $R^{2'}$, $R^3$, $R^{3'}$, and $R^4$ is hydrogen atom out of the compounds represented by the formula (I), the formula (I'), the formula (VI), the formula (XIII), the formula (XIII'), the formula (XIV), the formula (XIV'), the formula (XIX), the formula (XX), the formula (XXIV), the formula (XXIV'), the formula (XXV), and the formula (XXV') can be alkylated by using an alkylating agent such as: an alkyl halide typified by methyl iodide; or an alkylsulfuric acid typified by dimethyl sulfate in the presence of a base such as potassium hydroxide or sodium hydroxide in a solvent that is not involved in the reaction such as: a halogenated hydrocarbon solvent such as methylene chloride or chloroform; an ether solvent such as diethyl ether or tetrahydrofuran; a hydrocarbon solvent such as benzene or hexane; or a polar solvent such as dimethylformamide or dimethyl sulfoxide between 0°C and a temperature at which a reaction mixture is refluxed.

Furthermore, a compound in which any of $R^1$, $R^{1'}$, $R^2$, $R^{2'}$, $R^3$, and $R^{3'}$ is hydrogen atom out of the compounds represented by the formula (I), the formula (I'), and the formula (VI) or a compound in which any of $R^1$, $R^{1'}$, $R^2$, and $R^{2'}$ is hydrogen atom out of the compounds represented by the formula (XIII), the formula (XIII'), the formula (XIV), and the formula (XIV') can be also alkylated by using an aldehyde derivative or a ketone derivative according to the method for reductive amination described in <Step 1> of <Production Method 1>. A compound in which $R^{3'}$ is hydrogen atom out of the compounds represented by the formula (XIX) and the formula (XX) can be also alkylated by using an aldehyde derivative or a ketone derivative according to the method of <Step 1> of <Production Method 3>.

Furthermore, a compound in which any of $R^1$, $R^{1'}$, $R^2$, $R^{2'}$, $R^3$, and $R^{3'}$ is hydrogen atom out of the compounds represented by the formula (I), the formula (I'), and the formula (VI) or a compound in which any of $R^1$, $R^{1'}$, $R^2$, and $R^{2'}$ is hydrogen atom out of the compounds represented by the formula (XIII), the formula (XIII'), the formula (XIV), and the formula (XIV') can be acylated by using a carboxylic acid derivative according to (Method A) or (Method B) of <Step 4> of <Production Method 1>. At that time, the acylated product can also be led to an alkylated product by reducing the acylated product according to the method of <Step 5> of <Production Method 2>.

In the case where each compound synthesized by each of the above production methods has a reactive group such as a hydroxyl group, an amino group, or a carboxyl group, it is possible to appropriately protect the reactive group by means of a protective group in each production step and to remove the protective group at a suitable stage. Methods of introducing and removing such a protective group are appropriately carried out depending on types of group to be protected and protective group. For instance, the introduction and removal can be carried out by a method described in the topic of "Protective Groups in Organic Synthesis", second edition, 1991.

Next, a prophylactic/therapeutic agent and a medicinal composition of the present invention are described.

Diseases on which the medicine of the present invention effectively acts are diseases the crisis and evolution of which are considered to be attributed to β-tryptase, such as: respiratory failures such as bronchial asthma, lung fibrosis, and chronic obstructive pulmonary disease (COPD); and allergic diseases such as anaphylaxis, allergic rhinitis, allergic conjunctivitis, atopic dermatitis, and urticaria. In addition, diseases on which the medicine of the present invention effectively acts are diseases the crisis and evolution of which are considered to be attributed to β-tryptase, such as: an inflammatory bowel disease; a hyperproliferative skin disease; vascular edema; and rheumatoid arthritis. Particularly, diseases on which the medicine of the present invention effectively acts are bronchial asthma and an allergic disease such as atopic dermatitis.

An amine derivative represented by the formula (I) and a pharmaceutically acceptable salt thereof of the present invention (hereinafter, referred to as 'compound of the present invention') are administered alone or in combination with another pharmacologically active ingredient as β-tryptase inhibitors and as prophylactic/therapeutic agents for the above-exemplified diseases. Examples of such a pharmacologically active ingredient include well-known anti-inflammatory agents or other asthma treating agents such as: bronchodilators including β-adrenaline agonists, anticholinergic agents, and xanthine derivatives; adrenocortical steroid hormone agents; thromboxane antagonists; and thromboxane synthesis inhibitors.

The phrase 'used in combination' as used herein includes the case where the compound of the present invention and the pharmacologically active ingredient are administered as separate preparations at the same time or

with a time lag as well as the case where a mixture containing both the compound of the present invention and the pharmacologically active ingredient is administered. A mode of administration is arbitrary as long as the compound of the present invention and the pharmacologically active ingredient are simultaneously present in the blood of a patient.

A medicinal composition containing one or two or more kinds of compounds of the present invention and salts thereof acceptable as medicines is prepared to a capsule, a pill, a tablet, a granule, a fine particle preparation, or a powder in addition to a liquid for internal use such as a suspension, an emulsion, a limonade, an elixir, or a syrup, an injection, a nasal absorbent, a suppository, an ointment, a cataplasm, or the like as well as by using a commonly used carrier or excipient for preparation or another additive. Then, the resultant product is administered perorally or parenterally to human beings and other animals.

The commonly used carrier for preparation is not particularly limited, and examples of the carrier include sterile water, a physiological salt solution, a vegetable oil, a mineral oil, a higher alcohol, a higher fatty acid, and an innocent organic solvent. As required, the examples further include an excipient, a colorant, an emulsifier, a suspension, a surfactant, a dissolution aid, an adsorption preventing agent, a stabilizer, a preservative, a humectant, an anti-oxidant, a buffer, a tension agent, and an analgesic.

A capsule, a pill, a tablet, a powder, a granule, or the like is used for a solid composition for peroral administration according to the present invention. Such a solid composition is prepared by combining one or more active substances with at least one inactive carrier. More specifically, such carriers are excipients (for example, lactose, saccharose, mannitol, glucose, hydroxypropyl cellulose, microcrystalline cellulose, and metasilicate), binding agents (for example, crystalline cellulose, saccharides, dextrin, hydroxypropyl cellulose, hydroxypropyl methylcellulose, polyvinyl pyrrolidone, and a macrogol), lubricants (for example, magnesium stearate, calcium stearate, and talc), disintegrating agents (for example, corn starch, carboxymethyl cellulose, and calcium cellulose glycolate), stabilizers (for example, a sugar alcohol such as lactose and a sugar), solubilizing agents or dissolution aids (for example, cholesterol, triethanolamine, glutamic acid, and aspartic acid), colorants, flavors, antiseptics, tension agents, dispersants, anti-oxidants (for example, ascorbic acid and butylated hydroxyanisole), buffers, or preservatives (for example, paraben and benzyl alcohol). It should be noted that a stomach-soluble or enteric film coating such as sucrose, gelatin, hydroxypropylmethylcellulose, or phthalate may be applied as requied onto a tablet, a pill, a granule, or the like.

Injections for parenteral administration include sterile and aqueous or non-aqueous dissolving agents, suspensions, and emulsifiers. Examples of carriers of aqueous dissolving agents and suspensions include distilled water for injection and a physiological salt solution. Examples of carriers of non-aqueous dissolving agents and suspensions include propylene glycol, polyethylene glycol, a vegetable oil such as an olive oil, alcohols such as ethyl alcohol, and Polysolvate 80 (TM). Such compositions are additives for the tension agents, the antiseptics, the humectants, the emulsifiers, the dispersants, the stabilizer, the solubilizing agents or the dissolution aid, or the like. For example, such compositions are sterilized by filtration with a membrane filter, formulation of a sanitizer, ultraviolet irradiation, or the like. Moreover, such compositions produce sterile solid compositions, and can serve as injections to be used after extemporaneous dissolution, emulsion, or suspension. In the case where the compound of the present invention has low solubility, the compound may be subjected to a solubilizing treatment. Examples of the treatment include well-known methods applicable to medicinal preparations such as a method of adding a surfactant (for example, a polyoxyethylene hydrogenated castor oil, a polyoxyethylene sorbitan higher fatty acid ester, or a sucrose fatty acid ester) and a method of forming a solid dispersing element of a drug with a solubilizing agent, for example, a polymer (such as: a water-soluble polymer such as polyethylene glycol (PEG), hydroxypropyl methylcellulose (HPMC), or polyvinyl pyrrolidone (PVP); or an enteric polymer such as hydroxypropyl methylcellulose phthalate (HPMCP) or a methyl methacrylate-methacrylic acid copolymer (Eudragit L, S (TM); manufactured by Rohm and Haas)). Furthermore, as required, the examples also include a method of forming a clathrate compound by using α-, β-, or γ-cyclodextrin, hydroxypropylcyclodextrin, or the like. Furthermore, an approach to solubilization can be also modified appropriately depending on a target drug with reference to 'Pharmacy Monograph No.1, Biochemical Availability' by Koji Nagai et al., SOFT SCIENCE. Inc., pages 78 to 82, 1988, 'Latest Preparation Technology and Its Application', by Isami Uchiumi et al., Medicine and Drug Journal, pages 157 to 159, 1988, or the like. Of those, a method of forming a solid dispersing element of a drug with a solubilizing agent to improve solubility can be preferably employed (JP 56-49314 A, and FR, 2460667).

A clinical dosage of the compound of the present invention to a human being is appropriately determined in view of the symptom, body weight, age, sex, or the like of a patient to whom the compound is applied. The clinical dosage of an adult is generally 0.1 to 1,000 mg, preferably 1 to 300 mg per day perorally, and is generally 0.01 to 300 mg, preferably 0.1 to 100 mg parenterally. Such an amount of the compound is administered at once or is divided into several portions to be administered. A dosage varies depending on various conditions, so that an amount less than any of the above dosage ranges may suffice.

It should be noted that neither the compound of the present invention represented by the formula (I) nor the salt thereof exhibits toxicity at a dosage where a pharmacological effect can be observed.

In the invention of the present application, a β-tryptase inhibitory activity is 'active' or effective if the activity

is less than 10 μM in terms of $IC_{50}$.
Preferably, the activity is less than 1 μM. Here, $IC_{50}$ represents an inhibitor concentration that shows a suppression rate of 50%.

Examples

Hereinafter, the present invention is decribed in detail by way of examples. However, the present invention is not limited to those examples.

<<Synthesis Example>>

Nuclear magnetic resonance (NMR) spectra of an amine derivative and a pharmaceutically acceptable salt thereof of the present invention were measured by using JEOL JNM-EX 270 FT-NMR ('*' was given on data (Table 4 and Table 5), manufactured by JEOL) or JEOL JNM-LA 300 FT-NMR (manufactured by JEOL). Infrared absorption (IR) spectra thereof were measured by using HORIBA FT-720 (manufactured by HORIBA Ltd.). Melting points thereof were measured by using Mettler FP 80 or FP 90 (both the Mettler FP 80 and FP 90 were manufactured by METTLER TOLEDO).

It should be noted that an anhydrous solvent was used as required in the following examples.

(Example 1) Synthesis of N-[2-[N-[3-[4-(aminomethyl)phenyl]propyl]amino]ethyl]-2-naphthalenesulfohamide

<Step 1> Synthesis of 3-[4-(N-tert-butoxycarbonylaminomethyl)phenyl]propan-1-ol

A solution of diisobutylaluminum hydride in toluene (1.0 M; 210 mL) was slowly dropped in a solution of ethyl 3-[4-(N-tert-butoxycarbonylaminomethyl)phenyl] propionate (12.9 g) in toluene (100 mL) under ice-water cooling. The mixture was warmed to room temperature and was stirred for 3 hours at room temperature. Then, sodium sulfate decahydrate was added to the mixture, followed by stirring at room temperature. The mixture was filtered and washed with ethyl acetate. After that, mother liquor was concentrated under reduced pressure. The resultant residue was purified by means of silica gel column chromatography (eluent; ethyl acetate : hexane=3 : 15 to 3 : 2) to prepare the titled compound (4.0 g, Example Intermediate 1-1).

<Step 2> Synthesis of 3-[4-(N-tert-butoxycarbonylaminomethyl)phenyl]propanal

A solution of oxalyl chloride (2.63 mL) in anhydrous methylene chloride (210 mL) was cooled to -78°C under nitrogen atmosphere, and then a solution of anhydrous dimethyl sulfoxide (2.85 mL) in anhydrous methylene chloride (30 mL) was dropped to the solution for 15 minutes. After the mixture had been stirred for 30 minutes at -78°C, a solution of the compound prepared in Step 1 (4.00 g) in anhydrous methylene chloride (70 mL) was dropped to the mixture for 30 minutes. After the mixture had been stirred for 30 minutes at -78°C, triethylamine (15.32 mL) was added to the mixture at -78°C. The mixture was warmed to room temperature, and water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; ethyl acetate : hexane=1 : 9 to 1 : 1) to prepare the titled compound (3.26 g, Example Intermediate 1-2).

<Step 3> Synthesis of N-[2-[N-[3-[4-(N-tert-butoxycarbonylaminomethyl)phenyl]propyl]amino]ethyl]-2-naphthalenesulfonamide

Added to a solution of the compound prepared in Step 2 (0.15 g) in methylene chloride (3 mL) were N-(2-aminoethyl)-2-naphthalenesulfonamide (0.14 g), Molecular Sieves 3A (powder; 0.15 g), and then acetic acid (33 μL). After the mixture had been stirred under nitrogen atmosphere at room temperature for 30 minutes, sodium triacetoxyborohydride (0.18 g) was added to the mixture, and the whole was stirred overnight at room temperature. Water was added to the mixture, and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; methylene chloride : methanol=100 : 1 to 10 : 1) to prepare the titled compound (0.05 g, Example Intermediate 1-3).

<Step 4> Synthesis of N-[2-[N-[3-[4-(aminomethyl)phenyl]propyl]amino]ethyl]-2-naphthalenesulfonamide

Anisole (26 μL) and trifluoroacetic acid (2.4 mL) were added to the compound prepared in Step 3 (23.9 mg) under ice-water cooling, and the whole was stirred under nitrogen atmosphere at room temperature overnight. Water was added to the mixture, and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; methylene chloride : methanol=100 : 1 to 10 : 1) to prepare the titled compound (11.2 mg).

(Example 2) Synthesis of N-[2-[N-[3-[4-(aminomethyl)phenyl]propyl]amino]ethyl]-2-naphthalenesulfonamide dihydrochloride

A 10% hydrogen chloride in methanol (0.3 mL) was added to the compound prepared in Step 4 of Example 1 (15 mg). The solvent was removed under reduced pressure, and ethyl acetate was added to the resultant residue, followed by removal of the solvent under reduced pressure. Ether was added to the resultant residue, and the whole was filtered to prepare the titled compound (15 mg).

(Example 3) Synthesis of N-[2-[N-[3-[4-(aminomethyl)phenyl]propyl]amino]ethyl]benzenesulfonamide dihydrochloride

<Step 1> Synthesis of N-[2-[N-[3-[4-(N-tert-butoxycarbonylaminomethyl)phenyl]propyl]amino]ethyl]trifluo roacetamide

Added to a solution of the compound prepared in Step 2 of Example 1 (3.03 g) in toluene (100 mL) were N-(2-aminoethyl)trifluoroacetamide hydrochloride (4.43 g) and pyridine (0.93 mL). The solvent was removed under reduced pressure, toluene (80 mL) was added to the resultant residue, and the solvent was removed under reduced pressure, and this operation was repeated twice. Methylene chloride (50 mL) was added to the resultant residue, sodium triacetoxyborohydride (4.87 g) was added to the mixture, and the whole was stirred under nitrogen atmosphere at room temperature overnight. Water was added to the mixture, and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=10 : 1) to prepare the titled compound (1.86 g, Example Intermediate 3-1).

<Step 2> Synthesis of N-[2-[N-tert-butoxycarbonyl-N-[3-[4-(N-tert-butoxycarbonylaminomethyl)phenyl]propyl]amino] ethyl]-trifluoroacetamide

Added to a solution of the compound prepared in Step 1 (1.86 g) in methylene chloride (100 mL) was di-tert-butyl dicarbonate (1.26 g) and the whole was stirred at room temperature overnight. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=20 : 1) to prepare the titled compound (2.15 g, Example Intermediate 3-2).

<Step 3> Synthesis of 2-[N-tert-butoxycarbonyl-N-[3-[4-(N-tert-butoxycarbonylaminomethyl)phenyl]propyl]amino] ethylamine

An aqueous solution (10 mL) of potassium carbonate (3.07 g) was added to a solution of the compound prepared in Step 2 (2.15 g) in methanol (160 mL), followed by heating under reflux for 2 hours. The solvent was removed under reduced pressure, and water was added to the residue, followed by extraction with ethyl acetate. The extract was washed with water and a saturated brine, and was dried over anhydrous sodium sulfate. After that, the solvent was removed under reduced pressure to prepare the titled compound (1.78 g, Example Intermediate 3-3).

<Step 4> Synthesis of N-[2-[N-tert-butoxycarbonyl-N-[3-[4-(N-tert-butoxycarbonylaminomethyl)phenyl]propyl]amino] ethyl]benzenesulfonamide

Added to a solution of the compound prepared in Step 3 (45 g) in methylene chloride (1 mL) were benzenesulfonyl chloride (19 mg) and triethylamine (18 μL) and the whole was stirred at room temperature overnight. Water was added to the mixture and a 1 N aqueous solution of sodium hydroxide was added to alkalize the mixture, followed

by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; ethyl acetate : hexane=1 : 2) to prepare the titled compound (13 mg, Example Intermediate 3-4).

<Step 5> Synthesis of N-[2-[N-[3-[4-(aminomethyl)phenyl]propyl]amino]ethyl]benzenesulfonamide dihydrochloride

A 3 N hydrogen chloride in ethyl acetate (0.16 mL) was added to a solution of the compound prepared in Step 4 (13 mg) in ethyl acetate (1 mL), and the whole was stirred at room temperature for 2 hours. Ether (1.0 mL) was added to the mixture, and the whole was filtered to prepare the titled compound (5.9 mg).

(Example 4) Synthesis of N-[2-[N-[3-[4-(aminomethyl)phenyl]propyl]amino]ethyl]-(1-chloro-2-naphthalene) sulfonamide dihydrochloride

<Step 1> Synthesis of N-[2-[N-tert-butoxycarbonyl-N-[3-[4-(N-tert-butoxycarbonylaminomethyl)phenyl]propyl]amino] ethyl]-(1-chloro-2-naphthalene)sulfonamide

A reaction was carried out in the same manner as in Step 4 of Example 3 by using the compound prepared in Step 3 of Example 3 (10.0 mg) and (1-chloro-2-naphthalene)sulfonyl chloride (7.7 mg) to prepare the titled compound (13.5 mg, Example Intermediate 4-1).

<Step 2> Synthesis of N-[2-[N-[3-[4-(aminomethyl)phenyl]propyl]amino]ethyl]-(1-chloro-2-naphthalene)sulfonamide dihydrochloride

A reaction was carried out in the same manner as in Step 5 of Example 3 by using the compound prepared in Step 1 (13.5 mg) to prepare the titled compound (10.0 mg).

(Example 5) Synthesis of N-[2-[N-[3-[4-(aminomethyl)phenyl]propyl]amino]ethyl]-5-benzo[b]thiophenesulfonamide dihydrochloride

<Step 1> Synthesis of N-[2-[N-tert-butoxycarbonyl-N-[3-[4-(N-tert-butoxycarbonylaminomethyl)phenyl]propyl]amino] ethyl]-5-benzo[b]thiophenesulfonamide

A reaction was carried out in the same manner as in Step 4 of Example 3 by using the compound prepared in Step 3 of Example 3 (7.1 mg) and 5-benzo[b]thiophenesulfonyl chloride (4.1 mg) to prepare the titled compound (9.2 mg, Example Intermediate 5-1).

<Step 2> Synthesis of N-[2-[N-[3-[4-(aminomethyl)phenyl]propyl]amino]ethyl]-5-benzo[b]thiophenesulfonamide dihydrochloride

A reaction was carried out in the same manner as in Step 5 of Example 3 by using the compound prepared in Step 1 (9.2 mg) to prepare the titled compound (6.5 mg).

(Example 6) Synthesis of N-[2-[N-[3-[4-(aminomethyl)phenyl]propyl]amino]ethyl]-(2-methyl-6-benzothiazole) sulfonamide dihydrochloride

<Step 1> Synthesis of N-[2-[N-tert-butoxycarbonyl-N-[3-[4-(N-tert-butoxycarbonylaminomethyl)phenyl]propyl]amino] ethyl]-(2-methyl-6-benzothiazole)sulfonamide

A reaction was carried out in the same manner as in Step 4 of Example 3 by using the compound prepared in Step 3 of Example 3 (10.0 mg) and (2-methylbenzothiazole)-6-sulfonyl chloride (6.6 mg) to prepare the titled compound (11.0 mg, Example Intermediate 6-1).

<Step 2> Synthesis of N-[2-[N-[3-[4-(aminomethyl)phenyl]propyl]amino]ethyl]-(2-methyl-6-benzothiazole)sulfonamide dihydrochloride

A reaction was carried out in the same manner as in Step 5 of Example 3 by using the compound prepared in Step 1 (11 mg) to prepare the titled compound (10 mg).

(Example 7) Synthesis of N-[2-[N-[3-[4-(aminomethyl)phenyl]propyl]amino]ethyl]-5-indanesulfonamide dihydrochloride

<Step 1> Synthesis of N-[2-[N-tert-butoxycarbonyl-N-[3-[4-(N-tert-butoxycarbonylaminomethyl)phenyl]propyl]amino]ethyl]-5-indanesulfonamide

A reaction was carried out in the same manner as in Step 4 of Example 3 by using the compound prepared in Step 3 of Example 3 (31.0 mg) and 5-indanesulfonyl chloride (16.5 mg) to prepare the titled compound (29.1 mg, Example Intermediate 7-1).

<Step 2> Synthesis of N-[2-[N-[3-[4-(aminomethyl)phenyl]propyl]amino]ethyl]-5-indanesulfonamide dihydrochloride

A reaction was carried out in the same manner as in Step 5 of Example 3 by using the compound prepared in Step 1 (25.0 mg) to prepare the titled compound (12.9 mg).

(Example 8) Synthesis of N-[2-[N-[3-[4-(aminomethyl)phenyl]propyl]amino]ethyl]-2-indanesulfonamide dihydrochloride

<Step 1> Synthesis of N-[2-[N-tert-butoxycarbonyl-N-[3-[4-(N-tert-butoxycarbonylaminomethyl)phenyl]propyl]amino]ethyl]-2-indanesulfonamide

A reaction was carried out in the same manner as in Step 4 of Example 3 by using the compound prepared in Step 3 of Example 3 (33.0 mg) and 2-indanesulfonyl chloride (17.5 mg) to prepare the titled compound (22.0 mg, Example Intermediate 8-1).

<Step 2> Synthesis of N-[2-[N-[3-[4-(aminomethyl)phenyl]propyl]amino]ethyl]-2-indanesulfonamide dihydrochloride

A reaction was carried out in the same manner as in Step 5 of Example 3 by using the compound prepared in Step 1 (20.0 mg) to prepare the titled compound (8.7 mg).

(Example 9) Synthesis of N-[2-[N-[3-[4-(aminomethyl)phenyl]propyl]amino]ethyl]-(1,2,3,4-tetrahydro-7-isoquinoline)sulfonamide trihydrochloride

<Step 1> Synthesis of N-[2-[N-tert-butoxycarbonyl-N-[3-[4-(N-tert-butoxycarbonylaminomethyl)phenyl]propyl]amino]ethyl]-[1,2,3,4-tetrahydro-2-(trifluoroacetyl)-7-isoquinoline]sulfonamide

A reaction was carried out in the same manner as in Step 4 of Example 3 by using the compound prepared in Step 3 of Example 3 (50.0 mg) and [(1,2,3,4-tetrahydro-2-(trifluoroacetyl)isoquinoline)]-7-sulfonyl chloride (44.2 mg) to prepare the titled compound (70.0 mg, Example Intermediate 9-1).

<Step 2> Synthesis of N-[2-[N-tert-butoxycarbonyl-N-[3-[4-(N-tert-butoxycarbonylaminomethyl)phenyl]propyl]amino]ethyl]-(1,2,3,4-tetrahydro-7-isoquinoline)sulfonamide

An aqueous solution (0.2 mL) of potassium carbonate (12.4 mg) was added to a solution of the compound prepared in Step 1 (12.5 mg) in methanol (2 mL) and the whole was stirred at room temperature for 1 hour. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; methylene chloride : methanol=30 : 1) to prepare the titled compound (9.7 mg, Example Intermediate 9-2).

<Step 3> Synthesis of N-[2-[N-[3-[4-(aminomethyl)phenyl]propyl]amino]ethyl]-(1,2,3,4-tetrahydro-7-isoquinoline)sulfonamide trihydrochloride

A reaction was carried out in the same manner as in Step 5 of Example 3 by using the compound prepared in Step 2 (9.7 mg) to prepare the titled compound (7.3 mg).

(Example 10) Synthesis of N-[2-[N-[3-[4-(aminomethyl)phenyl]propyl]amino]ethyl]benzenecarboxamide dihydrochloride

<Step 1> Synthesis of N-[2-[N-tert-butoxycarbonyl-N-[3-[4-(N-tert-butoxycarbonylaminomethyl)phenyl]propyl]amino] ethyl]benzene-carboxamide

Added to a solution of benzoic acid (17.4 mg) in methylene chloride (1 mL) were 1-ethyl-3-(3-dimethylaminopropyl)carbodiimide hydrochloride (34.1 mg) and the compound prepared in Step 3 of Example 3 (29.0 mg) and the whole was stirred at room temperature overnight. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; ethyl acetate : hexane=1 : 2 to 1 : 1) to prepare the titled compound (24.6 mg, Example Intermediate 10-1).

<Step 2> Synthesis of N-[2-[N-[3-[4-(aminomethyl)phenyl]propyl]amino]ethyl]benzenecarboxamide dihydrochloride

A reaction was carried out in the same manner as in Step 5 of Example 3 by using the compound prepared in Step 1 (20 mg) to prepare the titled compound (11 mg).

(Example 11) Synthesis of N-[2-[N-[3-[4-(aminomethyl)phenyl]propyl]amino]ethyl]-2-naphthalenecarboxamide

<Step 1> Synthesis of N-[2-[N-tert-butoxycarbonyl-N-[3-[4-(N-tert-butoxycarbonylaminomethyl)phenyl]propyl]amino] ethyl]-2-naphthalenecarboxamide

Naphthalene-2-carboxylic acid (31.8 mg) was suspended in methylene chloride (0.5 mL), and thionyl chloride (0.3 mL) was added to the suspension, followed by stirring at room temperature overnight. The solvent was removed under reduced pressure, and the resultant residue was dissolved in methylene chloride (1 mL). The above acid chloride solution was added to a solution of the compound prepared in Step 3 of Example 3 (50 mg) in methylene chloride (1 mL). Furthermore, pyridine (0.5 mL) was added to the mixture, and the whole was stirred at room temperature for 30 minutes. 0.5 N hydrochloric acid was added to the mixture, followed by extraction with methylene chloride. The extract was washed with a 1 N aqueous solution of sodium hydroxide, water, and a saturated brine, and was dried over anhydrous sodium sulfate, followed by removal of the solvent under reduced pressure. The resultant residue was purified by means of silica gel column chromatography (eluent; methylene chloride to methylene chloride : methanol=20 : 1) to prepare the titled compound (24.6 mg, Example Intermediate 11-1).

<Step 2> Synthesis of N-[2-[N-[3-[4-(aminomethyl)phenyl]propyl]amino]ethyl]-2-naphthalenecarboxamide

A reaction was carried out in the same manner as in Step 4 of Example 1 by using the compound prepared in Step 1 (24.6 mg) to prepare the titled compound (16.5 mg).

(Example 12) Synthesis of N-[2-[N-[3-[4-(aminomethyl)phenyl]propyl]amino]ethyl]-1-naphthalenecarboxamide

<Step 1> Synthesis of N-[2-[N-tert-butoxycarbonyl-N-[3-[4-(N-tert-butoxycarbonylaminomethyl)phenyl]propyl]amino] ethyl]-1-naphthalenecarboxamide

A reaction was carried out in the same manner as in Step 1 of Example 10 by using the compound prepared in Step 3 of Example 3 (45.0 mg) and naphthalene-1-carboxylic acid (20.8 mg) to prepare the titled compound (29.7 mg, Example Intermediate 12-1).

<Step 2> Synthesis of N-[2-[N-[3-[4-(aminomethyl)phenyl]propyl]amino]ethyl]-1-naphthalenecarboxamide

A reaction was carried out in the same manner as in Step 4 of Example 1 by using the compound prepared in Step 1 (29.7 mg) to prepare the titled compound (17.7 mg).

(Example 13) Synthesis of N-[2-[N-[3-[4-(aminomethyl)phenyl]propyl]amino]ethyl]-6-quinolinecarboxamide trihydrochloride

<Step 1> Synthesis of N-[2-[N-tert-butoxycarbonyl-N-[3-[4-(N-tert-butoxycarbonylaminomethyl)phenyl]propyl]amino] ethyl]-6-quinolinecarboxamide

A reaction was carried out in the same manner as in Step 1 of Example 10 by using the compound prepared in Step 3 of Example 3 (25.0 mg) and quinoline-6-carboxylic acid (17.4 mg) to prepare the titled compound (30.6 mg, Example Intermediate 13-1).

<Step 2> Synthesis of N-[2-[N-[3-[4-(aminomethyl)phenyl]propyl]amino]ethyl]-6-quinolinecarboxamide trihydrochloride

A reaction was carried out in the same manner as in Step 5 of Example 3 by using the compound prepared in Step 1 (30.0 mg) to prepare the titled compound (19.6 mg).

(Example 14) Synthesis of N-[2-[N-[3-[4-(aminomethyl)phenyl]propyl]amino]ethyl]-(4-phenoxybenzene)carboxamide

<Step 1> Synthesis of N-[2-(N-tert-butoxycarbonyl-N-[3-[4-(N-tert-butoxycarbonylaminomethyl)phenyl]propyl]amino] ethyl]-(4-phenoxybenzene)carboxamide

A reaction was carried out in the same manner as in Step 1 of Example 10 by using the compound prepared in Step 3 of Example 3 (45.0 mg) and 4-phenoxybenzoic acid (25.9 mg) to prepare the titled compound (36.7 mg, Example Intermediate 14-1).

<Step 2> Synthesis of N-[2-[N-[3-[4-(aminomethyl)phenyl]propyl]amino]ethyl]-(4-phenoxybenzene)carboxamide

A reaction was carried out in the same manner as in Step 1 of Example 4 by using the compound prepared in Step 1 (36.7 mg) to prepare the titled compound (24.0 mg).

(Example 15) Synthesis of N-[2-[N-[3-[4-(aminomethyl)phenyl]propyl]amino]ethyl]phenoxyacetamide dihydrochloride

<Step 1> Synthesis of N-[2-[N-tert-butoxycarbonyl-N-[3-[4-(N-tert-butoxycarbonylaminomethyl)phenyl]propyl]amino] ethyl] phenoxyacetamide

A reaction was carried out in the same manner as in Step 1 of Example 10 by using the compound prepared in Step 3 of Example 3 (47 mg) and phenoxyacetic acid (35 mg) to prepare the titled compound (45 mg, Example Intermediate 15-1).

<Step 2> Synthesis of N-[2-[N-[3-[4-(aminomethyl)phenyl]propyl]amino]ethyl]phenoxyacetamide dihydrochloride

A reaction was carried out in the same manner as in Step 5 of Example 3 by using the compound prepared in Step 1 (40.0 mg) to prepare the titled compound (18.6 mg).

(Example 16) Synthesis of N-[2-[N-[3-[4-(aminomethyl)phenyl]propyl]amino]ethyl]-N-methyl-2-naphthalenesulfonamide dihydrochloride

<Step 1> Synthesis of lithium 3-[4-(N-tert-butoxycarbonylaminomethyl)phenyl]propionate

An aqueous solution (7 mL) of lithium hydroxide monohydrate (0.55 g) was added to a solution of ethyl 3-[4-(N-tert-butoxycarbonylaminomethyl)phenyl]propionate (4.06 g) in methanol (17 mL), followed by stirring under heating for 20 minutes at 60°C. The solvent was removed under reduced pressure. Toluene was added to the resultant residue, and the solvent was removed under reduced pressure. Hexane was added to the resultant residue, and the whole was filtered to prepare the titled compound (3.38 g, Example Intermediate 16-1).

<Step 2> Synthesis of N-[2-[N-[3-[4-(N-tert-butoxycarbonylaminomethyl)phenyl]propanoyl]amino]ethyl]-N-methyl-2-naphthalenesulfonamide

Added to a suspension of the compound prepared in Step 1 (2.37 g) in methylene chloride (100 mL) were a solution of 1M hydrochloric acid-ether (8.31 mL), 1-ethyl-3-(3-dimethylaminopropyl)carbodiimide hydrochloride (1.59 g), and N-(2-aminoethyl)-N-methyl-2-naphthalenesulfonamide (2.08 g), and the whole was stirred at room temperature for 1 hour. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; methylene chloride : methanol=100 : 1 to 10 : 1) to prepare the titled compound (2.02 g, Example Intermediate 16-2).

<Step 3> Synthesis of N-[2-[N-[3-[4-(aminomethyl)phenyl]propyl]amino]ethyl]-N-methyl-2-naphthalenesulfonamide dihydrochloride

A tetrahydrofuran solution of a boranetetrahydrofuran complex (1 M; 1.3 mL) was added to a solution of the compound prepared in Step 2 (69.0 mg) in tetrahydrofuran (3.5 mL), followed by stirring at room temperature overnight. Methanol (1 mL) was added to the mixture under ice-water cooling, and then a 10% hydrogen chloride in methanol (1 mL) was added to the mixture, followed by stirring for 1 hour at room temperature. The solvent was removed under reduced pressure, and water and a 1 N aqueous solution of sodium hydroxide were added to the residue to alkalize the residue, followed by extraction with methylene chloride. The extract was washed with water and a saturated brine, and was dried over anhydrous sodium sulfate, followed by removal of the solvent under reduced pressure. The resultant residue was dissolved in 1,4-dioxane (1 mL), and di-tert-butyl dicarbonate (190.0 mg) and an aqueous solution of sodium carbonate (1 M; 50 μL) were added to the solution, followed by stirring at 40°C for 1 hour. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; ethyl acetate : hexane=1 : 1). The resultant compound was treated in the same manner as in Step 5 of Example 3 to prepare the titled compound (4.0 mg).

(Example 17) Synthesis of N-[2-[N-[3-[4-(aminomethyl)phenyl]propyl]-N-methylamino]ethyl]-2-naphthalenesulfonamide

<Step 1> Synthesis of N-[2-[N-[3-[4-(N-tert-butoxycarbonylaminomethyl)phenyl]propyl]-N-methylamino]ethyl]-2-naphthalenesulfonamide

Added to a solution of the compound prepared in Step 3 of Example 1 (50.0 mg) and paraformaldehyde (90%; 8.3 mg) in chloroform (0.8 mL) was acetic acid (23 μL). After the mixture had been stirred under nitrogen atmosphere at room temperature for 30 minutes, sodium triacetoxyborohydride (115.6 mg) was added to the mixture, and the whole was stirred at 60°C for 2 hours. Water was added to the mixture, and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; hexane : ethyl acetate=2 : 1) to prepare the titled compound (10.9 mg, Example Intermediate 17-1).

<Step 2> Synthesis of N-[2-[N-[3-[4-(aminomethyl)phenyl]propyl]-N-methylamino]ethyl]-2-naphthalenesulfonamide

A reaction was carried out in the same manner as in Step 4 of Example 1 by using the compound prepared in Step 1 (10.9 mg) to prepare the titled compound (8.8 mg).

(Example 18) Synthesis of N-[2-[N-[3-[4-(aminomethyl)phenyl]propyl]-N-ethylamino]ethyl]-2-naphthalenesulfonamide

<Step 1> Synthesis of N-[2-[N-[3-[4-(N-tert-butoxycarbonylaminomethyl)phenyl]propyl]-N-ethylamino]ethyl]-2-naphthalenesulfonamide

Added to a solution of the compound prepared in Step 3 of Example 1 (50.0 mg) in methylene chloride (3 mL) were acetaldehyde (0.20 mL), Molecular Sieves 3A (powder; 0.20 g), and then acetic acid (19 μL). After the mixture had been stirred under nitrogen atmosphere at room temperature for 30 minutes, sodium triacetoxyborohydride (174.0

mg) was added to the mixture, and the whole was stirred at room temperature for 1.5 hours. Water was added to the mixture, and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; ethyl acetate to ethyl acetate : methanol=98 : 2) to prepare the titled compound (94.2 mg, Example Intermediate 18-1).

<Step 2> Synthesis of N-[2-[N-[3-[4-(aminomethyl)phenyl]propyl]-N-ethylamino]ethyl]-2-naphthalenesulfonamide

A reaction was carried out in the same manner as in Step 4 of Example 1 by using the compound prepared in Step 1 (90.0 mg) to prepare the titled compound (42.2 mg).

(Example 19) Synthesis of N-[2-[N-acetyl-N-[3-[4-(aminomethyl)phenyl]propyl]amino]ethyl]-2-naphthalenesulfonamide

<Step 1> Synthesis of N-[2-[N-acetyl-N-[3-[4-(N-tert-butoxycarbonylaminomethyl)phenyl]propyl]amino]ethyl]-2-naphthalenesulfonamide

Added to a solution of the compound prepared in Step 3 of Example 1 (0.15 g) in methylene chloride (6 mL) was triethylamine (46 μL). Then, acetyl chloride (22.5 μL) was added to the mixture under ice-water cooling, and the whole was stirred at room temperature for 10 minutes. The mixture was poured to water, followed by extraction with methylene chloride. The extract was washed with water and a saturated brine, and was dried over anhydrous sodium sulfate, followed by removal of the solvent under reduced pressure. The resultant residue was purified by means of silica gel column chromatography [Chromatorex NH™] (eluent; methylene chloride to methylene chloride : methanol=49 : 1) to prepare the titled compound (0.13 g, Example Intermediate 19-1).

<Step 2> Synthesis of N-[2-[N-acetyl-N-[3-[4-(aminomethyl)phenyl]propyl]amino]ethyl]-2-naphthalenesulfonamide

A reaction was carried out in the same manner as in Step 4 of Example 1 by using the compound prepared in Step 1 (100.0 mg) to prepare the titled compound (59.3 mg).

(Example 20) Synthesis of N-[2-[N-[3-[4-(aminomethyl)phenyl]propyl]-N-methylamino]ethyl]-(1-chloro-2-naphthalene) sulfonamide

<Step 1> Synthesis of N-(9-fluorenylmethoxycarbonyl)-N-[2-[N-[3-[4-(N-tert-butoxycarbonylaminomethyl)phenyl] propyl]-N-methylamino]ethyl]amine

Added to a solution of the compound prepared in Step 2 of Example 1 (2.8 g) in anhydrous methylene chloride (15 mL) were N-(9-fluorenylmethoxycarbonyl)-N-[2-(methylamino)ethyl]amine hydrochloride (3.6 g) and then sodium triacetoxyborohydride (4.6 g). The whole was stirred in a nitrogen atmosphere at room temperature overnight. An aqueous saturated sodium bicarbonate solution was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=20 : 1) to prepare the titled compound (4.1 g, Example Intermediate 20-1).

<Step 2> Synthesis of 2-[N-[3-[4-(N-tert-butoxycarbonylaminomethyl)phenyl]propyl]-N-methylamino]ethylamine

Added to the compound prepared in Step 1 (4.1 g) was morpholine (20 mL), and the whole was stirred at room temperature for 4 hours. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=3 : 1 to 1 : 1) to prepare the titled compound (1.5 g, Example Intermediate 20-2).

<Step 3> Synthesis of N-[2-[N-[3-[4-(N-tert-butoxycarbonylaminomethyl)phenyl]propyl]-N-methylamino]ethyl] -(1-chloro-2-naphthalene)sulfonamide

A reaction was carried out in the same manner as in Step 4 of Example 3 by using the compound prepared in Step 2 (53.4 mg) and (1-chloro-2-naphthalene)sulfonyl chloride (43.3 mg) to prepare the titled compound (76.7 mg, Example Intermediate 20-3).

<Step 4> Synthesis of N-[2-[N-[3-[4-(aminomethyl)phenyl]propyl]-N-methylamino]ethyl]-(1-chloro-2-naphthalene) sulfonamide dihydrochloride

A reaction was carried out in the same manner as in Step 5 of Example 3 by using the compound prepared in Step 3 (76 mg) to prepare the titled compound (45 mg).

(Example 21) Synthesis of N-[2-[N-[3-[4-(aminomethyl)phenyl]propyl]-N-methylamino]ethyl]-(2-chlorobenzene) sulfonamide dihydrochloride

<Step 1> Synthesis of N-[2-[N-[3-[4-(N-tert-butoxycarbonylaminomethyl)phenyl]propyl]-N-methylamino]ethyl] -(2-chlorobenzene)sulfonamide

A reaction was carried out in the same manner as in Step 4 of Example 3 by using the compound prepared in Step 2 of Example 20 (50.0 mg) and (2-chlorobenzene)sulfonyl chloride (32.8 mg) to prepare the titled compound (57.9 mg, Example Intermediate 21-1).

<Step 2> Synthesis of N-[2-[N-[3-[4-(aminomethyl)phenyl]propyl]-N-methylamino]ethyl]-(2-chlorobenzene) sulfonamide dihydrochloride

A reaction was carried out in the same manner as in Step 5 of Example 3 by using the compound prepared in Step 1 (50.0 mg) to prepare the titled compound (43.0 mg).

(Example 22) Synthesis of N-[2-[N-[3-[4-(aminomethyl)phenyl]propyl]-N-methylamino]ethyl]-(2-bromobenzene) sulfonamide dihydrochloride

<Step 1> Synthesis of N-[2-[N-[3-[4-(N-tert-butoxycarbonylaminomethyl)phenyl]propyl]-N-methylamino]ethyl] -(2-bromobenzene)sulfonamide

A reaction was carried out in the same manner as in Step 4 of Example 3 by using the compound prepared in Step 2 of Example 20 (50.0 mg) and (2-bromobenzene)sulfonyl chloride (39.7 mg) to prepare the titled compound (70.2 mg, Example Intermediate 22-1).

<Step 2> Synthesis of N-[2-[N-[3-[4-(aminomethyl)phenyl]propyl]-N-methylamino]ethyl]-(2-bromobenzene) sulfonamide dihydrochloride

A reaction was carried out in the same manner as in Step 5 of Example 3 by using the compound prepared in Step 1 (60.0 mg) to prepare the titled compound (40.0 mg).

(Example 23) Synthesis of N-[2-[N-[3-[4-(aminomethyl)phenyl]propyl]-N-methylamino]ethyl]-(2,3,4-trichlorobenzene) sulfonamide dihydrochloride

<Step 1> Synthesis of N-[2-[N-[3-[4-(N-tert-butoxycarbonylaminomethyl)phenyl]propyl]-N-methylamino]ethyl] -(2,3,4-trichlorobenzene)sulfonamide

A reaction was carried out in the same manner as in Step 4 of Example 3 by using the compound prepared in Step 2 of Example 20 (50.0 mg) and (2,3,4-trichlorobenzene)sulfonyl chloride (43.5 mg) to prepare the titled compound (74.6 mg, Example Intermediate 23-1).

<Step 2> Synthesis of N-[2-[N-[3-[4-(aminomethyl)phenyl]propyl]-N-methylamino]ethyl]-(2,3,4-trichlorobenzene) sulfonamide dihydrochloride

A reaction was carried out in the same manner as in Step 5 of Example 3 by using the compound prepared in Step 1 (60.0 mg) to prepare the titled compound (42.0 mg).

(Example 24) Synthesis of N-[2-[N-[3-[3-fluoro-4-(aminomethyl)phenyl]propyl]-N-methylamino]ethyl]-2-naphthalenesulfonamide dihydrochloride

<Step 1> Synthesis of ethyl 4-cyano-3-fluorocinnamate

Dichlorobis(triphenylphosphine)palladium(II) (0.66 g) and potassium carbonate (5.21 g) were added to a solution of 4-bromo-2-fluorobenzonitrile (3.77 g) and ethyl acrylate (7.55 g) in N,N-dimethylformamide (40 mL), and the whole was stirred at 90°C for 1 hour. The solvent was removed under reduced pressure, and the resultant residue was purified by means of silica gel column chromatography (eluent; ethyl acetate : hexane=2 : 1) to prepare the titled compound (3.91 g, Example Intermediate 24-1).

<Step 2> Synthesis of 3-[4-(N-tert-butoxycarbonylaminomethyl)-3-fluorophenyl]propan-1-ol

Cobalt chloride hexahydrate (0.24 g) was added to a solution of the compound prepared in Step 1 (3.73 g) in ethanol (150 mL). Sodium borohydride (2.7 g) was gradually added to the mixture under ice-water cooling, and the whole was stirred at room temperature for 1 hour. Furthermore, sodium borohydride (2.7 g) was gradually added to the mixture, and the whole was stirred at room temperature overnight. The solvent was removed under reduced pressure, and water was added to the solution, followed by extraction with methylene chloride. The extract was washed with water and a saturated brine, and was dried over anhydrous sodium sulfate, followed by removal of the solvent under reduced pressure. The resultant residue was dissolved in ethanol (130 mL), cobalt chloride hexahydrate (0.21 g) was added to the solution, and sodium borohydride (4.7 g) was gradually added to the solution under ice-water cooling, followed by heating under reflux for 4 hours. The solvent was removed under reduced pressure, and water was added to the solution, followed by extraction with methylene chloride. The extract was washed with water and a saturated brine, and was dried over anhydrous sodium sulfate, followed by removal of the solvent under reduced pressure.

The resultant residue was dissolved in dioxane (50 mL), and a solution of di-tert-butyl dicarbonate (2.0 g) and sodium carbonate (2.92 g) in water (30 mL) was added to the solution, followed by stirring at 40°C for 1 hour. The solvent was removed under reduced pressure, and water was added to the resultant residue, followed by extraction with ethyl acetate. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=20 : 1) to prepare the titled compound (1.46 g, Example Intermediate 24-2).

<Step 3> Synthesis of 3-[4-(N-tert-butoxycarbonylaminomethyl)-3-fluorophenyl]propanal

A reaction was carried out in the same manner as in Step 2 of Example 1 by using the compound prepared in Step 2 (1.46 g) to prepare the titled compound (0.86 g, Example Intermediate 24-3).

<Step 4> Synthesis of N-(9-fluorenylmethoxycarbonyl)-N-[2-[N-[3-[4-(N-tert-butoxycarbonylaminomethyl)-3-fluorophenyl]propyl]-N-methylamino]ethyl]amine

A reaction was carried out in the same manner as in Step 1 of Example 20 by using the compound prepared in Step 3 (0.60 g) and N-(9-fluorenylmethoxycarbonyl)-N-[2-(methylamino)ethylamine hydrochloride (0.71 g) to prepare the titled compound (0.99 g, Example Intermediate 24-4).

<Step 5> Synthesis of 2-[N-[3-[4-(N-tert-butoxycarbonylaminomethyl)-3-fluorophenyl]propyl]-N-methylamino] ethylamine

A reaction was carried out in the same manner as in Step 2 of Example 20 by using the compound prepared in Step 4 (690 mg) to prepare the titled compound (347 mg, Example Intermediate 24-5).

<Step 6> Synthesis of N-[2-[N-[3-[4-(N-tert-butoxycarbonylaminomethyl)-3-fluorophenyl]propyl]-N-methylamino] ethyl]-2-naphthalenesulfonamide

A reaction was carried out in the same manner as in Step 4 of Example 3 by using the compound prepared in Step 5 (46.7 mg) and 2-naphthalenesulfonyl chloride (31.2 mg) to prepare the titled compound (39.3 mg, Example Intermediate 24-6).

<Step 7> Synthesis of N-[2-[N-[3-[4-(aminomethyl)-3-fluorophenyl]propyl]-N-methylamino]ethyl]-2-naphthalenesulfonamide dihydrochloride

A reaction was carried out in the same manner as in Step 5 of Example 3 by using the compound prepared in Step 6 (38.0 mg) to prepare the titled compound (27.2 mg).

(Example 25) Synthesis of N-[2-[N-[3-[4-(aminomethyl)phenyl]propyl]-N-methylamino]ethyl]-(2-chlorobenzene) carboxamide dihydrochloride

<Step 1> Synthesis of N-[2-[N-[3-[4-(N-tert-butoxycarbonylaminomethyl)phenyl]propyl]-N-methylamino]ethyl] -(2-chlorobenzene)carboxamide

Added to a solution of the compound prepared in Step 2 of Example 20 (50.0 mg) in methylene chloride (5 mL) were 2-chlorobenzoyl chloride (34.2 mg) and triethylamine (22.8 μL) and the whole was stirred at room temperature overnight. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; hexane : ethyl acetate=2 : 1 to 1 : 2) to prepare the titled compound (50.4 mg, Example Intermediate 25-1).

<Step 2> Synthesis of N-[2-[N-[3-[4-(aminomethyl)phenyl]propyl]-N-methylamino]ethyl]-(2-chlorobenzene) carboxamide dihydrochloride

A reaction was carried out in the same manner as in Step 5 of Example 3 by using the compound prepared in Step 1 (45.0 mg) to prepare the titled compound (32.0 mg).

(Example 26) Synthesis of N-[2-[N-[3-[4-(aminomethyl)phenyl]propyl]-N-methylamino]ethyl]-(2-bromobenzene) carboxamide dihydrochloride

<Step 1> Synthesis of N-[2-[N-[3-[4-(N-tert-butoxycarbonylaminomethyl)phenyl]propyl]-N-methylamino]ethyl] -(2-bromobenzene)carboxamide

A reaction was carried out in the same manner as in Step 1 of Example 25 by using the compound prepared in Step 2 of Example 20 (50.0 mg) and 2-bromobenzoyl chloride (34.2 mg) to prepare the titled compound (41.3 mg, Example Intermediate 26-1).

<Step 2> Synthesis of N-[2-[N-[3-[4-(aminomethyl)phenyl]propyl]-N-methylamino]ethyl]-(2-bromobenzene) carboxamide dihydrochloride

A reaction was carried out in the same manner as in Step 5 of Example 3 by using the compound prepared in Step 1 (40.0 mg) to prepare the titled compound (26.0 mg).

(Example 27) Synthesis of N-[2-[N-[3-[4-(aminomethyl)phenyl]propyl]amino]ethyl]-N-(2-naphthylmethyl)amine trihydrochloride

<Step 1> Synthesis of N-[2-[N-tert-butoxycarbonyl-N-[3-[4-(N-tert-butoxycarbonylaminomethyl)phenyl]propyl]amino] ethyl]-N-(2-naphthylmethyl)amine

2-naphthalenecarbaldehyde (20.1 mg) was added to a solution of the compound prepared in Step 3 of Example 3 (50 mg) in toluene (3 mL) and the solvent was removed under reduced pressure, and this operation was repeated 5 times in total. The resultant residue was dissolved in methanol (1 mL), sodium borohydride (92.7 mg) was added to the solution, and the whole was stirred at room temperature for 2 hours. Water was added to the mixture,

and a 1 N aqueous solution of sodium hydroxide was added to the mixture to alkalize the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; methylene chloride : methanol=100 : 1 to 100 : 8) to prepare the titled compound (12.3 mg, Example Intermediate 27-1).

<Step 2> Synthesis of N-[2-[N-[3-[4-(aminomethyl)phenyl]propyl]amino]ethyl]-N-(2-naphthylmethyl)amine trihydrochloride

A reaction was carried out in the same manner as in Step 5 of Example 3 by using the compound prepared in Step 1 (12.3 mg) to prepare the titled compound (9.8 mg).

(Example 28) Synthesis of N-[2-[N-[3-[4-(aminomethyl)phenyl]propyl]amino]ethyl]-2-benzo[b]thiophenesulfonamide dihydrochloride

<Step 1> Synthesis of N-(2-aminoethyl)-2-benzo[b]thiophenesulfonamide hydrochloride

Benzo[b]thiophene-2-sulfonyl chloride (0.80 g) was slowly dropped in a solution of ethylenediamine (0.62 g) in methylene chloride (5 mL) for 20 minutes, and the whole was stirred at room temperature for 1 hour. A saturated aqueous solution of sodium hydrogen carbonate was added to the mixture to alkalize the solution, followed by extraction with methylene chloride. The extract was washed with water and a saturated brine, and was dried over anhydrous sodium sulfate, followed by removal of the solvent under reduced pressure. The resultant residue was dissolved in ethyl acetate (10 mL), and a saturated hydrogen chloride in methanol (10 mL) was added to the solution, followed by stirring at room temperature for 2 hours. The precipitate was filtered off to prepare the titled compound (0.88 g, Example Intermediate 28-1).

<Step 2> Synthesis of N-[2-[N-[3-[4-(N-tert-butoxycarbonylaminomethyl)phenyl]propanoyl]amino]ethyl]-2-benzo[b]thiophenesulfonamide

Added to a methylene chloride (10 mL) suspension of the compound prepared in Step 1 (0.21 g) and the compound prepared in Step 1 of Example 16 (0.20 g) was 1-ethyl-3-(3-dimethylaminopropyl)carbodiimide hydrochloride (0.15 g), and the whole was stirred at room temperature overnight. Water was added to the mixture, followed by extraction with methylene chloride. The extract was washed with water and a saturated brine, and was dried over anhydrous sodium sulfate, followed by removal of the solvent under reduced pressure. The resultant residue was purified by means of silica gel column chromatography (eluent; hexane : ethyl acetate=1 : 1 to 2 : 3) to prepare the titled compound (0.17 g, Example Intermediate 28-2).

<Step 3> Synthesis of N-[2-[N-[3-[4-(N-tert-butoxycarbonylaminomethyl)phenyl]propyl]amino]ethyl]-2-benzo[b]thiophenesulfonamide

A tetrahydrofuran solution of a boranetetrahydrofuran complex (1 M; 3.1 mL) was added to a solution of the compound prepared in Step 2 (0.16 g) in tetrahydrofuran (5 mL), followed by stirring between 60 and 70°C for 1 hour. Amberlite IRA 743 (3 g) was gradually added to the mixture at room temperature, and the whole was stirred at 60°C for 1 hour. The resin was removed by filtration, and the filtrate was concentrated under reduced pressure. The resultant residue was purified by means of silica gel column chromatography (eluent; methylene chloride to methylene chloride : methanol=40 : 1) to prepare the titled compound (62.0 mg, Example Intermediate 28-3).

<Step 4> Synthesis of N-[2-[N-[3-[4-(aminomethyl)phenyl]propyl]amino]ethyl]-2-benzo[b]thiophenesulfonamide dihydrochloride

A reaction was carried out in the same manner as in Step 5 of Example 3 by using the compound prepared in Step 3 (30 mg) to prepare the titled compound (22.7 mg).

(Example 29) Synthesis of N-[2-[N-[3-[4-(aminomethyl)phenyl]propyl]-N-methylamino]ethyl]-2-benzofuransulfonamide dihydrochloride

<Step 1> Synthesis of N-[2-[N-[3-[4-(N-tert-butoxycarbonylaminomethyl)phenyl]propyl]-N-methylamino]ethyl]-2-benzofuransulfonamide

A reaction was carried out in the same manner as in Step 4 of Example 3 by using the compound prepared in Step 2 of Example 20 (14.3 mg) and benzofuran-2-sulfonyl chloride (10.0 mg) to prepare the titled compound (17.9 mg, Example Intermediate 29-1).

<Step 2> Synthesis of N-[2-[N-[3-[4-(aminomethyl)phenyl]propyl]-N-methylamino]ethyl]-2-benzofuransulfonamide dihydrochloride

A reaction was carried out in the same manner as in Step 5 of Example 3 by using the compound prepared in Step 1 (18.0 mg) to prepare the titled compound (14.4 mg).

(Example 30) Synthesis of N-[2-[N-[3-[4-(aminomethyl)phenyl]propyl]-N-methylamino]ethyl]-2-benzo[b]thiophenesulfonamide dihydrochloride

<Step 1> Synthesis of N-[2-[N-[3-[4-(N-tert-butoxycarbonylaminomethyl)phenyl]propyl]-N-methylamino]ethyl]-2-benzo[b]thiophenesulfonamide

A reaction was carried out in the same manner as in Step 4 of Example 3 by using the compound prepared in Step 2 of Example 20 (10.0 mg) and benzo[b]thiophene-2-sulfonyl chloride (7.2 mg) to prepare the titled compound (14.0 mg, Example Intermediate 30-1).

<Step 2> Synthesis of N-[2-[N-[3-[4-(aminomethyl)phenyl]propyl]-N-methylamino]ethyl]-2-benzo[b]thiophenesulfonamide dihydrochloride

A reaction was carried out in the same manner as in Step 5 of Example 3 by using the compound prepared in Step 1 (14.0 mg) to prepare the titled compound (13.0 mg).

(Example 31) Synthesis of N-[2-[N-[3-[4-(aminomethyl)phenyl]propyl]-N-methylamino]ethyl]-(3-methyl-2-benzo[b]thiophene)sulfonamide dihydrochloride

<Step 1> Synthesis of N-[2-[N-[3-[4-(N-tert-butoxycarbonylaminomethyl)phenyl]propyl]-N-methylamino]ethyl]-(3-methyl-2-benzo[b]thiophene)sulfonamide

A reaction was carried out in the same manner as in Step 4 of Example 3 by using the compound prepared in Step 2 of Example 20 (42.7 mg) and 3-methylbenzo[b]thiophene-2-sulfonyl chloride (36.1 mg) to prepare the titled compound (60.0 mg, Example Intermediate 31-1).

<Step 2> Synthesis of N-[2-[N-[3-[4-(aminomethyl)phenyl]propyl]-N-methylamino]ethyl]-(3-methyl-2-benzo[b]thiophene)sulfonamide dihydrochloride

A reaction was carried out in the same manner as in Step 5 of Example 3 by using the compound prepared in Step 1 (55.0 mg) to prepare the titled compound (41.8 mg).

(Example 32) Synthesis of N-[2-[N-[3-[4-(aminomethyl)phenyl]propyl]-N-methylamino]ethyl]-(3-bromo-2-benzo[b]thiophene)sulfonamide dihydrochloride

<Step 1> Synthesis of N-[2-[N-[3-[4-(N-tert-butoxycarbonylaminomethyl)phenyl]propyl]-N-methylamino]ethyl]-(3-bromo-2-benzo[b]thiophene)sulfonamide

A reaction was carried out in the same manner as in Step 4 of Example 3 by using the compound prepared in Step 2 of Example 20 (10.0 mg) and 3-bromobenzo[b]thiophene-2-sulfonyl chloride (9.6 mg) to prepare the titled compound (17.6 mg, Example Intermediate 32-1).

<Step 2> Synthesis of N-[2-[N-[3-[4-(aminomethyl)phenyl]propyl]-N-methylamino]ethyl]-(3-bromo-2-benzo[b]thiophene)sulfonamide dihydrochloride

A reaction was carried out in the same manner as in Step 5 of Example 3 by using the compound prepared in Step 1 (16.0 mg) to prepare the titled compound (16.1 mg).

(Example 33) Synthesis of N-[3-[4-(aminomethyl)phenyl]propyl]-N-[2-(2-naphthalenesulfonylamino)ethyl]-3-aminopropionic acid ditrifluoroacetate

<Step 1> Synthesis of tert-butyl N-[3-[4-(N-tert-butoxycarbonylaminomethyl)phenyl]propyl]-N-[2-(2-naphthalenesulfonylamino)ethyl]-3-aminopropionate

Added to a solution of the compound prepared in Step 3 of Example 1 (25.0 mg) in ethanol (1 mL) was tert-butyl acrylate (64.4 mg), followed by heating under reflux for 4 hours. The solvent was removed under reduced pressure, and the resultant residue was purified by means of silica gel column chromatography (eluent; hexane: ethyl acetate=2 : 1) to prepare the titled compound (24.5 mg, Example Intermediate 33-1).

<Step 2> Synthesis of N-[3-[4-(aminomethyl)phenyl]propyl]-N-[2-(2-naphthalenesulfonylamino)ethyl]-3-aminopropionic acid ditrifluoroacetate

Trifluoroacetic acid (0.5 mL) was added to a solution of the compound prepared in Step 1 (24.5 mg) in methylene chloride (0.5 mL), and the whole was stirred at room temperature for 2 hours. The mixture was concentrated under reduced pressure to prepare the titled compound (22.0 mg).

(Example 34) Synthesis of N-[3-[4-(aminomethyl)phenyl]propyl]-N-[2-(2-benzo[b]thiophenesulfonylamino)ethyl]-3-aminopropionic acid ditrifluoroacetate

<Step 1> Synthesis of tert-butyl N-[3-[4-(N-tert-butoxycarbonylaminomethyl)phenyl]propyl]-N-[2-(2-benzo[b]thiophenesulfonylamino)ethyl]-3-aminopropionate

A reaction was carried out in the same manner as in Step 1 of Example 33 by using the compound prepared in Step 3 of Example 28 (30.0 mg) and tert-butyl acrylate (76.3 mg) to prepare the titled compound (28.3 mg, Example Intermediate 34-1).

<Step 2> Synthesis of N-[3-[4-(aminomethyl)phenyl]propyl]-N-[2-(2-benzo[b]thiophenesulfonylamino)ethyl]-3-aminopropionic acid dihydrochloride

A reaction was carried out in the same manner as in Step 2 of Example 33 by using the compound prepared in Step 1 (28.3 mg) to prepare the titled compound (26.0 mg).

(Example 35) Synthesis of N-[2-[N-[3-[4-(aminomethyl)-3-fluorophenyl]propyl]-N-methylamino]ethyl]-2-benzo[b]thiophenesulfonamide dihydrochloride

<Step 1> Synthesis of N-[2-[N-[3-[4-(N-tert-butoxycarbonylaminomethyl)-3-fluorophenyl]propyl]-N-methylamino]ethyl]-2-benzo[b]thiophenesulfonamide

A reaction was carried out in the same manner as in Step 4 of Example 3 by using the compound prepared in Step 5 of Example 24 (18.5 mg) and benzo[b]thiophene-2-sulfonyl chloride (7.9 mg) to prepare the titled compound (18.4 mg, Example Intermediate 35-1).

<Step 2> Synthesis of N-[2-[N-[3-[4-(aminomethyl)-3-fluorophenyl]propyl]-N-methylamino]ethyl]-2-benzo[b]thiophenesulfonamide dihydrochloride

A reaction was carried out in the same manner as in Step 5 of Example 3 by using the compound prepared in Step 1 (16.0 mg) to prepare the titled compound (12.8 mg).

(Example 36) Synthesis of N-[2-[N-[3-(2-aminomethyl-5-pyridyl)propyl]-N-methylamino]ethyl]-(1-chloro-2-naphthalene)sulfonamide trihydrochloride

<Step 1> Synthesis of (2-aminomethyl-5-pyridyl)methanol

A tetrahydrofuran solution of a boranetetrahydrofuran complex (1 M; 50 mL) was added to a solution of 6-cyanonicotinic acid (0.85 g) in tetrahydrofuran (20 mL) under ice-water cooling, followed by stirring at room temperature overnight. Methanol was added to the mixture under ice-water cooling, and then a 10% hydrogen chloride in methanol was added to the mixture, followed by stirring for 1 hour at 40°C at room temperature. The solvent was removed under reduced pressure, and the resultant residue was purified by means of silica gel column chromatography [Chromatorex NH™] (eluent; methanol : methylene chloride=1 : 1) to prepare the titled compound (1.54 g, Example Intermediate 36-1).

<Step 2> Synthesis of 6-(N-tert-butoxycarbonylaminomethyl)nicotinaldehyde

Di-tert-butyl dicarbonate (1.88 g) was added to a solution of the compound prepared in Step 1 (1.54 g) in dioxane (30 mL), and the whole was stirred at 40°C for 1 hour. After the solvent was removed under reduced pressure, water and a 1 N aqueous solution of sodium hydroxide were added to the residue to alkalize the residue, followed by extraction with methylene chloride. The extract was washed with water and a saturated brine, and was dried over anhydrous sodium sulfate, followed by removal of the solvent under reduced pressure. The resultant residue was dissolved in methylene chloride (30 mL), and manganese dioxide (1.00 g) was added to the solution, followed by stirring at room temperature overnight. The mixture was filtered over celite by suction filtration. After the filtrate was concentrated, the concentrate was purified by means of silica gel column chromatography (eluent; methanol : methylene chloride=1 : 9) to prepare the titled compound (0.35 g, Example Intermediate 36-2).

<Step 3> Synthesis of ethyl 3-[2-(N-tert-butoxycarbonylaminomethyl)-5-pyridyl]acrylate

A solution of ethyl diethylphosphonoacetate (1.13 mL) in dimethoxyethane (5 mL) was dropped in a suspension of sodium hydride (60% dispersion in oil; 0.23 g) in dimethoxyethane (10 mL) under ice-water cooling, and the whole was stirred for 30 minutes. A solution of the compound prepared in Step 2 (1.22 g) in dimethoxyethane (5 mL) was dropped in the mixture under ice-water cooling, and the whole was stirred for 1 hour. A saturated aqueous solution of ammonium chloride was added to the mixture, followed by removal of the solvent under reduced pressure. A 1 N aqueous solution of sodium hydroxide was added to the residue to alkalize the residue, followed by extraction with ethyl acetate. The extract was washed with water and a saturated brine, and was dried over anhydrous sodium sulfate, followed by removal of the solvent under reduced pressure. The resultant residue was purified by means of silica gel column chromatography (eluent; hexane : ethyl acetate=1 : 1) to prepare the titled compound (0.71 g, Example Intermediate 36-3).

<Step 4> Synthesis of methyl 3-[2-(N-tert-butoxycarbonylaminomethyl)-5-pyridyl]propionate

Cobalt chloride hexahydrate (0.16 g) was added to a solution of the compound prepared in Step 3 (0.65 g) in methanol (50 mL). Sodium borohydride (1.81 g) was gradually added to the mixture under ice-water cooling, and the whole was stirred at room temperature for 2 hours. The solvent was removed under reduced pressure, and water was added to the solution, followed by extraction with ethyl acetate. The extract was washed with water and a saturated brine, and was dried over anhydrous sodium sulfate, followed by removal of the solvent under reduced pressure. The resultant residue was purified by means of silica gel column chromatography (eluent; hexane : ethyl acetate=1 : 1) to prepare the titled compound (0.51 g, Example Intermediate 36-4).

<Step 5> Synthesis of 3-[2-(N-tert-butoxycarbonylaminomethyl)-5-pyridyl]propanal

A solution of the compound prepared in Step 4 (0.45 g) in toluene (60 mL) was cooled to -78°C, and diisobutylaluminum hydride (1.5 M; 7.5 mL) was gradually dropped in the solution, followed by stirring at the same temperature for 10 minutes. Methanol was added to the mixture, and furthermore ethyl acetate was added to the solution, followed by stirring. After that, the mixture was filtered over celite by suction filtration. After the filtrate had been concentrated, the concentrate was purified by means of silica gel column chromatography (eluent; hexane : ethyl acetate=1 : 1) to prepare the titled compound (0.16 g, Example Intermediate 36-5).

<Step 6> Synthesis of N-(9-fluorenylmethoxycarbonyl)-N-[2-[N-[3-[2-(N-tert-butoxycarbonylaminomethyl)-5-pyridyl] propyl]-N-methylamino]ethyl]amine

A reaction was carried out in the same manner as in Step 1 of Example 20 by using the compound prepared in Step 5 (0.12 g) and N-(9-fluorenylmethoxycarbonyl)-N-[2-(methylamino)ethyl]amine hydrochloride (0.15 g) to prepare the titled compound (0.22 g, Example Intermediate 36-6).

<Step 7> Synthesis of 2-[N-[3-[2-(N-tert-butoxycarbonylaminomethyl)-5-pyridyl]propyl]-N-methylamino]ethylamine

A reaction was carried out in the same manner as in Step 2 of Example 20 by using the compound prepared in Step 6 (0.20 g) to prepare the titled compound (0.12 g, Example Intermediate 36-7).

<Step 8> Synthesis of N-[2-[N-[3-[2-(N-tert-butoxycarbonylaminomethyl)-5-pyridyl]propyl]-N-methylamino)ethyl] -(1-chloro-2-naphthalene)sulfonamide

A reaction was carried out in the same manner as in Step 4 of Example 3 by using the compound prepared in Step 7 (10.2 mg) and (1-chloro-2-naphthalene)sulfonyl chloride (7.8 mg) to prepare the titled compound (10.0 mg, Example Intermediate 36-8).

<Step 9> Synthesis of N-[2-[N-[3-(2-aminomethyl-5-pyridyl)propyl]-N-methylamino]ethyl]-(1-chloro-2-naphthalene) sulfonamide trihydrochloride

A reaction was carried out in the same manner as in Step 5 of Example 3 by using the compound prepared in Step 8 (9.0 mg) to prepare the titled compound (3.0 mg).

(Example 37) Synthesis of N-[2-[N-[3-(2-aminomethyl-5-pyridyl)propyl]-N-methylamino]ethyl]-2-benzo[b] thiophenesulfonamide trihydrochloride

<Step 1> Synthesis of N-[2-[N-[3-[2-(N-tert-butoxycarbonylaminomethyl)-5-pyridyl]propyl]-N-methylamino]ethyl]-2-benzo[b]thiophenesulfonamide

A reaction was carried out in the same manner as in Step 4 of Example 3 by using the compound prepared in Step 7 of Example 36 (13.7 mg) and benzo[b]thiophene-2-sulfonyl chloride (9.4 mg) to prepare the titled compound (15.7 mg, Example Intermediate 37-1).

<Step 2> Synthesis of N-[2-[N-[3-(2-aminomethyl-5-pyridyl)propyl]-N-methylamino]ethyl-2-benzo[b] thiophenesulfonamide trihydrochloride

A reaction was carried out in the same manner as in Step 5 of Example 3 by using the compound prepared in Step 1 (14.0 mg) to prepare the titled compound (13.0 mg).

(Example 38) Synthesis of N-[2-[N-[3-[4-(aminomethyl)phenyl]propyl]amino]ethyl]-2-benzo[b]thiophenecarboxamide dihydrochloride

<Step 1> Synthesis of N-(2-aminoethyl)-2-benzo[b]thiophenecarboxamide hydrochloride

A reaction was carried out in the same manner as in Step 1 of Example 10 by using N-tert-butoxycarbonyl-N-(2-aminoethyl)amine (0.80 mg) and benzo[b]thiophene-2-carboxylic acid (0.89 mg). The resultant compound is treated in the same manner as in Step 5 of Example 3 to prepare the titled compound (0.40 mg, Example Intermediate 38-1).

<Step 2> Synthesis of N-[2-[N-[3-[4-(N-tert-butoxycarbonylaminomethyl)phenyl]propyl]amino]ethyl]-2-benzo[b] thiophenecarboxamide

A reaction was carried out in the same manner as in Step 1 of Example 20 by using the compound prepared in Step 1 (31.1 mg) and the compound prepared in Step 2 of Example 1 (30.0 mg) to prepare the titled compound (20.0 mg, Example Intermediate 38-2).

<Step 3> Synthesis of N-[2-[N-[3-[4-(aminomethyl)phenyl]propyl]amino]ethyl]-2-benzo[b]thiophenecarboxamide dihydrochloride

A reaction was carried out in the same manner as in Step 5 of Example 3 by using the compound prepared in Step 2 (20.0 mg) to prepare the titled compound (15.0 mg).

(Example 39) Synthesis of N-[2-[N-[3-[4-(aminomethyl)phenyl]propyl]-N-methylamino]ethyl]-2-naphthalenecarboxamide dihydrochloride

<Step 1> Synthesis of N-[2-[N-[3-[4-(N-tert-butoxycarbonylaminomethyl)phenyl]propyl]-N-methylamino]ethyl]-2-naphthalenecarboxamide

A reaction was carried out in the same manner as in Step 1 of Example 10 by using the compound prepared in Step 2 of Example 20 (50.0 mg) and naphthalene-2-carboxylic acid (32.8 mg) to prepare the titled compound (55.0 mg, Example Intermediate 39-1).

<Step 2> Synthesis of N-[2-[N-[3-[4-(aminomethyl)phenyl]propyl]-N-methylamino]ethyl]-2-naphthalenecarboxamide dihydrochloride

A reaction was carried out in the same manner as in Step 5 of Example 3 by using the compound prepared in Step 1 (55.0 mg) to prepare the titled compound (41.5 mg).

(Example 40) Synthesis of N-[2-[N-[3-[4-(aminomethyl)phenyl]propyl]-N-methylamino]ethyl]-(1-bromo-2-naphthalene) carboxamide dihydrochloride

<Step 1> Synthesis of N-[2-[N-[3-[4-(N-tert-butoxycarbonylaminomethyl)phenyl]propyl]-N-methylamino]ethyl] -(1-bromo-2-naphthalene)carboxamide

A reaction was carried out in the same manner as in Step 1 of Example 10 by using the compound prepared in Step 2 of Example 20 (50.0 mg) and 1-bromonaphthalene-2-carboxylic acid (41.0 mg) to prepare the titled compound (34.5 mg, Example Intermediate 40-1).

<Step 2> Synthesis of N-[2-[N-[3-[4-(aminomethyl)phenyl]propyl]-N-methylamino]ethyl]-(1-bromo-2-naphthalene) carboxamide dihydrochloride

A reaction was carried out in the same manner as in Step 5 of Example 3 by using the compound prepared in Step 1 (20.0 mg) to prepare the titled compound (11.0 mg).

(Example 41) Synthesis of N-[2-[N-[3-[4-(aminomethyl)phenyl]propyl]-N-methylamino]ethyl]-(6-bromo-2-naphthalene) carboxamide dihydrochloride

<Step 1> Synthesis of N-[2-[N-[3-[4-(N-tert-butoxycarbonylaminomethyl)phenyl]propyl]-N-methylamino]ethyl] -(6-bromo-2-naphthalene)carboxamide

A reaction was carried out in the same manner as in Step 1 of Example 10 by using the compound prepared in Step 2 of Example 20 (20.0 mg) and 6-bromonaphthalene-2-carboxylic acid (17.2 mg) to prepare the titled compound (34.0 mg, Example Intermediate 41-1).

<Step 2> Synthesis of N-[2-[N-[3-[4-(aminomethyl)phenyl]propyl]-N-methylamino]ethyl]-(6-bromo-2-naphthalene) carboxamide dihydrochloride

A reaction was carried out in the same manner as in Step 5 of Example 3 by using the compound prepared in Step 1 (34.0 mg) to prepare the titled compound (27.2 mg).

(Example 42) Synthesis of N-[2-[N-[3-[4-(aminomethyl)phenyl]propyl]-N-methylamino]ethyl]-(6-methoxy-2-naphthalene)carboxamide dihydrochloride

<Step 1> Synthesis of N-[2-[N-[3-[4-(N-tert-butoxycarbonylaminomethyl)phenyl]propyl]-N-methylamino]ethyl]-(6-methoxy-2-naphthalene)carboxamide

A reaction was carried out in the same manner as in Step 1 of Example 10 by using the compound prepared in Step 2 of Example 20 (20.0 mg) and 6-methoxynaphthalene-2-carboxylic acid (13.8 mg) to prepare the titled compound (25.0 mg, Example Intermediate 42-1).

<Step 2> Synthesis of N-[2-[N-[3-[4-(aminomethyl)phenyl]propyl]-N-methylamino]ethyl]-(6-methoxy-2-naphthalene) carboxamide dihydrochloride

A reaction was carried out in the same manner as in Step 5 of Example 3 by using the compound prepared in Step 1 (25.0 mg) to prepare the titled compound (24.0 mg).

(Example 43) Synthesis of 6-[2-[N-[3-[4-(aminomethyl)phenyl]propyl]-N-methylamino]ethylcarbamoyl]naphthalene-2-carboxylic acid ditrifluoroacetate

<Step 1> Synthesis of tert-butyl 6-[2-[N-[3-[4-(N-tert-butoxycarbonylaminomethyl)phenyl]propyl]-N-methylamino] ethylcarbamoyl]naphthalene-2-carboxylate

A reaction was carried out in the same manner as in Step 1 of Example 10 by using the compound prepared in Step 2 of Example 20 (20.0 mg) and 6-tert-butoxycarbonylnaphthalene-2-carboxylic acid (19.0 mg) to prepare the titled compound (32.0 mg, Example Intermediate 43-1).

<Step 2> Synthesis of 6-[2-[N-[3-[4-(aminomethyl)phenyl]propyl]-N-methylamino]ethylcarbamoyl]naphthalene-2-caroxylic acid ditrifluoroacetate

A reaction was carried out in the same manner as in Step 2 of Example 33 by using the compound prepared in Step 1 (32.0 mg) to prepare the titled compound (30.0 mg).

(Example 44) Synthesis of N-[2-[N-[3-[4-(aminomethyl)phenyl]propyl]-N-methylamino]ethyl]-6-(N,N-dimethylcarbamoyl)-2-naphthalene] carboxamide dihydrochloride

<Step 1> Synthesis of N-[2-[N-[3-[4-(N-tert-butoxycarbonylaminomethyl)phenyl]propyl]-N-methylamino]ethyl]-[6-(N,N-dimethylcarbamoyl)-2-naphthalene]-carboxamide

A reaction was carried out in the same manner as in Step 1 of Example 10 by using the compound prepared in Step 2 of Example 20 (0.15 g) and 6-(N,N-dimethylcarbamoyl)naphthalene-2-carboxylic acid (0.13 g) to prepare the titled compound (0.19 g, Example Intermediate 44-1).

<Step 2> Synthesis of N-[2-[N-[3-[4-(aminomethyl)phenyl]propyl]-N-methylamino]ethyl]-[6-(N,N-dimethylcarbamoyl)-2-naphthalene]carboxamide dihydrochloride

A reaction was carried out in the same manner as in Step 5 of Example 3 by using the compound prepared in Step 1 (0.19 g) to prepare the titled compound (0.10 g).

(Example 45) Synthesis of N-[2-[N-[3-[4-(aminomethyl)phenyl]propyl]-N-methylamino]ethyl]-2-benzofurancarboxamide dihydrochloride

<Step 1> Synthesis of N-[2-[N-[3-[4-(N-tert-butoxycarbonylaminomethyl)phenyl]propyl]-N-methylamino]ethyl]-2-benzofurancarboxamide

A reaction was carried out in the same manner as in Step 1 of Example 10 by using the compound prepared in Step 2 of Example 20 (48.0 mg) and benzofuran-2-carboxylic acid (25.9 mg) to prepare the titled compound (42.0 mg, Example Intermediate 45-1).

<Step 2> Synthesis of N-[2-[N-[3-[4-(aminomethyl)phenyl]propyl]-N-methylamino]ethyl]-2-benzofurancarboxamide dihydrochloride

A reaction was carried out in the same manner as in Step 5 of Example 3 by using the compound prepared in Step 1 (42.0 mg) to prepare the titled compound (32.4 mg).

(Example 46) Synthesis of N-[2-[N-[3-[4-(aminomethyl)phenyl]propyl]-N-methylamino]ethyl]-5-benzofurancarboxamide dihydrochloride

<Step 1> Synthesis of N-[2-[N-[3-[4-(N-tert-butoxycarbonylaminomethyl)phenyl]propyl]-N-methylamino]ethyl]-5-benzofurancarboxamide

A reaction was carried out in the same manner as in Step 1 of Example 10 by using the compound prepared in Step 2 of Example 20 (20.9 mg) and benzofuran-5-carboxylic acid (11.1 mg) to prepare the titled compound (18.2 mg, Example Intermediate 46-1).

<Step 2> Synthesis of N-[2-[N-[3-[4-(aminomethyl)phenyl]propyl]-N-methylamino]ethyl]-5-benzofurancarboxamide dihydrochloride

A reaction was carried out in the same manner as in Step 5 of Example 3 by using the compound prepared in Step 1 (16.2 mg) to prepare the titled compound (12.6 mg).

(Example 47) Synthesis of N-[2-[N-[3-[4-(aminomethyl)phenyl]propyl]-N-methylamino]ethyl]-2-benzo[b]thiophenecarboxamide dihydrochloride

<Step 1> Synthesis of N-[2-[N-[3-[4-(N-tert-butoxycarbonylaminomethyl)phenyl]propyl]-N-methylamino]ethyl]-2-benzo[b]thiophenecarboxamide

A reaction was carried out in the same manner as in Step 1 of Example 10 by using the compound prepared in Step 2 of Example 20 (30.0 mg) and benzo[b]thiophene-2-carboxylic acid (18.3 mg) to prepare the titled compound (34.8 mg, Example Intermediate 47-1).

<Step 2> Synthesis of N-[2-[N-[3-[4-(aminomethyl)phenyl]propyl]-N-methylamino]ethyl]-2-benzo[b]thiophenecarboxamide dihydrochloride

A reaction was carried out in the same manner as in Step 5 of Example 3 by using the compound prepared in Step 1 (30.0 mg) to prepare the titled compound (23.0 mg).

(Example 48) Synthesis of N-[2-[N-[3-[4-(aminomethyl)phenyl]propyl]-N-methylamino]ethyl]-5-benzo[b]thiophenecarboxamide dihydrochloride

<Step 1> Synthesis of N-[2-[N-[3-[4-(N-tert-butoxycarbonylaminomethyl)phenyl]propyl]-N-methylamino]ethyl]-5-benzo[b]thiophenecarboxamide

A reaction was carried out in the same manner as in Step 1 of Example 10 by using the compound prepared in Step 2 of Example 20 (22.7 mg) and benzo[b]thiophene-5-carboxylic acid (13.2 mg) to prepare the titled compound (23.9 mg, Example Intermediate 48-1).

<Step 2> Synthesis of N-[2-[N-[3-[4-(aminomethyl)phenyl]propyl]-N-methylamino]ethyl]-5-benzo[b]thiophenecarboxamide dihydrochloride

A reaction was carried out in the same manner as in Step 5 of Example 3 by using the compound prepared in Step 1 (20.1 mg) to prepare the titled compound (15.6 mg).

(Example 49) Synthesis of N-[2-[N-[3-[4-(aminomethyl)phenyl]propyl]-N-methylamino]ethyl]-(3-chloro-2-benzo[b]thiophene)carboxamide dihydrochloride

<Step 1> Synthesis of N-[2-[N-[3-[4-(N-tert-butoxycarbonylaminomethyl)phenyl]propyl]-N-methylamino]ethyl]-(3-chloro-2-benzo[b]thiophene)carboxamide

A reaction was carried out in the same manner as in Step 1 of Example 10 by using the compound prepared in Step 2 of Example 20 (40.1 mg) and 3-chlorobenzo[b]thiophene-2-carboxylic acid (29.2 mg) to prepare the titled compound (37.5 mg, Example Intermediate 49-1).

<Step 2> Synthesis of N-[2-[N-[3-[4-(aminomethyl)phenyl]propyl]-N-methylamino]ethyl]-(3-chloro-2-benzo[b]thiophene)carboxamide dihydrochloride

A reaction was carried out in the same manner as in Step 5 of Example 3 by using the compound prepared in Step 1 (33.2 mg) to prepare the titled compound (28.8 mg).

(Example 50) Synthesis of N-[2-[N-[3-[4-(aminomethyl)phenyl]propyl]-N-methylamino]ethyl]-(2-indolecarboxamide dihydrochloride

<Step 1> Synthesis of N-[2-[N-[3-[4-(N-tert-butoxycarbonylaminomethyl)phenyl]propyl]-N-methylamino]ethyl]-2-indolecarboxamide

A reaction was carried out in the same manner as in Step 1 of Example 10 by using the compound prepared in Step 2 of Example 20 (36.5 mg) and indole-2-carboxylic acid (20.1 mg) to prepare the titled compound (35.0 mg, Example Intermediate 50-1).

<Step 2> Synthesis of N-[2-[N-[3-[4-(aminomethyl)phenyl]propyl]-N-methylamino]ethyl]-2-indolecarboxamide dihydrochloride

A reaction was carried out in the same manner as in Step 5 of Example 3 by using the compound prepared in Step 1 (32.1 mg) to prepare the titled compound (23.5 mg).

(Example 51) Synthesis of N-[2-[N-[3-[4-(aminomethyl)phenyl]propyl]-N-methylamino]ethyl]-3-indolecarboxamide dihydrochloride

<Step 1> Synthesis of N-[2-[N-[3-[4-(N-tert-butoxycarbonylaminomethyl)phenyl]propyl]-N-methylamino]ethyl]-3-indolecarboxamide

A reaction was carried out in the same manner as in Step 1 of Example 10 by using the compound prepared in Step 2 of Example 20 (25.2 mg) and indole-3-carboxylic acid (13.9 mg) to prepare the titled compound (28.2 mg, Example Intermediate 51-1).

<Step 2> Synthesis of N-[2-[N-[3-[4-(aminomethyl)phenyl]propyl]-N-methylamino]ethyl]-3-indolecarboxamide dihydrochloride

A reaction was carried out in the same manner as in Step 5 of Example 3 by using the compound prepared in Step 1 (27.3 mg) to prepare the titled compound (24.0 mg).

(Example 52) Synthesis of N-[2-[N-[3-[4-(aminomethyl)phenyl]propyl]-N-methylamino]ethyl]-5-indolecarboxamide dihydrochloride

<Step 1> Synthesis of N-[2-[N-[3-[4-(N-tert-butoxycarbonylaminomethyl)phenyl]propyl]-N-methylamino]ethyl]-5-indolecarboxamide

A reaction was carried out in the same manner as in Step 1 of Example 10 by using the compound prepared in Step 2 of Example 20 (50.0 mg) and indole-5-carboxylic acid (30.1 mg) to prepare the titled compound (50.1 mg, Example Intermediate 52-1).

<Step 2> Synthesis of N-[2-[N-[3-[4-(aminomethyl)phenyl]propyl]-N-methylamino]ethyl]-5-indolecarboxamide dihydrochloride

A reaction was carried out in the same manner as in Step 5 of Example 3 by using the compound prepared in Step 1 (50.1 mg) to prepare the titled compound (26.3 mg).

(Example 53) Synthesis of N-[2-[N-[3-[4-(aminomethyl)phenyl]propyl]-N-methylamino]ethyl]-6-indolecarboxamide dihydrochloride

<Step 1> Synthesis of N-[2-[N-[3-[4-(N-tert-butoxycarbonylaminomethyl)phenyl]propyl]-N-methylamino]ethyl]-6-indolecarboxamide

A reaction was carried out in the same manner as in Step 1 of Example 10 by using the compound prepared in Step 2 of Example 20 (22.6 mg) and indole-6-carboxylic acid (13.6 mg) to prepare the titled compound (22.5 mg, Example Intermediate 53-1).

<Step 2> Synthesis of N-[2-[N-[3-[4-(aminomethyl)phenyl]propyl]-N-methylamino]ethyl]-6-indolecarboxamide dihydrochloride

A reaction was carried out in the same manner as in Step 5 of Example 3 by using the compound prepared in Step 1 (21.5 mg) to prepare the titled compound (19.3 mg).

(Example 54) Synthesis of N-[2-[N-[3-[4-(aminomethyl)phenyl]propyl]-N-methylamino]ethyl]-(1-methyl-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(1-methyl-2-indole)carboxamide

A reaction was carried out in the same manner as in Step 1 of Example 10 by using the compound prepared in Step 2 of Example 20 (48.3 mg) and 1-methylindole-2-carboxylic acid (29.0 mg) to prepare the titled compound (48.4 mg, Example Intermediate 54-1).

<Step 2> Synthesis of N-[2-[N-[3-[4-(aminomethyl)phenyl]propyl]-N-methylamino]ethyl]-(1-methyl-2-indole) carboxamide dihydrochloride

A reaction was carried out in the same manner as in Step 5 of Example 3 by using the compound prepared in Step 1 (45.2 mg) to prepare the titled compound (35.5 mg).

(Example 55) Synthesis of N-[2-[N-[3-[4-(aminomethyl)phenyl]propyl]-N-methylamino]ethyl]-(5-methyl-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(5-methyl-2-indole)carboxamide

A reaction was carried out in the same manner as in Step 1 of Example 10 by using the compound prepared in Step 2 of Example 20 (0.10 g) and 5-methylindole-2-carboxylic acid (65.0 mg) to prepare the titled compound (0.14 g, Example Intermediate 55-1).

<Step 2> Synthesis of N-[2-[N-[3-[4-(aminomethyl)phenyl]propyl]-N-methylamino]ethyl]-(5-methyl-2-indole) carboxamide dihydrochloride

A reaction was carried out in the same manner as in Step 5 of Example 3 by using the compound prepared in Step 1 (0.14 g) to prepare the titled compound (93.0 mg).

(Example 56) Synthesis of N-[2-[N-[3-[4-(aminomethyl)phenyl]propyl]-N-methylamino]ethyl]-(5-isopropyl-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(5-isopropyl-2-indole)carboxamide

A reaction was carried out in the same manner as in Step 1 of Example 10 by using the compound prepared in Step 2 of Example 20 (0.15 g) and 5-isopropylindole-2-carboxylic acid (0.11 g) to prepare the titled compound (0.17 g, Example Intermediate 56-1).

<Step 2> Synthesis of N-[2-[N-[3-[4-(aminomethyl)phenyl]propyl]-N-methylamino]ethyl]-(5-isopropyl-2-indole) carboxamide dihydrochloride

A reaction was carried out in the same manner as in Step 5 of Example 3 by using the compound prepared in Step 1 (0.17 g) to prepare the titled compound (0.15 g).

(Example 57) Synthesis of N-[2-[N-[3-[4-(aminomethyl)phenyl]propyl]-N-methylamino]ethyl]-(4-chloro-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(4-chloro-2-indole)carboxamide

A reaction was carried out in the same manner as in Step 1 of Example 10 by using the compound prepared in Step 2 of Example 20 (0.10 g) and 4-chloroindole-2-carboxylic acid (72.4 mg) to prepare the titled compound (80.0 mg, Example Intermediate 57-1).

<Step 2> Synthesis of N-[2-[N-[3-[4-(aminomethyl)phenyl]propyl]-N-methylamino]ethyl]-(4-chloro-2-indole) carboxamide dihydrochloride

A reaction was carried out in the same manner as in Step 5 of Example 3 by using the compound prepared in Step 1 (80.0 mg) to prepare the titled compound (45.0 mg).

(Example 58) Synthesis of N-[2-[N-[3-[4-(aminomethyl)phenyl]propyl]-N-methylamino]ethyl]-(5-chloro-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(5-chloro-2-indole)carboxamide

A reaction was carried out in the same manner as in Step 1 of Example 10 by using the compound prepared in Step 2 of Example 20 (56.8 mg) and 5-chloroindole-2-carboxylic acid (36.5 mg) to prepare the titled compound (87.1 mg, Example Intermediate 58-1).

<Step 2> Synthesis of N-[2-[N-[3-[4-(aminomethyl)phenyl]propyl]-N-methylamino]ethyl)-(5-chloro-2-indole) carboxamide dihydrochloride

A reaction was carried out in the same manner as in Step 5 of Example 3 by using the compound prepared in Step 1 (80.0 mg) to prepare the titled compound (60.7 mg).

(Example 59) Synthesis of N-[2-[N-[3-[4-(aminomethyl)phenyl]propyl]-N-methylamino]ethyl]-(5-chloro-2-indole) carboxamide

The compound prepared in Step 2 of Example 58 (1.12 g) was purified by means of silica gel column chromatography [Chromatorex NH™] (eluent; methylene chloride : methanol=20 : 1) to prepare the titled compound (0.94 g).

(Example 60) Synthesis of N-[2-[N-[3-[4-(aminomethyl)phenyl]propyl]-N-methylamino]ethyl]-(6-chloro-2-indole)carboxamide dihydrochloride

<Step 1> Synthesis of N-[2-[N-[3-[4-(N-tert-butoxycarbonylaminomethyl)phenyl]propyl]-N-methylamino)ethyl]-(6-chloro-2-indole)carboxamide

A reaction was carried out in the same manner as in Step 1 of Example 10 by using the compound prepared in Step 2 of Example 20 (0.10 g) and 6-chloroindole-2-carboxylic acid (73.0 mg) to prepare the titled compound (0.12 g, Example Intermediate 60-1).

<Step 2> Synthesis of N-[2-[N-[3-[4-(aminomethyl)phenyl]propyl]-N-methylamino]ethyl]-(6-chloro-2-indole)carboxamide dihydrochloride

A reaction was carried out in the same manner as in Step 5 of Example 3 by using the compound prepared in Step 1 (0.10 g) to prepare the titled compound (88.1 mg).

(Example 61) Synthesis of N-[2-[N-[3-[4-(aminomethyl)phenyl]propyl]-N-methylamino]ethyl]-(5-bromo-2-indole)carboxamide dihydrochloride

<Step 1> Synthesis of N-[2-[N-[3-[4-(N-tert-butoxycarbonylaminomethyl)phenyl]propyl]-N-methylamino]ethyl]-(5-bromo-2-indole)carboxamide

A reaction was carried out in the same manner as in Step 1 of Example 10 by using the compound prepared in Step 2 of Example 20 (98.2 mg) and 5-bromoindole-2-carboxylic acid (88.0 mg) to prepare the titled compound (0.12 g, Example Intermediate 61-1).

<Step 2> Synthesis of N-[2-[N-[3-[4-(aminomethyl)phenyl]propyl]-N-methylamino]ethyl]-(5-bromo-2-indole)carboxamide dihydrochloride

A reaction was carried out in the same manner as in Step 5 of Example 3 by using the compound prepared in Step 1 (0.11 g) to prepare the titled compound (92.3 mg).

(Example 62) Synthesis of N-[2-[N-[3-[4-(aminomethyl)phenyl]propyl]-N-methylamino]ethyl]-(5-fluoro-2-indole)carboxamide dihydrochloride

<Step 1> Synthesis of N-[2-[N-[3-[4-(N-tert-butoxycarbonylaminomethyl)phenyl]propyl]-N-methylamino]ethyl]-(5-fluoro-2-indole)carboxamide

A reaction was carried out in the same manner as in Step 1 of Example 10 by using the compound prepared in Step 2 of Example 20 (98.4 mg) and 5-fluoroindole-2-carboxylic acid (65.8 mg) to prepare the titled compound (0.11 g, Example Intermediate 62-1).

<Step 2> Synthesis of N-[2-[N-[3-[4-(aminomethyl)phenyl]propyl]-N-methylamino]ethyl]-(5-fluoro-2-indole)carboxamide dihydrochloride

A reaction was carried out in the same manner as in Step 5 of Example 3 by using the compound prepared in Step 1 (0.11 g) to prepare the titled compound (63.5 mg).

(Example 63) Synthesis of N-[2-[N-[3-[4-(aminomethyl)phenyl]propyl]-N-methylamino]ethyl]-(5-trifluoromethyl-2-indole)carboxamide dihydrochloride

<Step 1> Synthesis of N-[2-[N-[3-[4-(N-tert-butoxycarbonylaminomethyl)phenyl]propyl]-N-methylamino]ethyl]-(5-trifluoromethyl-2-indole)carboxamide

A reaction was carried out in the same manner as in Step 1 of Example 10 by using the compound prepared in Step 2 of Example 20 (96.5 mg) and 5-trifluoromethylindole-2-carboxylic acid (58.3 mg) to prepare the titled compound (62.7 mg, Example Intermediate 63-1).

<Step 2> Synthesis of N-[2-[N-[3-[4-(aminomethyl)phenyl]propyl]-N-methylamino]ethyl]-(5-trifluoromethyl-2-indole) carboxamide dihydrochloride

A reaction was carried out in the same manner as in Step 5 of Example 3 by using the compound prepared in Step 1 (58.2 mg) to prepare the titled compound (27.9 mg).

(Example 64) Synthesis of N-[2-[N-[3-[4-(aminomethyl)phenyl]propyl]-N-methylamino]ethyl]-(4-methoxy-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(4-methoxy-2-indole)carboxamide

A reaction was carried out in the same manner as in Step 1 of Example 10 by using the compound prepared in Step 2 of Example 20 (43.8 mg) and 4-methoxyindole-2-carboxylic acid (28.7 mg) to prepare the titled compound (51.2 mg, Example Intermediate 64-1).

<Step 2> Synthesis of N-[2-[N-[3-[4-(aminomethyl)phenyl]propyl]-N-methylamino]ethyl]-(4-methoxy-2-indole) carboxamide dihydrochloride

A reaction was carried out in the same manner as in Step 5 of Example 3 by using the compound prepared in Step 1 (48.5 mg) to prepare the titled compound (37.1 mg).

(Example 65) Synthesis of N-[2-[N-[3-[4-(aminomethyl)phenyl]propyl]-N-methylamino]ethyl]-(5-methoxy-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(5-methoxy-2-indole)carboxamide

A reaction was carried out in the same manner as in Step 1 of Example 10 by using the compound prepared in Step 2 of Example 20 (55.8 mg) and 5-methoxyindole-2-carboxylic acid (37.8 mg) to prepare the titled compound (85.1 mg, Example Intermediate 65-1).

<Step 2> Synthesis of N-[2-[N-[3-[4-(aminomethyl)phenyl]propyl]-N-methylamino]ethyl]-(5-methoxy-2-indole) carboxamide dihydrochloride

A reaction was carried out in the same manner as in Step 5 of Example 3 by using the compound prepared in Step 1 (80.0 mg) to prepare the titled compound (55.7 mg).

(Example 66) Synthesis of N-[2-[N-[3-[4-(aminomethyl)phenyl]propyl]-N-methylamino]ethyl]-(6-methoxy-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(6-methoxy-2-indole)carboxamide

A reaction was carried out in the same manner as in Step 1 of Example 10 by using the compound prepared in Step 2 of Example 20 (0.10 g) and 6-methoxyindole-2-carboxylic acid (71.1 mg) to prepare the titled compound (50.0 mg, Example Intermediate 66-1).

<Step 2> Synthesis of N-[2-[N-[3-[4-(aminomethyl)phenyl]propyl]-N-methylamino]ethyl]-(6-methoxy-2-indole) carboxamide dihydrochloride

A reaction was carried out in the same manner as in Step 5 of Example 3 by using the compound prepared in Step 1 (50.0 mg) to prepare the titled compound (47.0 mg).

(Example 67) Synthesis of N-[2-[N-[3-[4-(aminomethyl)phenyl]propyl]-N-methylamino]ethyl]-(5-trifluoromethoxy-2-indole)carboxamide dihydrochloride

<Step 1> Synthesis of N-[2-[N-[3-[4-(N-tert-butoxycarbonylaminomethyl)phenyl]propyl]-N-methylamino]ethyl]-(5-trifluoromethoxy-2-indole)carboxamide

A reaction was carried out in the same manner as in Step 1 of Example 10 by using the compound prepared in Step 2 of Example 20 (0.11 g) and 5-trifluoromethoxyindole-2-carboxylic acid (0.10 g) to prepare the titled compound (0.15 g, Example Intermediate 67-1).

<Step 2> Synthesis of N-[2-[N-[3-[4-(aminomethyl)phenyl]propyl]-N-methylamino]ethyl]-(5-trifluoromethoxy-2-indole) carboxamide dihydrochloride

A reaction was carried out in the same manner as in Step 5 of Example 3 by using the compound prepared in Step 1 (0.14 g) to prepare the titled compound (45.9 mg).

(Example 68) Synthesis of N-[2-[N-[3-[4-(aminomethyl)phenyl]propyl]-N-methylamino]ethyl]-(5-cyano-2-indole) carboxamide dihydrochloride

<Step 1> Synthesis of N-[2-[N-[3-[4-(N-tert-butoxycarbonylaminomethyl)phenyl]propyl]-N-methylamino]ethyl]-(5-cyano-2-indole)carboxamide

A reaction was carried out in the same manner as in Step 1 of Example 10 by using the compound prepared in Step 2 of Example 20 (0.10 g) and 5-cyanoindole-2-carboxylic acid (70.0 mg) to prepare the titled compound (0.12 g, Example Intermediate 68-1).

<Step 2> Synthesis of N-[2-[N-[3-[4-(aminomethyl)phenyl]propyl]-N-methylamino]ethyl]-(5-cyano-2-indole) carboxamide dihydrochloride

A reaction was carried out in the same manner as in Step 5 of Example 3 by using the compound prepared in Step 1 (0.11 g) to prepare the titled compound (86.0 mg).

(Example 69) Synthesis of 2-[2-[N-[3-[4-(aminomethyl)phenyl]propyl]-N-methylamino]ethylcarbamoyl]indole-5-carboxylic acid dihydrochloride

<Step 1> Synthesis of tert-butyl 2-[2-[N-[3-[4-(N-tert-butoxycarbonylaminomethyl)phenyl]propyl]-N-methylamino] ethylcarbamoyl]indole-5-carboxylate

A reaction was carried out in the same manner as in Step 1 of Example 10 by using the compound prepared in Step 2 of Example 20 (25.0 mg) and 5-tert-butoxycarbonylindole-2-carboxylic acid (17.0 mg) to prepare the titled compound (20.1 mg, Example Intermediate 69-1).

<Step 2> Synthesis of 2-[2-[N-[3-[4-(aminomethyl)phenyl]propyl]-N-methylamino]ethylcarbamoyl]indole-5-carboxylic acid dihydrochloride

A reaction was carried out in the same manner as in Step 5 of Example 3 by using the compound prepared in Step 1 (19.6 mg) to prepare the titled compound (13.8 mg).

(Example 70) Synthesis of N-[2-[N-[3-[4-(aminomethyl)phenyl]propyl]-N-methylamino]ethyl]-(5-acetyl-2-indole) carboxamide dihydrochloride

<Step 1> Synthesis of N-[2-[N-[3-[4-(N-tert-butoxycarbonylaminomethyl)phenyl]propyl]-N-methylamino]ethyl]-(5-acetyl-2-indole)carboxamide

A reaction was carried out in the same manner as in Step 1 of Example 10 by using the compound prepared in Step 2 of Example 20 (0.10 g) and 5-acetylindole-2-carboxylic acid (78.1 mg) to prepare the titled compound (0.12 g, Example Intermediate 70-1).

<Step 2> Synthesis of N-[2-[N-[3-[4-(aminomethyl)phenyl]propyl]-N-methylamino]ethyl]-(5-acetyl-2-indole) carboxamide dihydrochloride

A reaction was carried out in the same manner as in Step 5 of Example 3 by using the compound prepared in Step 1 (0.12 g) to prepare the titled compound (0.12 g).

(Example 71) Synthesis of N-[2-[N-[3-[4-(aminomethyl)phenyl]propyl]-N-methylamino]ethyl]-(5-methanesulfonyl-2-indole)carboxamide dihydrochloride

<Step 1> Synthesis of N-[2-[N-[3-[4-(N-tert-butoxycarbonylaminomethyl)phenyl]propyl]-N-methylamino]ethyl] -(5-methanesulfonyl-2-indole)carboxamide

A reaction was carried out in the same manner as in Step 1 of Example 10 by using the compound prepared in Step 2 of Example 20 (0.10 g) and 5-methanesulfonylindole-2-carboxylic acid (90.2 mg) to prepare the titled compound (0.11 g, Example Intermediate 71-1).

<Step 2> Synthesis of N-[2-[N-[3-[4-(aminomethyl)phenyl]propyl]-N-methylamino]ethyl]-(5-methanesulfonyl-2-indole) carboxamide dihydrochloride

A reaction was carried out in the same manner as in Step 5 of Example 3 by using the compound prepared in Step 1 (0.11 g) to prepare the titled compound (88.9 mg).

(Example 72) Synthesis of N-[2-[N-[3-[4-(aminomethyl)phenyl]propyl]-N-methylamino]ethyl]-(5-benzyloxy-2-indole) carboxamide dihydrochloride

<Step 1> Synthesis of N-[2-[N-[3-[4-(N-tert-butoxycarbonylaminomethyl)phenyl]propyl]-N-methylamino]ethyl] -(5-benzyloxy-2-indole)carboxamide

A reaction was carried out in the same manner as in Step 1 of Example 10 by using the compound prepared in Step 2 of Example 20 (52.4 mg) and 5-benzyloxyindole-2-carboxylic acid (52.3 mg) to prepare the titled compound (86.0 mg, Example Intermediate 72-1).

<Step 2> Synthesis of N-[2-[N-[3-[4-(aminomethyl)phenyl]propyl]-N-methylamino]ethyl]-(5-benzyloxy-2-indole) carboxamide dihydrochloride

A reaction was carried out in the same manner as in Step 5 of Example 3 by using the compound prepared in Step 1 (85.0 mg) to prepare the titled compound (77.9 mg).

(Example 73) Synthesis of N-[2-[N-[3-[4-(aminomethyl)phenyl]propyl]-N-methylamino]ethyl]-(1-methyl-5-indole) carboxamide dihydrochloride

<Step 1> Synthesis of N-[2-[N-[3-[4-(N-tert-butoxycarbonylaminomethyl)phenyl]propyl]-N-methylamino)ethyl] -(1-methyl-5-indole)carboxamide

A reaction was carried out in the same manner as in Step 1 of Example 10 by using the compound prepared in Step 2 of Example 20 (0.10 g) and 1-methylindole-5-carboxylic acid (65.2 mg) to prepare the titled compound (82.0 mg, Example Intermediate 73-1).

<Step 2> Synthesis of N-[2-[N-[3-[4-(aminomethyl)phenyl]propyl]-N-methylamino]ethyl]-(1-methyl-5-indole) carboxamide dihydrochloride

A reaction was carried out in the same manner as in Step 5 of Example 3 by using the compound prepared in Step 1 (82.0 mg) to prepare the titled compound (78.0 mg).

(Example 74) Synthesis of N-[2-[N-[3-[4-(aminomethyl)phenyl]propyl]-N-methylamino]ethyl]-(2-methyl-5-indole) carboxamide dihydrochloride

<Step 1> Synthesis of N-[2-[N-[3-[4-(N-tert-butoxycarbonylaminomethyl)phenyl]propyl]-N-methylamino]ethyl] -(2-methyl-5-indole)carboxamide

A reaction was carried out in the same manner as in Step 1 of Example 10 by using the compound prepared in Step 2 of Example 20 (51.2 mg) and 2-methylindole-5-carboxylic acid (33.5 mg) to prepare the titled compound (64.3 mg, Example Intermediate 74-1).

<Step 2> Synthesis of N-[2-[N-[3-[4-(aminomethyl)phenyl]propyl]-N-methylamino]ethyl]-(2-methyl-5-indole) carboxamide dihydrochloride

A reaction was carried out in the same manner as in Step 5 of Example 3 by using the compound prepared in Step 1 (61.2 mg) to prepare the titled compound (51.2 mg).

(Example 75) Synthesis of N-[2-[N-[3-[4-(aminomethyl)phenyl]propyl]-N-methylamino]ethyl]-(2,3-dimethyl-5-indole) carboxamide dihydrochloride

<Step 1> Synthesis of N-[2-[N-[3-[4-(N-tert-butoxycarbonylaminomethyl)phenyl]propyl]-N-methylamino]ethyl] -(2,3-dimethyl-5-indole)carboxamide

A reaction was carried out in the same manner as in Step 1 of Example 10 by using the compound prepared in Step 2 of Example 20 (60.0 mg) and 2,3-dimethylindole-5-carboxylic acid (42.4 mg) to prepare the titled compound (88.6 mg, Example Intermediate 75-1).

<Step 2> Synthesis of N-[2-[N-[3-[4-(aminomethyl)phenyl]propyl]-N-methylamino]ethyl]-(2,3-dimethyl-5-indole) carboxamide dihydrochloride

A reaction was carried out in the same manner as in Step 5 of Example 3 by using the compound prepared in Step 1 (80.4 mg) to prepare the titled compound (77.3 mg).

(Example 76) Synthesis of N-[2-[N-[3-[4-(aminomethyl)phenyl]propyl]-N-methylamino]ethyl]-(1-benzoyl-5-indole) carboxamide dihydrochloride

<Step 1> Synthesis of N-[2-[N-[3-[4-(N-tert-butoxycarbonylaminomethyl)phenyl]propyl]-N-methylamino]ethyl] -(1-benzoyl-5-indole)carboxamide

A reaction was carried out in the same manner as in Step 1 of Example 10 by using the compound prepared in Step 2 of Example 20 (0.15 g) and 1-benzoylindole-5-carboxylic acid (0.14 g) to prepare the titled compound (0.23 g, Example Intermediate 76-1).

<Step 2> Synthesis of N-[2-[N-[3-[4-(aminomethyl)phenyl]propyl]-N-methylamino]ethyl]-(1-benzoyl-5-indole) carboxamide dihydrochloride

A reaction was carried out in the same manner as in Step 5 of Example 3 by using the compound prepared in Step 1 (0.22 g) to prepare the titled compound (0.19 g).

(Example 77) Synthesis of N-[2-[N-[3-[4-(aminomethyl)phenyl]propyl]-N-methylamino]ethyl]-2-benzothiazolecarboxamide dihydrochloride

<Step 1> Synthesis of N-[2-[N-[3-[4-(N-tert-butoxycarbonylaminomethyl)phenyl]propyl]-N-methylamino]ethyl]-2-benzothiazolecarboxamide

A reaction was carried out in the same manner as in Step 1 of Example 10 by using the compound prepared in Step 2 of Example 20 (26.8 mg) and benzothiazole-2-carboxylic acid (16.4 mg) to prepare the titled compound (16.7 mg, Example Intermediate 77-1).

<Step 2> Synthesis of N-[2-[N-[3-[4-(aminomethyl)phenyl]propyl]-N-methylamino]ethyl]-2-benzothiazolecarboxamide dihydrochloride

A reaction was carried out in the same manner as in Step 5 of Example 3 by using the compound prepared in Step 1 (14.1 mg) to prepare the titled compound (14.0 mg).

(Example 78) Synthesis of N-[2-[N-[3-[4-(aminomethyl)phenyl]propyl]-N-methylamino]ethyl]-6-benzothiazolecarboxamide dihydrochloride

<Step 1> Synthesis of N-[2-[N-[3-[4-(N-tert-butoxycarbonylaminomethyl)phenyl]propyl]-N-methylamino]ethyl]-6-benzothiazolecarboxamide

A reaction was carried out in the same manner as in Step 1 of Example 10 by using the compound prepared in Step 2 of Example 20 (0.10 mg) and benzothiazole-6-carboxylic acid (66.7 mg) to prepare the titled compound (50.0 mg, Example Intermediate 78-1).

<Step 2> Synthesis of N-[2-[N-[3-[4-(aminomethyl)phenyl]propyl]-N-methylamino]ethyl]-6-benzothiazolecarboxamide dihydrochloride

A reaction was carried out in the same manner as in Step 5 of Example 3 by using the compound prepared in Step 1 (50.0 mg) to prepare the titled compound (45.0 mg).

(Example 79) Synthesis of N-[2-[N-[3-[4-(aminomethyl)phenyl]propyl]-N-methylamino]ethyl]-3,4-methylenedioxybenzenecarboxamide dihydrochloride

<Step 1> Synthesis of N-[2-[N-[3-[4-(N-tert-butoxycarbonylaminomethyl)phenyl]propyl]-N-methylamino]ethyl]-3,4-methylenedioxybenzenecarboxamide

A reaction was carried out in the same manner as in Step 1 of Example 10 by using the compound prepared in Step 2 of Example 20 (50.0 mg) and 3,4-methylenedioxybenzoic acid (31.6 mg) to prepare the titled compound (48.0 mg, Example Intermediate 79-1).

<Step 2> Synthesis of N-[2-[N-[3-[4-(aminomethyl)phenyl]propyl]-N-methylamino]ethyl]-3,4-methylenedioxybenzenecarboxamide dihydrochloride

A reaction was carried out in the same manner as in Step 5 of Example 3 by using the compound prepared in Step 1 (48.0 mg) to prepare the titled compound (40.0 mg).

(Example 80) Synthesis of N-[2-[N-[3-[4-(aminomethyl)phenyl]propyl]-N-methylamino]ethyl]-2-quinolinecarboxamide trihydrochloride

<Step 1> Synthesis of N-[2-[N-[3-[4-(N-tert-butoxycarbonylaminomethyl)phenyl]propyl]-N-methylamino]ethyl]-2-quinolinecarboxamide

A reaction was carried out in the same manner as in Step 1 of Example 10 by using the compound prepared in Step 2 of Example 20 (0.10 g) and quinoline-2-carboxylic acid (64.1 mg) to prepare the titled compound (90.0 mg, Example Intermediate 80-1).

<Step 2> Synthesis of N-[2-[N-[3-[4-(aminomethyl)phenyl]propyl]-N-methylamino]ethyl]-2-quinolinecarboxamide trihydrochloride

A reaction was carried out in the same manner as in Step 5 of Example 3 by using the compound prepared in Step 1 (90.0 mg) to prepare the titled compound (79.8 mg).

(Example 81) Synthesis of N-[2-[N-[3-[4-(aminomethyl)phenyl]propyl]-N-methylamino]ethyl]-6-quinolinecarboxamide trihydrochloride

<Step 1> Synthesis of N-[2-[N-[3-[4-(N-tert-butoxycarbonylaminomethyl)phenyl]propyl]-N-methylamino]ethyl]-6-quinolinecarboxamide

A reaction was carried out in the same manner as in Step 1 of Example 10 by using the compound prepared in Step 2 of Example 20 (0.10 g) and quinoline-6-carboxylic acid (64.1 mg) to prepare the titled compound (0.10 g, Example Intermediate 81-1).

<Step 2> Synthesis of N-[2-[N-[3-[4-(aminomethyl)phenyl]propyl]-N-methylamino]ethyl]-6-quinolinecarboxamide trihydrochloride

A reaction was carried out in the same manner as in Step 5 of Example 3 by using the compound prepared in Step 1 (0.10 g) to prepare the titled compound (50.0 mg).

(Example 82) Synthesis of N-[2-[N-[3-[4-(aminomethyl)phenyl]propyl]-N-methylamino]ethyl]-2-quinolone-6-carboxamide dihydrochloride

<Step 1> Synthesis of N-[2-[N-[3-[4-(N-tert-butoxycarbonylaminomethyl)phenyl]propyl]-N-methylamino]ethyl]-2-quinolone-6-carboxamide

A reaction was carried out in the same manner as in Step 1 of Example 10 by using the compound prepared in Step 2 of Example 20 (30.0 mg) and 2-quinolone-6-carboxylic acid (19.4 mg) to prepare the titled compound (35.8 mg, Example Intermediate 82-1).

<Step 2> Synthesis of N-[2-[N-[3-[4-(aminomethyl)phenyl]propyl]-N-methylamino]ethyl]-2-quinolone-6-carboxamide dihydrochloride

A reaction was carried out in the same manner as in Step 5 of Example 3 by using the compound prepared in Step 1 (35.8 mg) to prepare the titled compound (25.0 mg).

(Example 83) Synthesis of N-[2-[N-[3-[4-(aminomethyl)phenyl]propyl]-N-formylamino]ethyl]-2-benzo[b]thiophenecarboxamide hydrochloride

<Step 1> Synthesis of N-[2-[N-[3-[4-(N-tert-butoxycarbonylaminomethyl)phenyl]propyl]-N-formylamino]ethyl]-2-benzo[b]thiophenecarboxamide

A reaction was carried out in the same manner as in Step 1 of Example 10 by using the compound prepared in Step 2 of Example 38 (40.2 mg) and formic acid (5.9 mg) to prepare the titled compound (9.4 mg, Example Intermediate 83-1).

<Step 2> Synthesis of N-[2-[N-[3-[4-(aminomethyl)phenyl]propyl]-N-formylamino]ethyl]-2-benzo[b]thiophene carboxamide hydrochloride

A reaction was carried out in the same manner as in Step 5 of Example 3 by using the compound prepared in Step 1 (8.4 mg) to prepare the titled compound (3.6 mg).

(Example 84) Synthesis of N-[2-[N-acetyl-N-[3-[4-(aminomethyl)phenyl]propyl]amino]ethyl]-2-benzo[b]thiophenecarboxamide hydrochloride

<Step 1> Synthesis of N-[2-[N-acetyl-N-[3-[4-(N-tert-butoxycarbonylaminomethyl)phenyl]propyl]amino]ethyl]-2-benzo[b]thiophenecarboxamide

A reaction was carried out in the same manner as in Step 1 of Example 10 by using the compound prepared in Step 2 of Example 38 (40.2 mg) and acetic acid (7.7 mg) to prepare the titled compound (36.7 mg, Example Intermediate 84-1).

<Step 2> Synthesis of N-[2-[N-acetyl-N-[3-[4-(aminomethyl)phenyl]propyl]amino]ethyl]-2-benzo[b]thiophenecarboxamide hydrochloride

A reaction was carried out in the same manner as in Step 5 of Example 3 by using the compound prepared in Step 1 (30.2 mg) to prepare the titled compound (17.8 mg).

(Example 85) Synthesis of N-[3-[4-(aminomethyl)phenyl]propyl]-N-[2-(2-benzo[b]thiophenecarbonylamino)ethyl]aminoacetic acid ditrifluoroacetate

<Step 1> Synthesis of tert-butyl N-[3-[4-(N-tert-butoxycarbonylaminomethyl)phenyl]propyl]-N-[2-(2-benzo[b]thiophenecarbonylamino)ethyl]aminoacetate

Added to a solution of the compound prepared in Step 2 of Example 38 (50 mg) in N,N-dimethylformamide (1 mL) were potassium carbonate (41.4 mg) and tert-butyl bromoacetate (58.5 mg), and the whole was stirred at room temperature for 1 hour. The mixture was purified by means of silica gel column chromatography (eluent; hexane : ethyl acetate=3 : 1) to prepare the titled compound (49.8 mg, Example Intermediate 85-1).

<Step 2> Synthesis of N-[3-[4-(aminomethyl)phenyl]propyl]-N-[2-(2-benzo[b]thiophenecarbonylamino)ethyl]aminoacetic acid ditrifluoroacetate

A reaction was carried out in the same manner as in Step 2 of Example 33 by using the compound prepared in Step 1 (49.0 mg) to prepare the titled compound (55.9 mg).

(Example 86) Synthesis of N-[2-[N-[3-[4-(N-acetylaminomethyl)phenyl]propyl]-N-methylamino]ethyl]-(5-chloro-2-indole)carboxamide hydrochloride

Triethylamine (18.5 μL) was added to a solution of the compound prepared in Example 59 (50.0 mg) in methylene chloride (2 mL), acetyl chloride (9.8 μL) was dropped in the solution, and the whole was stirred at room temperature for 1 hour. A saturated aqueous solution of sodium hydrogen carbonate was added to the mixture, followed by extraction with methylene chloride. The extract was washed with water and a saturated brine, and was dried over anhydrous sodium sulfate, followed by removal of the solvent under reduced pressure. A saturated hydrochloric acid-ethyl acetate (5 mL) was added to the resultant residue, followed by stirring at room temperature for 2 hours. The precipitate was filtered off to prepare the titled compound (48.0 mg).

(Example 87) Synthesis of N-[2-[N-[3-[4-(aminomethyl)phenyl]propyl]-N-methylamino]ethyl]-N-methyl-(5-chloro-2-indole)carboxamide dihydrochloride

<Step 1> Synthesis of N-[2-[N-[3-[4-(N-tert-butoxycarbonylaminomethyl)phenyl]propyl]-N-methylamino]ethyl]-N-(9-fluorenylmethoxycarbonyl)-N-methylamine

A reaction was carried out in the same manner as in Step 1 of Example 20 by using the compound prepared in Step 2 of Example 1 (1.05 g) and N-(9-fluorenylmethoxycarbonyl)-N-methyl-N-[2-(methylamino)ethyl]amine hydrochloride (1.38 g) to prepare the titled compound (1.70 g, Example Intermediate 87-1).

<Step 2> Synthesis of N-[2-[N-[3-[4-(N-tert-butoxycarbonylaminomethyl)phenyl]propyl]-N-methylamino]ethyl]-N-methylamine

A reaction was carried out in the same manner as in Step 2 of Example 20 by using the compound prepared in Step 1 (1.70 g) to prepare the titled compound (0.80 g, Example Intermediate 87-2).

<Step 3> Synthesis of N-[2-[N-[3-[4-(N-tert-butoxycarbonylaminomethyl)phenyl]propyl]-N-methylamino]ethyl]-N-methyl-(5-chloro-2-indole)carboxamide

A reaction was carried out in the same manner as in Step 1 of Example 10 by using the compound prepared in Step 2 (0.15 g) and 5-chloroindole-2-carboxylic acid (0.11 g) to prepare the titled compound (0.20 g, Example Intermediate 87-3).

<Step 4> Synthesis of N-[2-[N-[3-[4-(aminomethyl)phenyl]propyl]-N-methylamino]ethyl]-N-methyl-(5-chloro-2-indole) carboxamide dihydrochloride

A reaction was carried out in the same manner as in Step 5 of Example 3 by using the compound prepared in Step 3 (0.20 g) to prepare the titled compound (0.17 g).

(Example 88) Synthesis of N-[2-[N-[3-[2-aminomethyl-5-pyridyl)propyl]-N-methylamino]ethyl]-2-benzo[b] thiophenecarboxamide trihydrochloride

<Step 1> Synthesis of N-[2-[N-[3-[2-(N-tert-butoxycarbonylaminomethyl)-5-pyridyl]propyl]-N-methylamino]ethyl]-2-benzo[b]thiophenecarboxamide

A reaction was carried out in the same manner as in Step 1 of Example 20 by using the compound prepared in Step 7 of Example 36 (12.1 mg) and benzo[b]thiophene-2-carboxylic acid (13.4 mg) to prepare the titled compound (6.2 mg, Example Intermediate 88-1).

<Step 2> Synthesis of N-[2-[N-[3-(2-aminomethyl-5-pyridyl)propyl]-N-methylamino]ethyl]-2-benzo[b] thiophenecarboxamide trihydrochloride

A reaction was carried out in the same manner as in Step 5 of Example 3 by using the compound prepared in Step 1 (5.0 mg) to prepare the titled compound (2.9 mg).

(Example 89) Synthesis of N-[2-[N-[3-(2-aminomethyl-5-pyridyl)propyl]-N-methylamino]ethyl]-5-indolecarboxamide trihydrochloride

<Step 1> Synthesis of N-[2-[N-[3-[2-(N-tert-butoxycarbonylaminomethyl)-5-pyridyl]propyl]-N-methylamino]ethyl]-5-indolecarboxamide

A reaction was carried out in the same manner as in Step 1 of Example 10 by using the compound prepared in Step 7 of Example 36 (32.1 mg) and indole-5-carboxylic acid (18.6 mg) to prepare the titled compound (32.5 mg, Example Intermediate 89-1).

<Step 2> Synthesis of N-[2-[N-[3-(2-aminomethyl-5-pyridyl)propyl]-N-methylamino]ethyl]-5-indolecarboxamide trihydrochloride

A reaction was carried out in the same manner as in Step 5 of Example 3 by using the compound prepared in Step 1 (29.1 mg) to prepare the titled compound (27.8 mg).

(Example 90) Synthesis of N-[2-[N-[3-[5-(aminomethyl)-2-thienyl]propyl]-N-methylamino]ethyl]-2-benzo[b] thiophenecarboxamide dihydrochloride

<Step 1> Synthesis of 3-[5-(N-tert-butoxycarbonylaminomethyl)-2-thienyl]propanal

Cobalt chloride hexahydrate (0.16 g) was added to a solution of ethyl 3-(5-cyano-2-thienyl)propionate (2.0 g) in methanol (60 mL). Sodium borohydride (1.81 g) was gradually added to the solution under ice-water cooling, and the whole was stirred at room temperature overnight. Water and 1 N hydrochloric acid were added to the mixture, followed by washing with ether. A 1 N aqueous solution of sodium hydroxide was added to an aqueous layer to alkalize the layer, followed by extraction with ethyl acetate. The extract was washed with water and a saturated brine, and was dried over anhydrous sodium sulfate, followed by removal of the solvent under reduced pressure. The resultant residue was dissolved in dioxane (20 mL), and di-tert-butyl dicarbonate (1.02 g) and sodium carbonate (1.42 g) were added to the solution, followed by stirring at 40°C for 1 hour. After the solvent had been removed under reduced pressure, water was added to the residue, followed by extraction with ethyl acetate. The extract was washed with water and a saturated brine, and was dried over anhydrous sodium sulfate, followed by removal of the solvent under reduced pressure. The resultant residue was dissolved in toluene (95 mL), and the solution was cooled to -78°C. Diisobutylaluminum hydride (1.5 M; 9 mL) was gradually dropped in the solution, and the whole was stirred at the temperature for 10 minutes. Methanol was added to the mixture, and furthermore ethyl acetate was added to the solution, followed by stirring. After that, the solution was filtered over celite by suction filtration. After the filtrate had been concentrated, the concentrate was purified by means of silica gel column chromatography (eluent; hexane : ethyl acetate=1 : 1) to

prepare the titled compound (0.37 g, Example Intermediate 90-1).

<Step 2> Synthesis of N-[2-(N-tert-butoxycarbonyl-N-methylamino)ethyl]-2-benzo[b]thiophenecarboxamide

A reaction was carried out in the same manner as in Step 1 of Example 10 by using 2-(N-tert-butoxycarbonyl-N-methylamino)ethylamine (1.0 g) and benzo[b]thiophene-2-carboxylic acid (1.53 g) to prepare the titled compound (1.86 g, Example Intermediate 90-2).

<Step 3> Synthesis of N-[2-(methylamino)ethyl]-2-benzo[b]thiophenecarboxamide hydrochloride

A reaction was carried out in the same manner as in Step 5 of Example 3 by using the compound prepared in Step 2 (1.81 g) to prepare the titled compound (1.31 g, Example Intermediate 90-3).

<Step 4> Synthesis of N-[2-[N-[3-[5-(N-tert-butoxycarbonylaminomethyl)-2-thienyl]propyl]-N-methylamino]ethyl]-2-benzo[b]thiophenecarboxamide

A reaction was carried out in the same manner as in Step 1 of Example 20 by using the compound prepared in Step 1 (44.0 mg) and the compound prepared in Step 3 (44.2 mg) to prepare the titled compound (23.1 mg, Example Intermediate 90-4).

<Step 5> Synthesis of N-[2-[N-[3-[5-(aminomethyl)-2-thienyl]propyl]-N-methylamino]ethyl]-2-benzo[b]thiophenecarboxamide dihydrochloride

A reaction was carried out in the same manner as in Step 5 of Example 3 by using the compound prepared in Step 4 (22.0 mg) to prepare the titled compound (16.5 mg).

(Example 91) Synthesis of N-[2-[N-[3-[4-aminomethyl)phenyl]propyl]-N-methylamino]ethyl]-N-benzo[b]thiophen-2-ylmethylamine trihydrochloride

<Step 1> Synthesis of N-[2-[N-[3-[4-(N-tert-butoxycarbonylaminomethyl)phenyl]propyl]-N-methylamino]ethyl]-N-benzo[b]thiophen-2-ylmethylamine

A reaction was carried out in the same manner as in Step 1 of Example 27 by using the compound prepared in Step 2 of Example 20 (50.0 mg) and 2-benzo[b]thiophenecarbaldehyde (28.5 mg) to prepare the titled compound (20.0 mg, Example Intermediate 91-1).

<Step 2> Synthesis of N-[2-[N-[3-[4-(aminomethyl)phenyl]propyl]-N-methylamino]ethyl]-N-benzo[b]thiophen-2-ylmethylamine trihydrochloride

A reaction was carried out in the same manner as in Step 5 of Example 3 by using the compound prepared in Step 1 (20.0 mg) to prepare the titled compound (12.4 mg).

Table 2 shows the structures of the novel amine derivatives of the present invention prepared in Examples 1 to 91 (hereinafter, abbreviated to 'Example Compounds'). Table 4 shows physical property data for Example Compounds. Table 3 shows the structures of intermediates of Example Compounds (hereinafter, abbreviated to 'Example Intermediates'). Table 5 shows $^1$H-NMR data for Example Intermediates. Table 1 explains abbreviations for the structures of Tables 2 and 3.

It should be noted that, in Table 4, an example compound which was oily and the melting of which could not be measured was defined as an 'oil' and an example compound which had extremely high hygroscopicity and the melting of which could not be measured was defined as a 'hygroscopic solid'. Furthermore, in Examples and Table 5, for example, the phrase 'Example Intermediate 1-1' means an intermediate prepared in 'Step 1 of Example 1'.

Table 1

| Abbreviation | Substituent |
|---|---|
| —Me | $-CH_3$ |
| —Et | $-CH_2CH_3$ |
| —iPr | $-CH(CH_3)_2$ |
| —tBu | $-C(CH_3)_3$ |
| —Ac | 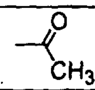 |
| —Ph | 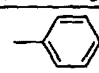 |
| —Boc | $-COOC(CH_3)_3$ |
| —Fmoc | 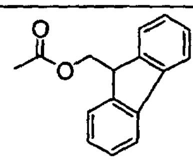 |

# Table 2 ( Part 1 )

Example 1

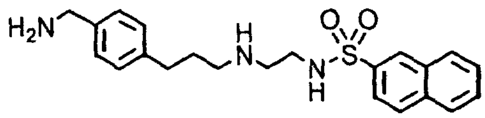

Example 2

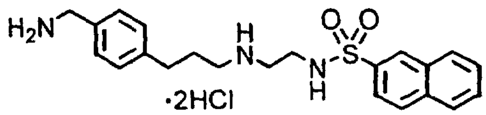

Example 3

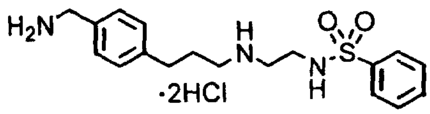

Example 4

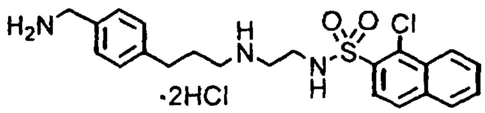

Example 5

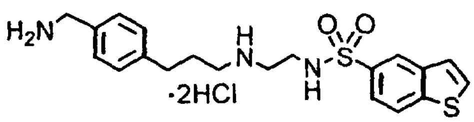

Example 6

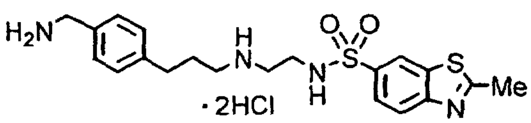

Example 7

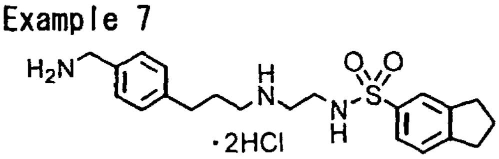

Example 8

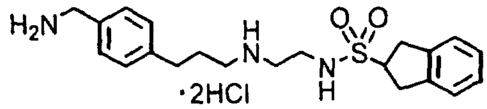

Example 9

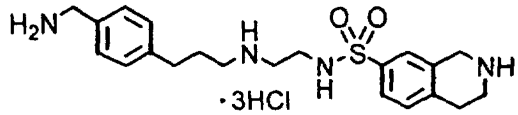

Example 10

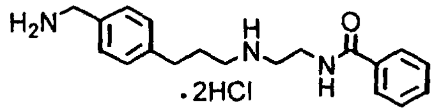

Example 11

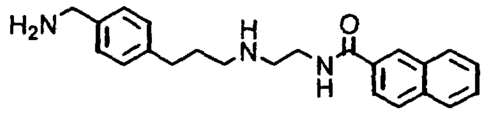

Example 12

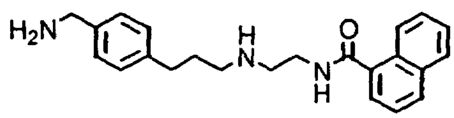

Example 13

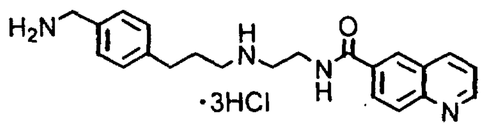

Example 14

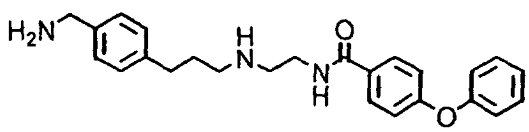

Table 2 ( Part 2 )

Example 15

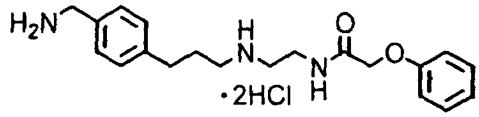

Example 16

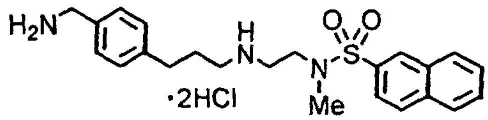

Example 17

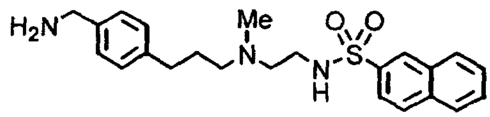

Example 18

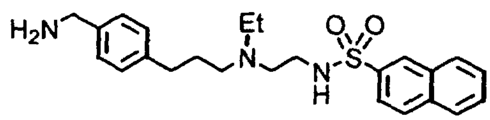

Example 19

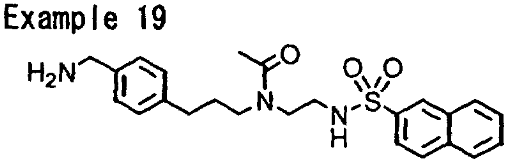

Example 20

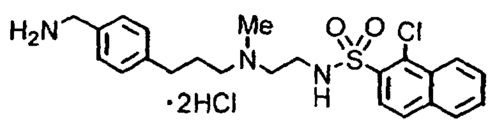

Example 21

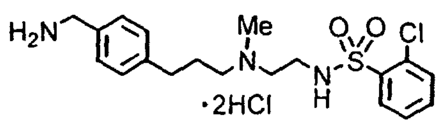

Example 22

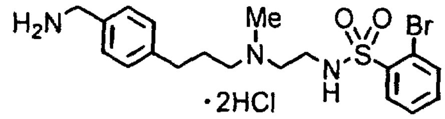

Example 23

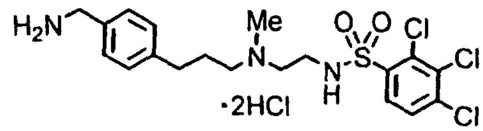

Example 24

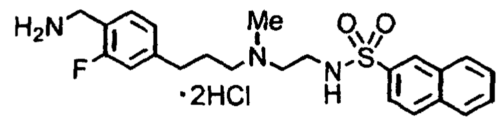

Example 25

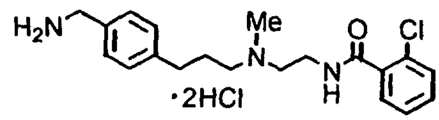

Example 26

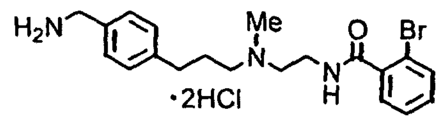

Example 27

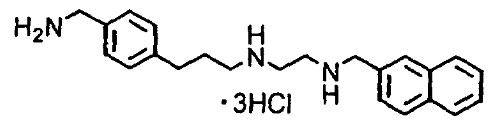

Example 28

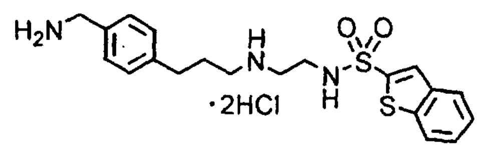

Table 2 ( Part 3 )

Example 29

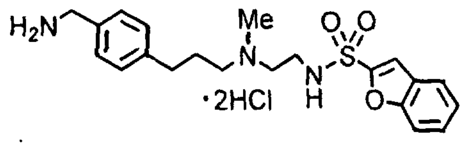

Example 36

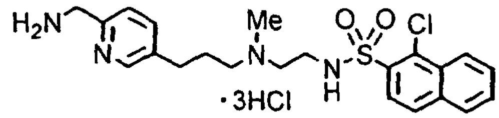

Example 30

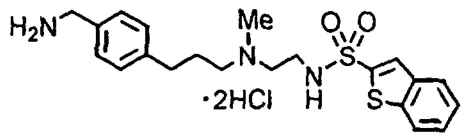

Example 37

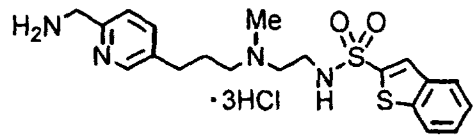

Example 31

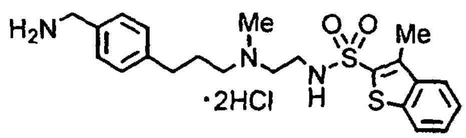

Example 38

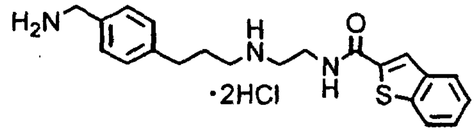

Example 32

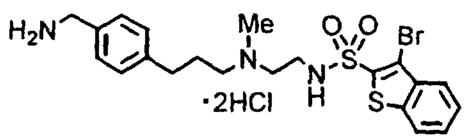

Example 39

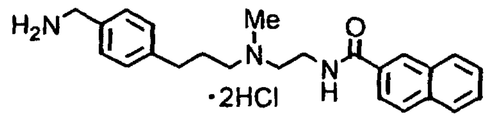

Example 33

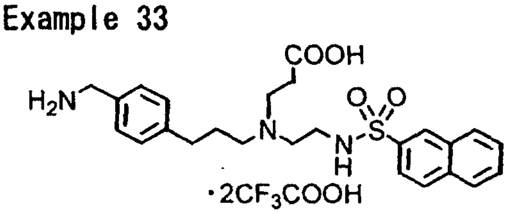

Example 40

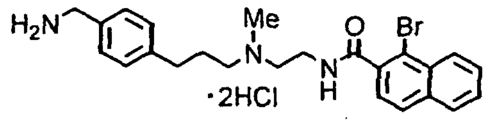

Example 34

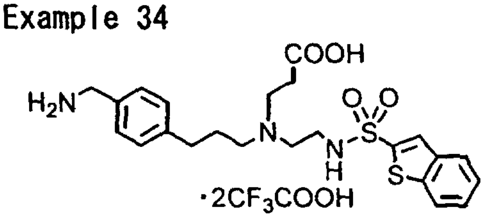

Example 41

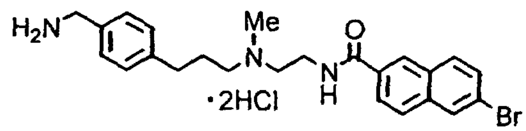

Example 35

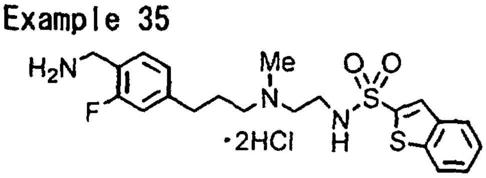

Example 42

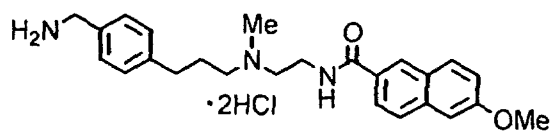

Table 2 ( Part 4 )

Example 43

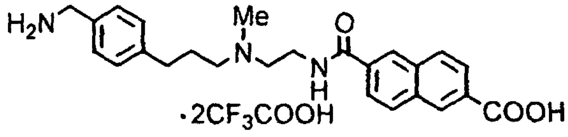

Example 44

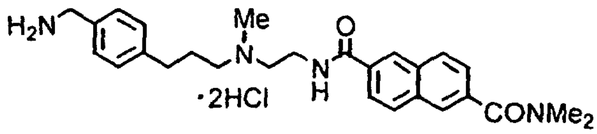

Example 45

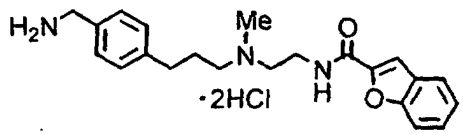

Example 46

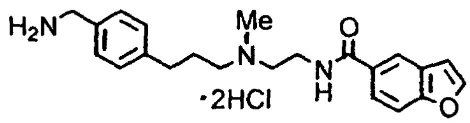

Example 47

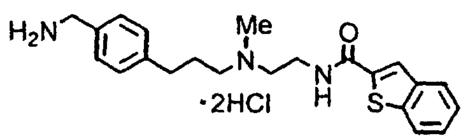

Example 48

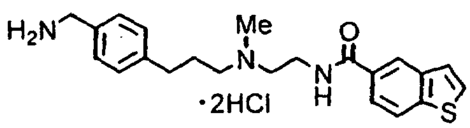

Example 49

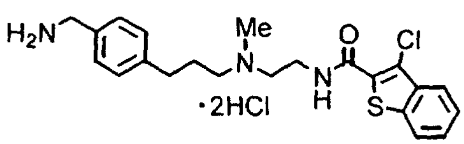

Example 50

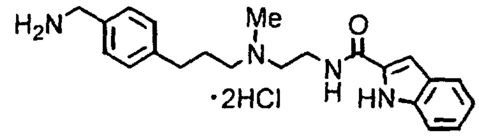

Example 51

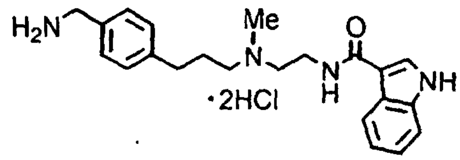

Example 52

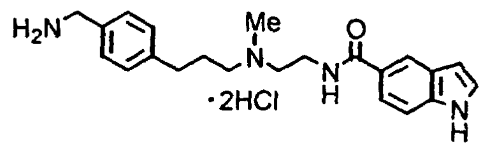

Example 53

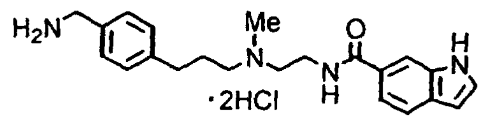

Example 54

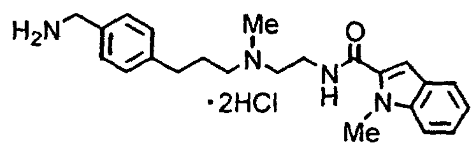

Example 55

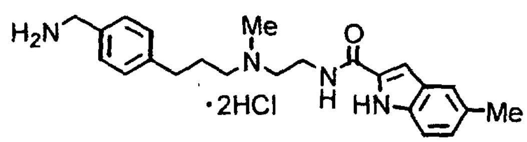

Example 56

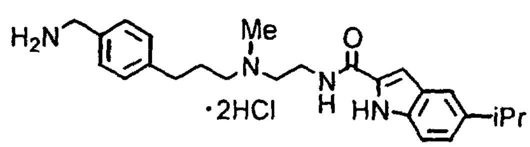

Table 2 ( Part 5 )

Example 57

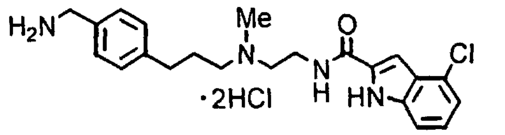

Example 64

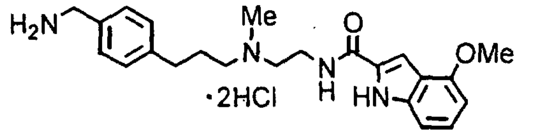

Example 58

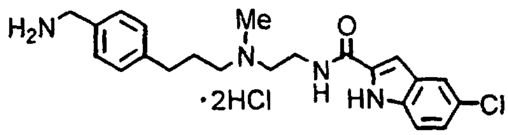

Example 65

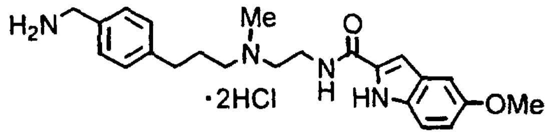

Example 59

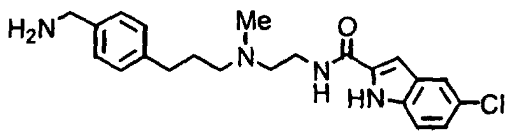

Example 66

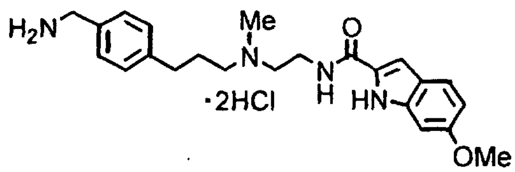

Example 60

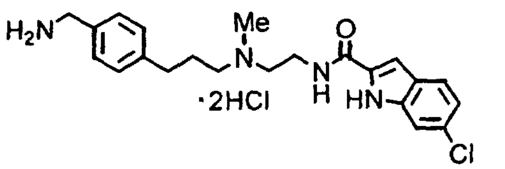

Example 67

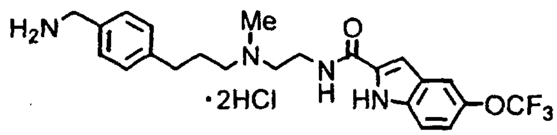

Example 61

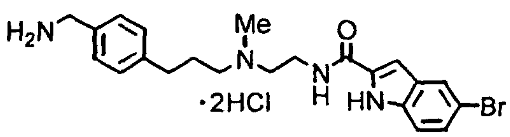

Example 68

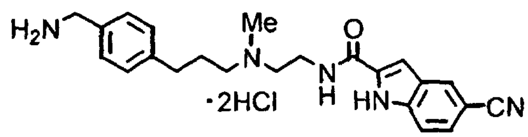

Example 62

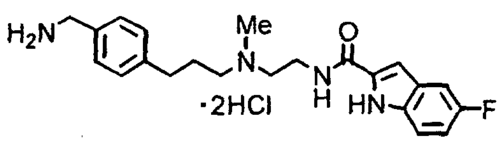

Example 69

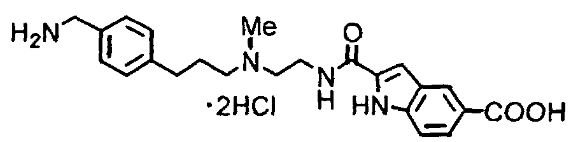

Example 63

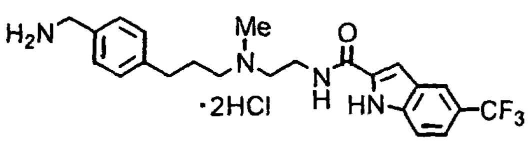

Example 70

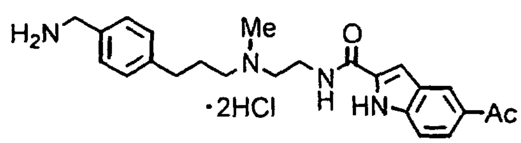

Table 2 ( Part 6 )

Example 71

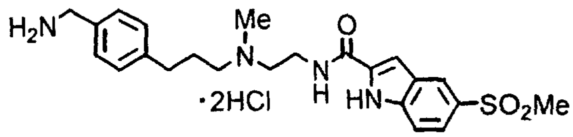

Example 72

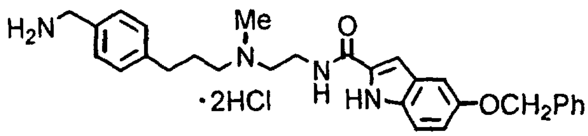

Example 73

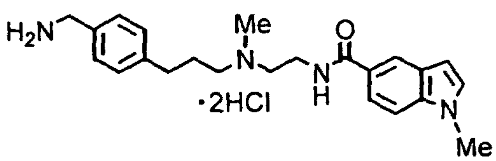

Example 74

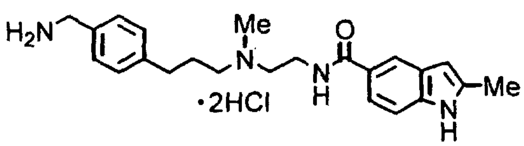

Example 75

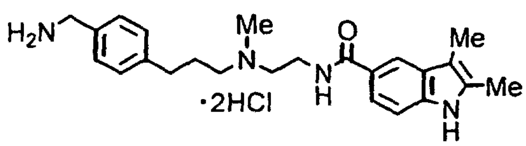

Example 76

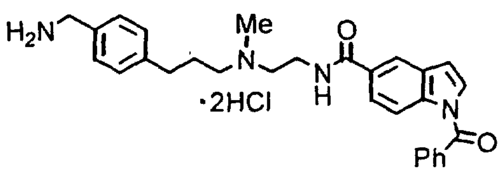

Example 77

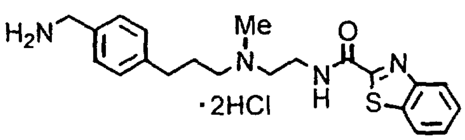

Example 78

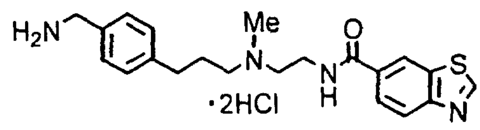

Example 79

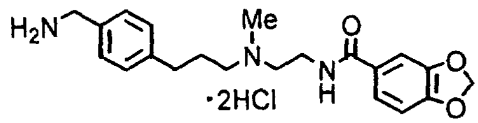

Example 80

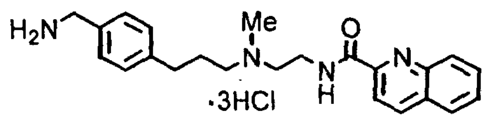

Example 81

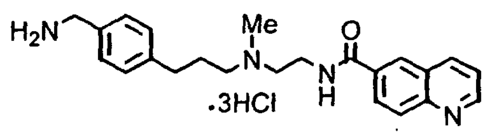

Example 82

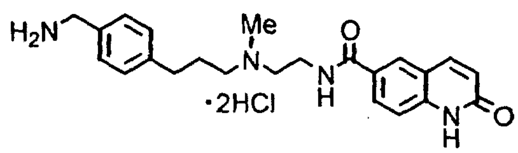

Example 83

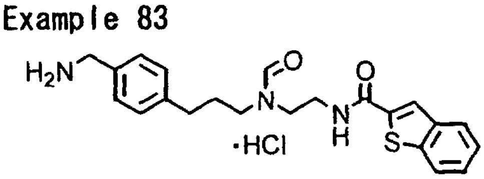

Table 2 ( Part 7 )

Example 84

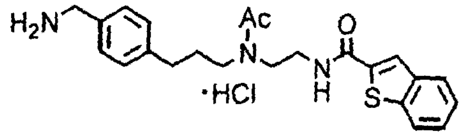

Example 91

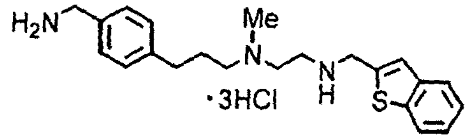

Example 85

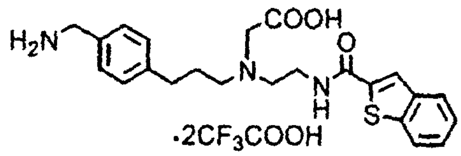

Example 86

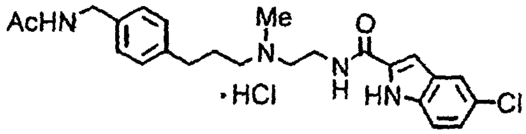

Example 87

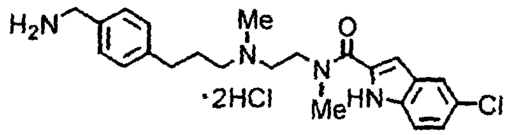

Example 88

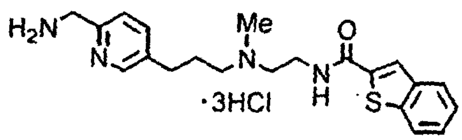

Example 89

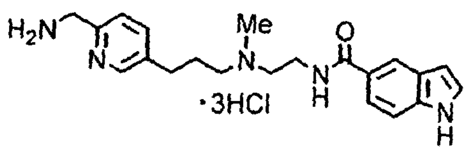

Example 90

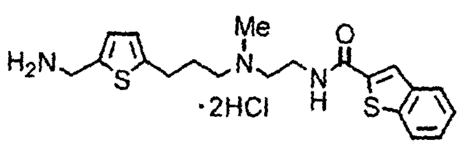

Table 3 ( Part 1 )

Example Intermediate 1-1

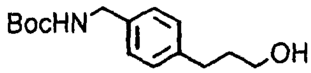

Example Intermediate 1-2

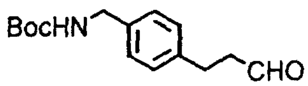

Example Intermediate 1-3

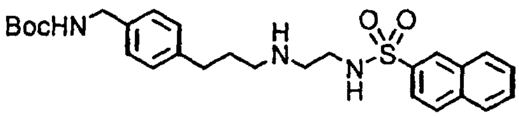

Example Intermediate 3-1

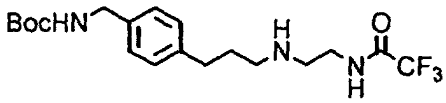

Example Intermediate 3-2

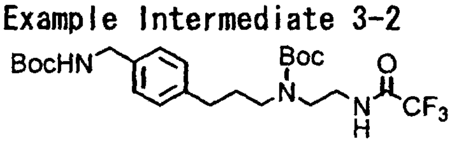

Example Intermediate 3-3

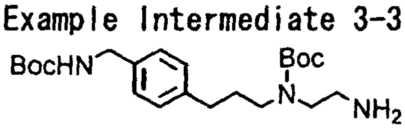

Example Intermediate 3-4

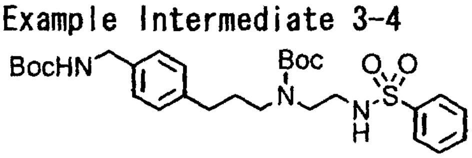

Example Intermediate 4-1

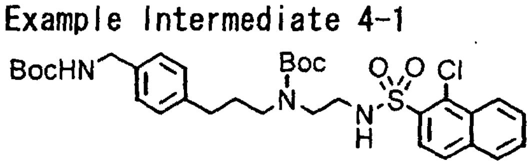

Example Intermediate 5-1

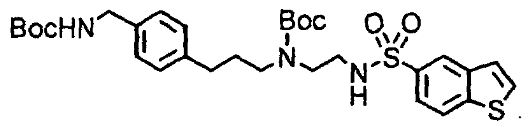

Example Intermediate 6-1

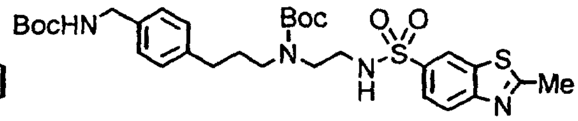

Example Intermediate 7-1

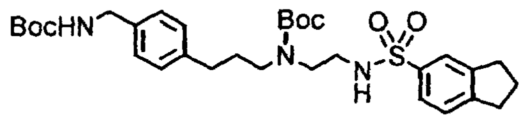

Example Intermediate 8-1

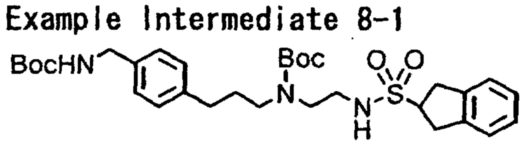

Example Intermediate 9-1

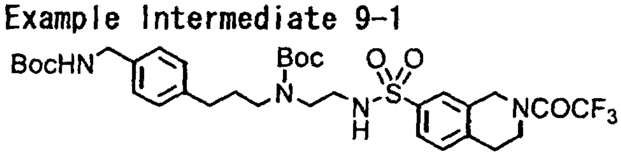

Example Intermediate 9-2

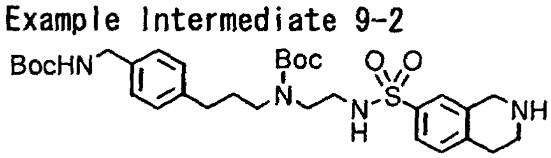

Table 3 ( Part 2 )

Example Intermediate 10-1

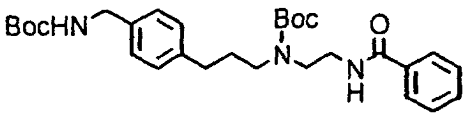

Example Intermediate 11-1

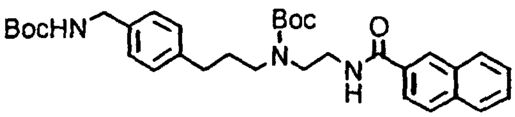

Example Intermediate 12-1

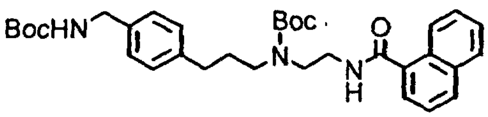

Example Intermediate 13-1

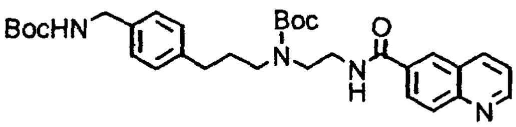

Example Intermediate 14-1

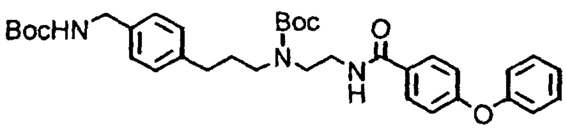

Example Intermediate 15-1

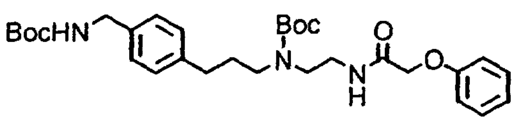

Example Intermediate 16-1

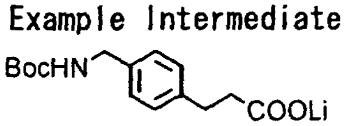

Example Intermediate 16-2

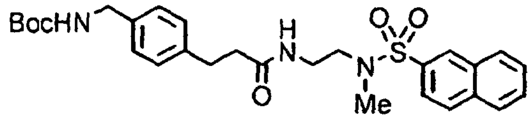

Example Intermediate 17-1

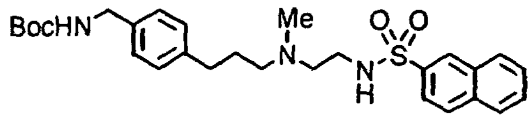

Example Intermediate 18-1

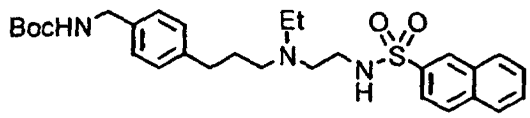

Example Intermediate 19-1

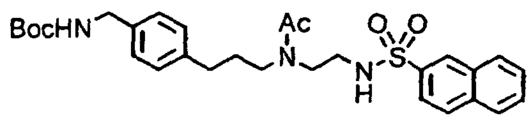

Example Intermediate 20-1

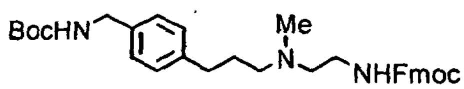

Example Intermediate 20-2

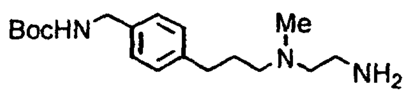

Example Intermediate 20-3

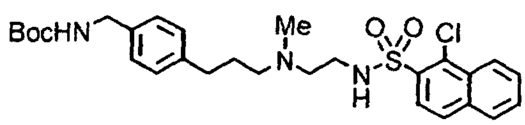

## Table 3 ( Part 3 )

Example Intermediate 21-1

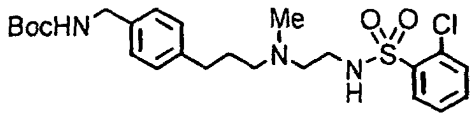

Example Intermediate 22-1

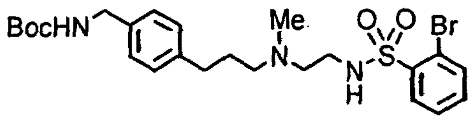

Example Intermediate 23-1

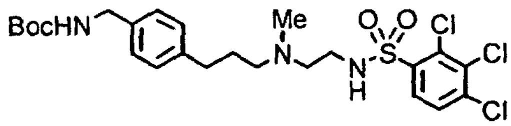

Example Intermediate 24-1

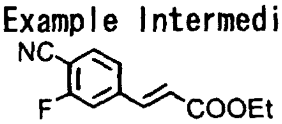

Example Intermediate 24-2

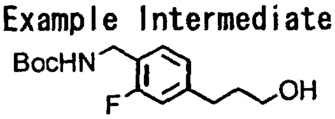

Example Intermediate 24-3

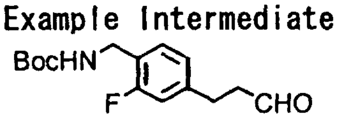

Example Intermediate 24-4

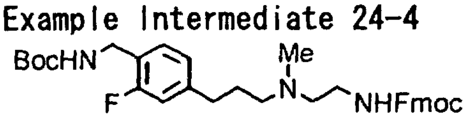

Example Intermediate 24-5

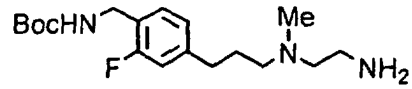

Example Intermediate 24-6

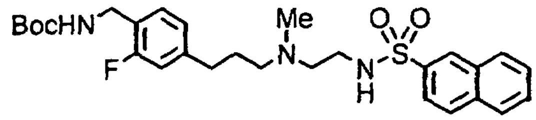

Example Intermediate 25-1

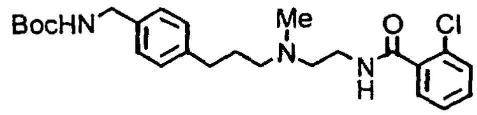

Example Intermediate 26-1

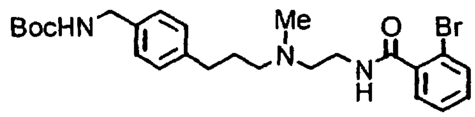

Example Intermediate 27-1

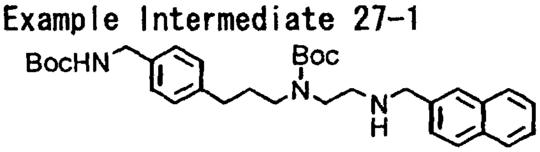

Example Intermediate 28-1

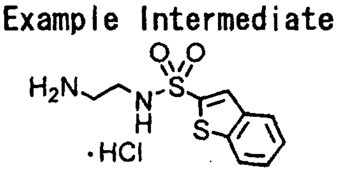

Example Intermediate 28-2

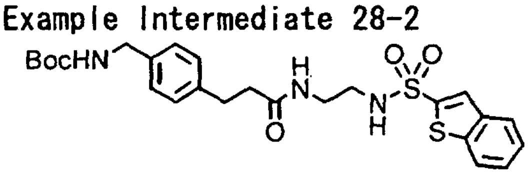

# Table 3 ( Part 4 )

Example Intermediate 28-3

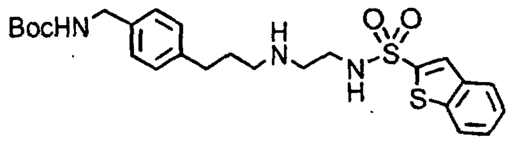

Example Intermediate 29-1

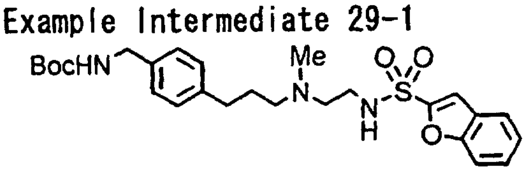

Example Intermediate 30-1

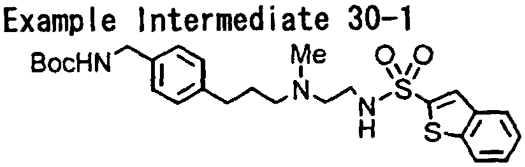

Example Intermediate 31-1

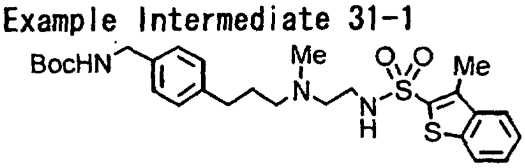

Example Intermediate 32-1

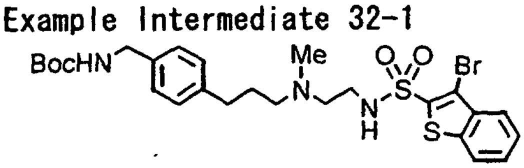

Example Intermediate 33-1

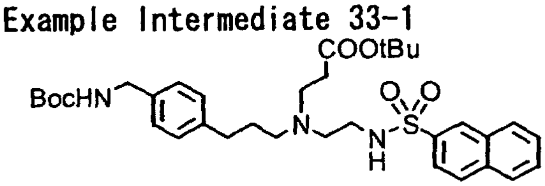

Example Intermediate 34-1

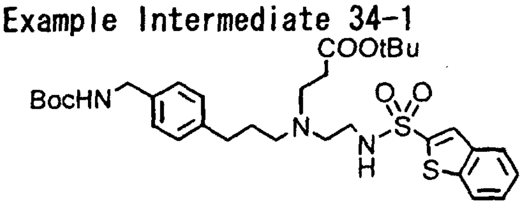

Example Intermediate 35-1

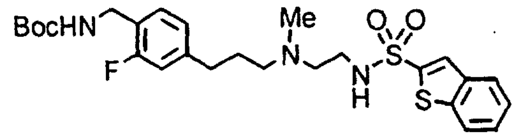

Example Intermediate 36-1

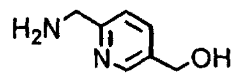

Example Intermediate 36-2

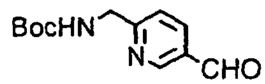

Example Intermediate 36-3

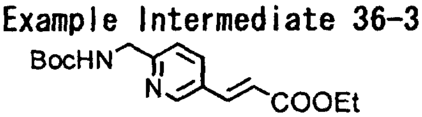

Example Intermediate 36-4

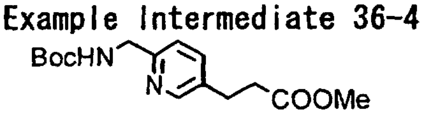

Example Intermediate 36-5

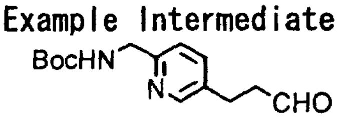

Example Intermediate 36-6

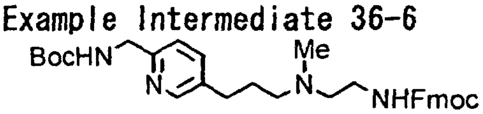

Table 3 ( Part 5 )

Example Intermediate 36-7

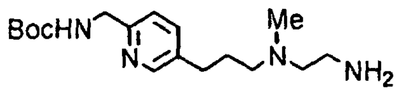

Example Intermediate 41-1

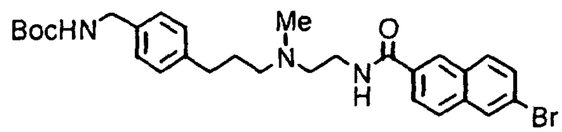

Example Intermediate 36-8

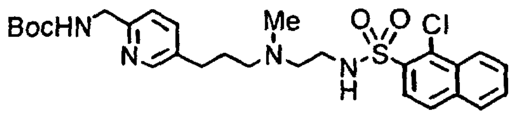

Example Intermediate 42-1

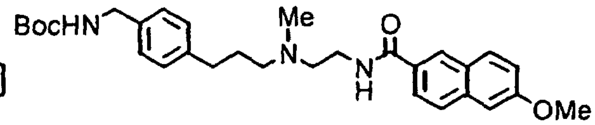

Example Intermediate 37-1

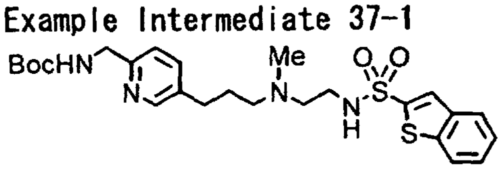

Example Intermediate 43-1

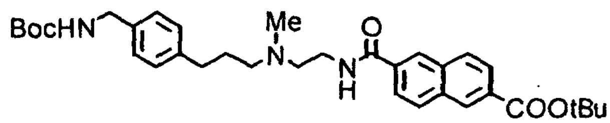

Example Intermediate 38-1

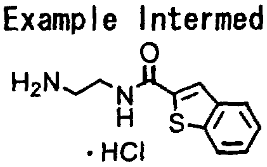

Example Intermediate 44-1

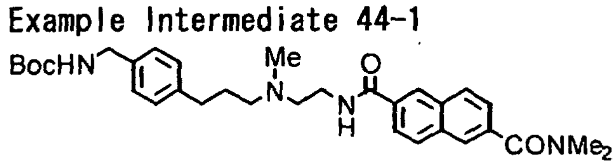

Example Intermediate 38-2

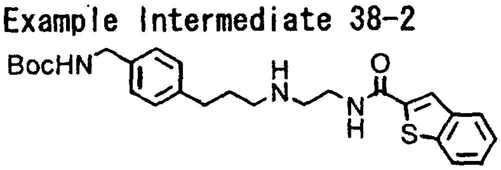

Example Intermediate 45-1

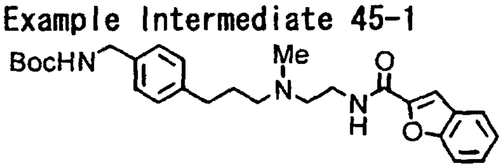

Example Intermediate 39-1

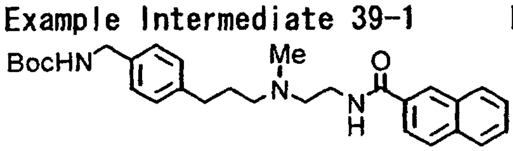

Example Intermediate 46-1

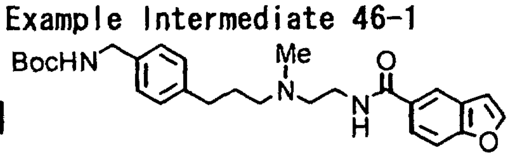

Example Intermediate 40-1

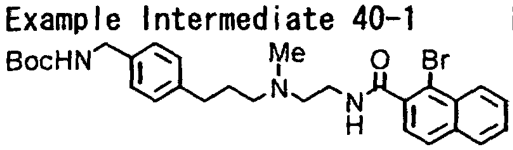

Example Intermediate 47-1

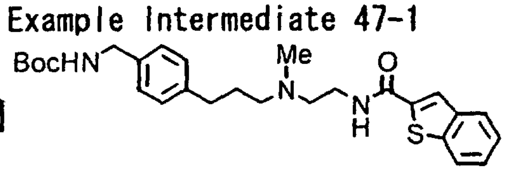

Table 3 ( Part 6 )

Example Intermediate 48-1

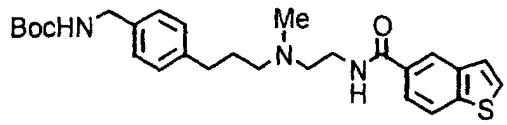

Example Intermediate 55-1

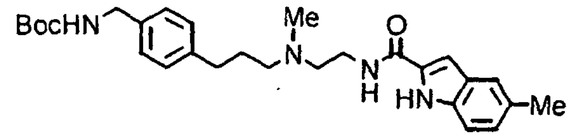

Example Intermediate 49-1

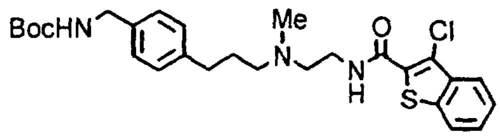

Example Intermediate 56-1

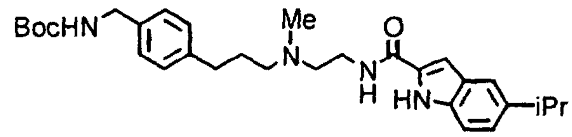

Example Intermediate 50-1

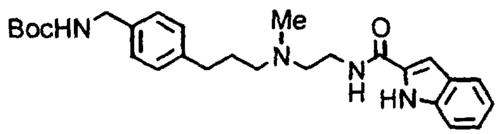

Example Intermediate 57-1

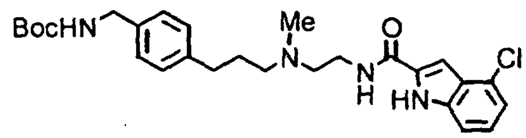

Example Intermediate 51-1

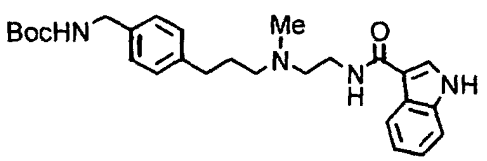

Example Intermediate 58-1

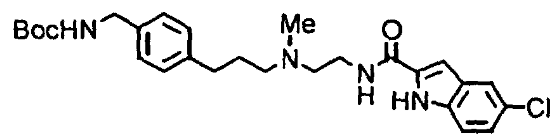

Example Intermediate 52-1

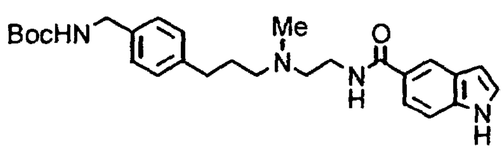

Example Intermediate 60-1

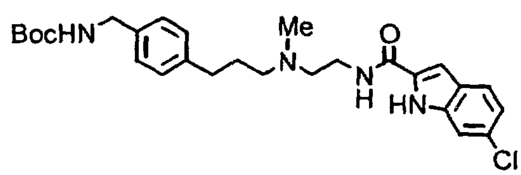

Example Intermediate 53-1

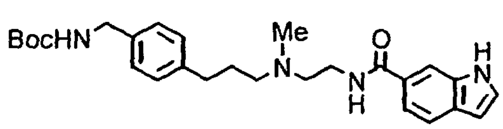

Example Intermediate 61-1

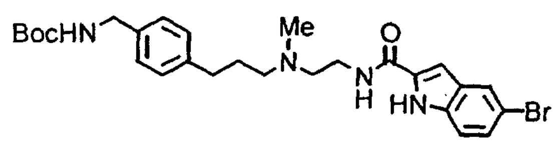

Example Intermediate 54-1

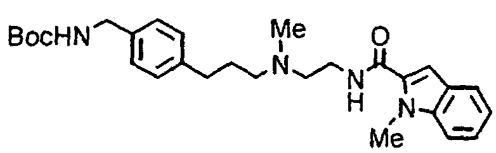

Example Intermediate 62-1

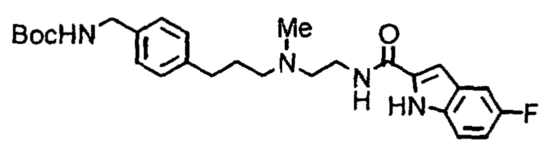

Table 3 ( Part 7 )

Example Intermediate 63-1

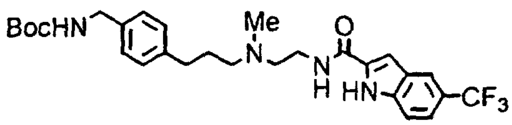

Example Intermediate 64-1

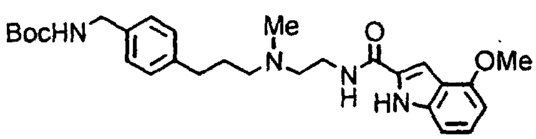

Example Intermediate 65-1

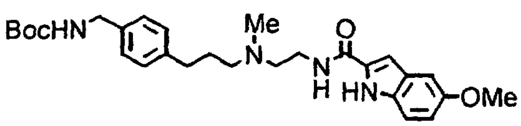

Example Intermediate 66-1

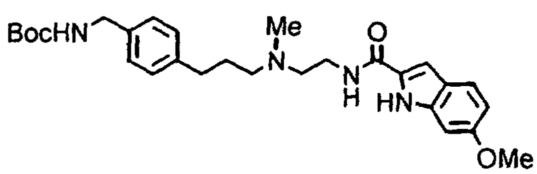

Example Intermediate 67-1

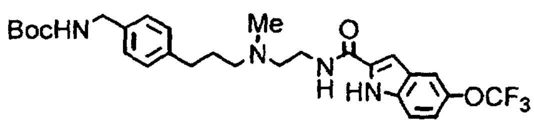

Example Intermediate 68-1

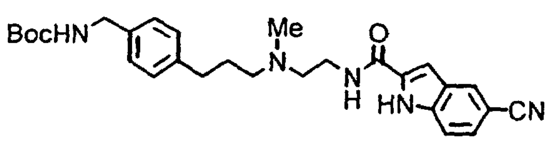

Example Intermediate 69-1

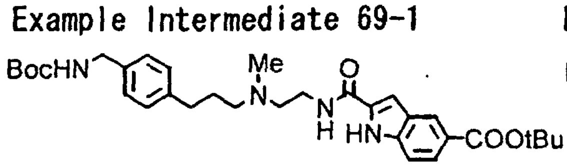

Example Intermediate 70-1

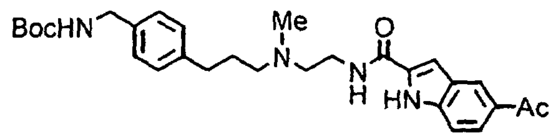

Example Intermediate 71-1

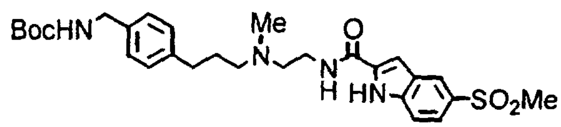

Example Intermediate 72-1

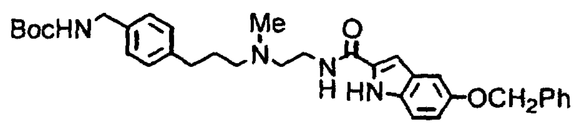

Example Intermediate 73-1

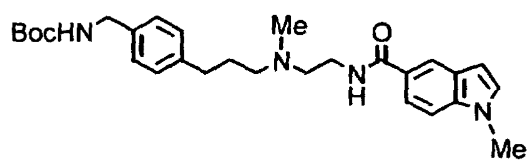

Example Intermediate 74-1

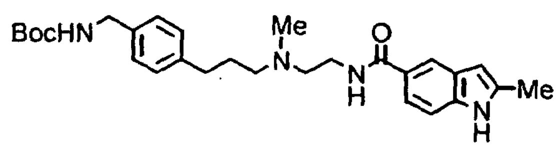

Example Intermediate 75-1

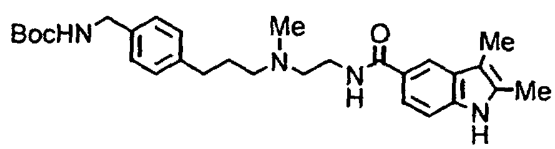

Example Intermediate 76-1

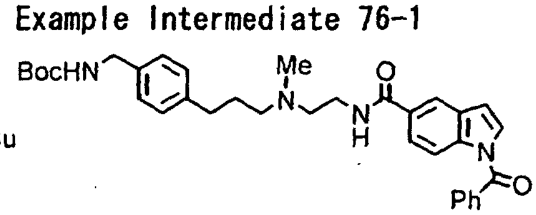

Table 3 ( Part 8 )

Example Intermediate 77-1

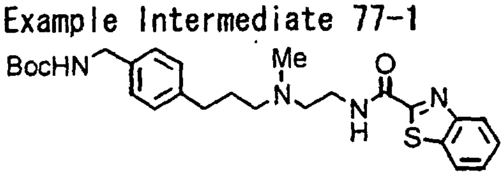

Example Intermediate 78-1

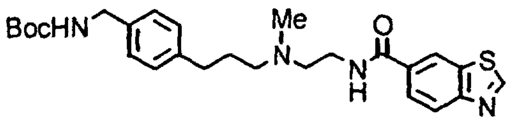

Example Intermediate 79-1

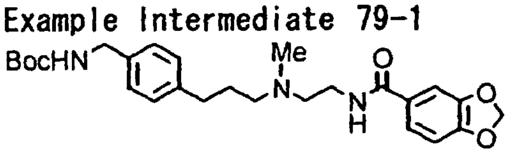

Example Intermediate 80-1

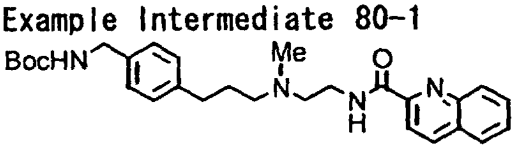

Example Intermediate 81-1

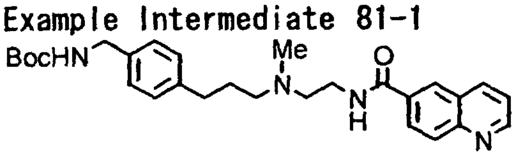

Example Intermediate 82-1

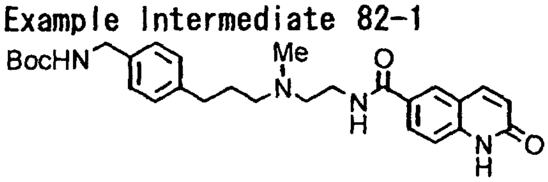

Example Intermediate 83-1

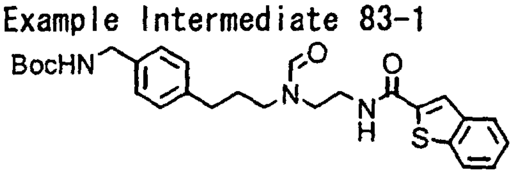

Example Intermediate 84-1

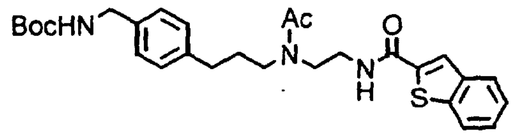

Example Intermediate 85-1

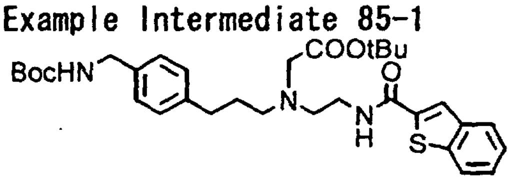

Example Intermediate 87-1

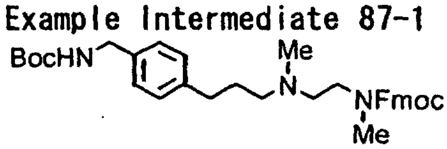

Example Intermediate 87-2

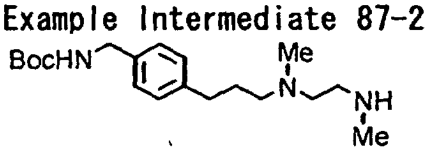

Example Intermediate 87-3

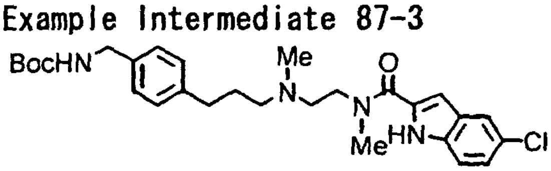

Example Intermediate 88-1

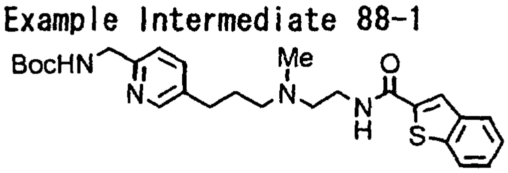

Table 3 ( Part 9 )

Example Intermediate 89-1

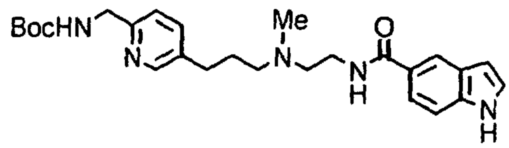

Example Intermediate 90-1

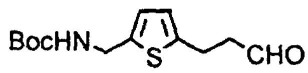

Example Intermediate 90-2

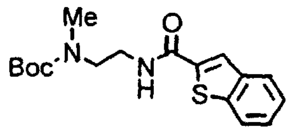

Example Intermediate 90-3

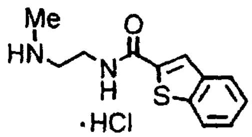

Example Intermediate 90-4

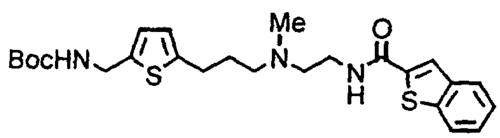

Example Intermediate 91-1

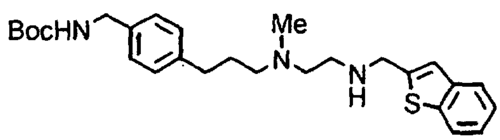

Table 4 ( Part 1 )

| Ex-ample | IR ($cm^{-1}$) | NMR (ppm) (No Mark : 300MHz, * : 270MHz) | Melting Point (°C) |
|---|---|---|---|
| 1 | KBr : 2933, 1473, 1317, 1151, 1076, 654, 550 | $CDCl_3$ : 8.46-8.42(1H,m), 8.01-7.88(3H, m), 7.83(1H, dd, J=2, 9Hz), 7.69-7.58(2H, m), 7.20(2H, d, J=8Hz), 7.08(2H, d, J=8Hz), 3.83(2H, s), 3.05-2.98(2H, m), 2.70-2.62(2H, m), 2.54(2H, t, J=8Hz), 2.44(2H, t, J=7Hz), 1.75-1.61(2H, m) | 68.4-69.0 |
| 2 | KBr : 2993, 1502, 1435, 1317, 1155, 831, 746 | DMSO : 8.97(1H, br.s), 8.52-8.46(1H, m), 8.33(1H, br.s), 8.18(2H, d, J=9Hz), 8.07(1H, d, J=8Hz), 7.86(1H, dd, J=2, 9Hz), 7.78-7.67(2H, m), 7.40(2H, d, J=8Hz), 7.26(2H, d, J=8Hz), 3.98(2H, s), 3.12-2.94(4H, m), 2.88(2H, t, J=8Hz), 2.63(2H, t, J=8Hz), 1.94-1.82(2H, m) | 258.0-260.1 |
| 3 | KBr : 2939, 1446, 1313, 1159, 1093, 640 | DMSO : 8.95(2H, br.s), 8.25(3H, br.s), 8.06(1H, br.s), 7.88-7.78(2H, m), 7.73-7.56(3H, m), 7.40(2H, d, J=8Hz), 7.25(2H, d, J=8Hz), 4.03-3.93(2H,m), 3.10-2.77(6H, m), 2.68-2.57(2H, m), 1.95-1.78(2H, m) | 227.4-228.5 |
| 4 | KBr : 2937, 1500, 1429, 1329, 1165, 818, 687 | DMSO* : 9.04(2H, br.s), 8.55-8.20(5H, m), 8.17(1H, d, J=9Hz), 8.16(1H, d, J=9Hz), 8.04(1H, d, J=9Hz), 7.90-7.75(2H, m), 7.41(2H, d, J=8Hz), 7.25(2H, d, J=8Hz), 3.98(2H, s), 3.25-3.10(2H, m), 3.08-2.80(4H, m), 2.64(2H, t, J=7Hz), 1.97-1.80(2H, m) | 264.3-265.6 |
| 5 | KBr : 2939, 1433, 1313, 1147, 1093, 1045, 702 | DMSO* : 9.00(2H, br.s), 8.39(1H, d, J=2Hz), 8.30(2H, br.s), 8.29(1H, d, J=8Hz), 8.08(1H, br.s), 8.01(1H, d, J=5Hz), 7.77(1H, dd, J=2, 8Hz), 7.67(1H, d, J=5Hz), 7.40(2H, d, J=8Hz), 7.26(2H, d, J=8Hz), 3.98(2H, s), 3.13-2.78(6H, m), 2.63(2H, t, J=8Hz), 1.95-1.80(2H, m) | 264.2-265.4 |
| 6 | KBr : 2939, 1437, 1311, 1159, 1099, 829, 606 | DMSO* : 8.92(2H, br.s), 8.61(1H, d, J=2Hz), 8.35-8.15(3H, m), 8.15-8.05(1H, m), 8.12(1H, d, J=9Hz), 7.90(1H, dd, J=2, 9Hz), 7.40(2H, d, J=8Hz), 7.26(2H, d, J=8Hz), 4.05-3.90(2H, m), 3.15-2.80(6H, m), 2.87(3H, s), 2.64(2H, t, J=8Hz), 1.95-1.80(2H, m) | 257.7-258.8 |

Table 4 ( Part 2 )

| Example | IR ($cm^{-1}$) | NMR (ppm) (No Mark : 300MHz, * : 270MHz) | Melting Point (°C) |
|---|---|---|---|
| 7 | KBr : 2941, 1435, 1313, 1144, 1105, 829 | DMSO* : 9.07(2H, br.s), 8.32(3H, br.s), 8.00-7.85(1H, m), 7.66(1H, s), 7.62-7.53(1H, m), 7.43(1H, d, J=9Hz), 7.40(2H, d, J=8Hz), 7.25(2H, d, J=8Hz), 4.03-3.91(2H, m), 3.07-2.77(10H, m), 2.64(2H, t, J=8Hz), 2.13-1.97(2H, m), 1.96-1.80(2H, m) | 199.0-199.7 |
| 8 | KBr : 2954, 1441, 1319, 1292, 1138, 1109, 741 | DMSO* : 9.09(2H, br.s), 8.31(3H, br.s), 7.64(1H, t, J=6Hz), 7.41(2H, d, J=8Hz), 7.27(2H, d, J=8Hz), 7.24-7.12(4H, m), 4.26-4.12(1H, m), 4.04-3.90(2H, m), 3.40-3.17(6H, m), 3.03-2.82(4H, m), 2.66(2H, t, J=8Hz), 1.98-1.83(2H, m) | 270.7-272.8 |
| 9 | KBr : 3003, 1589, 1496, 1423, 1331, 1151, 1092 | DMSO : 9.37(2H, br.s), 8.44(1H, br.s), 8.17(1H, s), 7.88-7.63(2H, m), 7.60-7.15(5H, m), 4.36(2H, s), 3.98(2H, s), 3.70-2.30(12H, m), 2.10-1.80(2H, m) | 233.9-234.5 |
| 10 | KBr : 2939, 1647, 1539, 1321, 829, 692 | DMSO : 9.02(1H, br.s), 8.84(1H, t, J=6Hz), 8.31(2H, br.s), 7.96-7.87(2H, m), 7.58-7.43(3H, m), 7.40(2H, d, J=8Hz), 7.26(2H, d, J=8Hz), 3.97(2H, s), 3.65-3.53(2H, m), 3.08(2H, t, J=6Hz), 2.92(2H, t, J=7Hz), 2.67(2H, t, J=8Hz), 2.00-1.85(2H, m) | 263.7-272.9 |
| 11 | KBr : 3267, 2929, 1622, 1552, 1308, 1144, 779 | $CDCl_3$ : 8.31(1H, s), 7.94-7.82(4H, m), 7.60-7.48(2H, m), 7.19(2H, d, J=8Hz), 7.15(2H, d, J=8Hz), 7.07-6.98(1H, m), 3.81(2H, s), 3.58(2H, q, J=6Hz), 2.89(2H, t, J=6Hz), 2.69(2H, t, J=6Hz), 2.67(2H, t, J=7Hz), 1.89-1.76(2H, m) | 69.7-72.2 |
| 12 | Liquid Film : 3284, 3049, 1637, 1541, 1308, 783 | $CDCl_3$* : 8.38-8.29(1H, m), 7.96-7.83(2H, m), 7.62(1H, dd, J=1, 7Hz), 7.57-7.42(3H, m), 7.17(2H, d, J=8Hz), 7.12(2H, d, J=8Hz), 6.56(1H, br.s), 3.81(2H, s), 3.70-3.54(2H, m), 2.90(2H, t, J=6Hz), 2.67(2H, t, J=6Hz), 2.65(2H, t, J=7Hz), 1.87-1.72(2H, m) | Oil |

Table 4 ( Part 3 )

| Example | IR ($cm^{-1}$) | NMR (ppm) (No Mark : 300MHz, * : 270MHz) | Melting Point (°C) |
|---|---|---|---|
| 13 | KBr : 2943, 2792, 1657, 1547, 1290, 1209, 771 | DMSO : 9.26(3H, br.s), 9.14(1H, d, J=4Hz), 8.79(1H, s), 8.75(1H, d, J=8Hz), 8.50-8.25 (3H, m), 8.41(1H, d, J=9Hz), 8.23(1H, d, J=9Hz), 7.82(1H, dd, J=4, 8Hz), 7.42(2H, d, J=8Hz), 7.28(2H, d, J=8Hz), 4.05-3.90 (2H, m), 3.78-3.60(2H, m), 3.25-3.07(2H, m), 3.04-2.86(2H, m), 2.70(2H, t, J=8Hz), 2.05-1.88(2H, m) | 269.5-271.2 |
| 14 | Liquid Film: 3290, 1639, 1489, 1242, 1173, 752, 692 | $CDCl_3$* : 7.83-7.72(2H, m), 7.45-7.32(2H, m), 7.26-7.12(4H, m), 7.10-6.96(4H, m), 6.72-6.69(1H, m), 3.83(2H, s), 3.60-3.44(2H, m), 2.85(2H, t, J=6Hz), 2.76-2.60(4H, m), 1.90-1.73(2H, m) | Oil |
| 15 | KBr : 2958, 1664, 1552, 1500, 1248, 748 | DMSO* : 9.08(2H, br.s), 8.55-8.20(4H, m), 7.41(2H, d, J=8Hz), 7.26(2H, d, J=8Hz), 7.35-7.20(2H, m), 7.05-6.90(3H, m), 4.49(2H, s), 4.06-3.90(2H, m), 3.55-3.40(2H, m), 3.10-2.80(4H, m), 2.66(2H, t, J=8Hz), 2.00-1.84(2H, m) | 241.1-245.5 |
| 16 | KBr : 2945, 1439, 1336, 1159, 748, 715, 550 | DMSO* : 9.02(2H, br.s), 8.50(1H, s), 8.31(2H, br.s), 8.23(1H, d, J=7Hz), 8.21(1H, d, J=9Hz), 8.10(1H, d, J=7Hz), 7.81(1H, dd, J=2, 9Hz), 7.78-7.67(2H, m), 7.42(2H, d, J=8Hz), 7.29(2H, d, J=8Hz), 4.06-3.93(2H, m), 3.41-3.27(2H, m), 3.20-3.07(2H, m), 3.02-2.88(2H, m), 2.78(3H, s), 2.67(2H, t, J=8Hz), 2.03-1.87(2H, m) | 225.3-225.9 |
| 17 | KBr : 2798, 1456, 1315, 1163, 1147, 823, 656 | $CDCl_3$ : 8.47-8.42(1H, m), 8.00-7.88(3H, m), 7.82(1H, dd, J=2, 9Hz), 7.70-7.58(2H, m), 7.21(2H, d, J=8Hz), 7.09(2H, d, J=8Hz ), 3.84(2H, s), 2.99(2H, t, J=6Hz), 2.48(2H, t, J=8Hz), 2.36(2H, t, J=6Hz), 2.22(2H, t, J=8Hz), 2.00(3H, s), 1.73-1.60(2H, m) | 91.6-92.0 |
| 18 | Liquid Film: 1462, 1325, 1159, 1076, 752, 658, 550 | $CDCl_3$ : 8.44(1H, s), 7.99-7.88(3H, m), 7.87-7.79(1H, m), 7.69-7.57(2H, m), 7.18(2H, d, J=8Hz), 7.04(2H, d, J=8Hz), 3.83(2H, s), 2.96(2H, t, J=5Hz), 2.50-2.35(4H, m), 2.34(2H, q, J=7Hz), 2.27(2H, t, J=7Hz), 1.68-1.53(2H, m), 0.86(3H, t, J=7Hz) | Oil |

Table 4 ( Part 4 )

| Example | IR ($cm^{-1}$) | NMR (ppm) (No Mark : 300MHz, * : 270MHz) | Melting Point (°C) |
|---|---|---|---|
| 19 | Liquid Film: 1626, 1423, 1325, 1157, 754, 658, 550 | $CDCl_3$ : 8.41(1H, s), 7.99-7.87(3H, m), 7.85-7.78(1H, m), 7.69-7.57(2H, m), 7.21(2H, d, J=8Hz), 7.04(2H, d, J=8Hz), 3.84(2H, s), 3.40(2H, t, J=6Hz), 3.18-3.13(2H, m), 3.08(2H, t, J=8Hz), 2.49(2H, t, J=7Hz), 1.88(3H, s), 1.82-1.66(2H, m) | Oil |
| 20 | KBr : 2952, 1450, 1331, 1173, 1159, 681, 561 | DMSO : 10.37(1H, br.s), 8.50-8.38(2H, m), 8.26(3H, br.s), 8.23-8.10(1H, m), 8.17(1H, d, J=9Hz), 8.04(1H, d, J=9Hz), 7.90-7.78(2H, m), 7.41(2H, d, J=8Hz), 7.27(2H, d, J=8Hz), 4.06-3.90(2H, m), 3.43-2.95(6H, m), 2.75(3H, d, J=5Hz), 2.70-2.45(2H, m), 2.04-1.85(2H, m) | 221.5-222.7 |
| 21 | KBr : 2954, 1618, 1454, 1333, 1163, 766, 586 | DMSO* : 10.51(1H, br.s), 8.27(4H, br.s), 8.03-7.92(1H, m), 7.75-7.63(2H, m), 7.62-7.50(1H, m), 7.41(2H, d, J=8Hz), 7.27(2H, d, J=8Hz), 4.09-3.90(2H, m), 3.50-2.95(6H, m), 2.73(3H, d, J=5Hz), 2.62(2H, t, J=8Hz), 2.05-1.85(2H, m) | 133.8-134.5 |
| 22 | KBr : 2952, 1425, 1331, 1163, 1026, 764, 584 | DMSO* : 10.64(1H, br.s), 8.50-8.15(4H, m), 8.00(1H, dd, J=2, 7Hz), 7.87(1H, dd, J=2, 7Hz), 7.65-7.50(2H, m), 7.41(2H, d, J=8Hz), 7.27(2H, d, J=8Hz), 4.05-3.90(2H, m), 3.50-2.90(6H, m), 2.73(3H, d, J=4Hz), 2.62(2H, t, J=8Hz), 2.05-1.85(2H, m) | Hygroscopic Solid |
| 23 | KBr : 2956, 1618, 1423, 1362, 1167, 839 | DMSO* : 10.73(1H, br.s), 8.65-8.54(1H, m), 8.34(3H, br.s), 7.96(1H, d, J=9Hz), 7.89(1H, d, J=9Hz), 7.41(2H, d, J=8Hz), 7.27(2H, d, J=8Hz), 4.05-3.92(2H, m), 3.60-2.90(6H, m), 2.72(3H, d, J=5Hz), 2.61(2H, t, J=8Hz), 2.05-1.85(2H, m) | Hygroscopic Solid |
| 24 | KBr : 2952, 1433, 1325, 1157, 1132, 980, 552 | DMSO : 10.52(1H, br.s), 8.50(1H, s), 8.38(3H, br.s), 8.23(1H, br.s), 8.17(2H, d, J=8Hz), 8.07(1H, d, J=8Hz), 7.92-7.83(1H, m), 7.78-7.65(2H, m), 7.50(1H, t, J=8Hz), 7.23-7.08(2H, m), 4.03(2H, s), 3.30-2.92(6H, m), 2.72(3H, s), 2.62(2H, t, J=7Hz), 2.04-1.86(2H, m) | 194.6-197.6 |

Table 4 ( Part 5 )

| Example | IR ($cm^{-1}$) | NMR (ppm) (No Mark : 300MHz, * : 270MHz) | Melting Point (°C) |
|---|---|---|---|
| 25 | KBr : 2954, 1649, 1535, 1311, 1128, 756 | DMSO* : 10.54(1H, br.s), 8.80-8.68(1H, m), 8.29(3H, br.s), 7.58-7.40(4H, m), 7.40(2H, d, J=8Hz), 7.28(2H, d, J=8Hz), 4.04-3.90(2H, m), 3.70-3.53(2H, m), 3.50-2.95(4H, m), 2.79(3H, s), 2.64(2H, t, J=8Hz), 2.10-1.90(2H, m) | Hygroscopic Solid |
| 26 | KBr : 2954, 1649, 1535, 1311, 1028, 754 | DMSO* : 10.41(1H, br.s), 8.73(1H, br.s), 8.25(3H, br.s), 7.66(1H, d, J=7Hz), 7.45(1H, d, J=7Hz), 7.55-7.30(2H, m), 7.40(2H, d, J=8Hz), 7.29(2H, d, J=8Hz), 4.08-3.90(2H, m), 3.70-3.50(2H, m), 3.50-2.90(4H, m), 2.80(3H, s), 2.75-2.55(2H, m), 2.10-1.85(2H, m) | Hygroscopic Solid |
| 27 | KBr : 2935, 2767, 1601, 1452, 818, 744, 476 | DMSO* : 10.25-9.25(4H, m), 8.33(3H, br.s), 8.11(1H, s), 8.05-7.83(2H, m), 8.00(1H, d, J=8Hz), 7.79-7.70(1H, m), 7.59(1H, d, J=6Hz), 7.58(1H, d, J=6Hz), 7.42(2H, d, J=8Hz), 7.28(2H, d, J=8Hz), 4.38(2H, s), 3.99(2H, s), 3.60-3.10(4H, m), 3.05-2.81(2H, m), 2.70(2H, t, J=7Hz), 2.05-1.85(2H, m) | 300.5 (Decomposition) |
| 28 | KBr : 2939, 1425, 1327, 1151, 1020, 744, 555 | DMSO * : 9.10-8.74(1H, m), 8.40-8.10(3H, m), 8.06(1H, s), 8.09-8.02(1H, m), 7.62-7.49(2H, m), 7.40(2H, d, J=8Hz), 7.26(2H, d, J=8Hz), 3.99(2H, s), 3.26-3.14(2H, m), 3.10-2.80(4H, m), 2.64(2H, d, J=7Hz), 1.96-1.80(2H, m) | 252.2-256.4 |
| 29 | KBr : 2875, 1444, 1352, 1161, 980, 766, 552 | DMSO* : 10.75-10.55(1H, m), 8.83-8.70(1H,m), 8.50-8.15(3H, m), 7.83(1H, d, J=8Hz), 7.74(1H, d, J=9Hz), 7.65(1H, s), 7.62-7.50(1H, m), 7.42(2H, d, J=8Hz), 7.48-7.36(1H m), 7.27(2H, d, J=8Hz), 4.09-3.90(2H, m), 3.50-2.94(6H, m), 2.75(3H, d, J=2Hz), 2.70-2.53(2H, m), 2.04-1.87(2H, m) | Hygroscopic Solid |

Table 4 ( Part 6 )

| Example | IR ($cm^{-1}$) | NMR (ppm) (No Mark : 300MHz, * : 270MHz) | Melting Point (°C) |
|---|---|---|---|
| 30 | KBr : 2954, 1502, 1333, 1153, 997, 619, 553 | DMSO* : 10.70-10.40(1H, m), 8.63-8.50(1H, m), 8.43-8.20(3H, m), 8.18-8.00(3H, m), 7.63-7.48(2H, m), 7.41(2H, d, J=8Hz), 7.27(2H, d, J=8Hz), 4.07-3.90(2H, m), 3.60-2.95(6H, m), 2.85-2.67(3H, m), 2.69-2.50(2H, m), 2.05-1.85(2H, m) | Hygroscopic Solid |
| 31 | KBr : 3400, 1639, 1323, 1155, 646, 565 | $CD_3OD$ : 8.01-7.90(2H, m), 7.60-7.49(2H, m), 7.42(2H, d, J=8Hz), 7.36(2H, d, J=8Hz), 4.09(2H, s), 3.32-3.29(6H, m), 2.92(3H, s), 2.80-2.70(2H, m), 2.72(3H, s), 2.20-1.99(2H, m) | Hygroscopic Solid |
| 32 | KBr : 2949, 1487, 1336, 1157, 752, 633, 565 | DMSO* : 10.70-10.30(1H, m), 8.90-8.75(1H, m), 8.44-8.10(4H, m), 7.98-7.86(1H, m), 7.74-7.61(2H, m), 7.41(2H, d, J=8Hz), 7.27(2H, d, J=8Hz), 4.08-3.90(2H, m), 3.53-2.97(6H, m), 2.84-2.67(3H, m), 2.70-2.50(2H, m), 2.05-1.85(2H, m) | 231.9-241.2 |
| 33 | Liquid Film : 3014, 1682, 1435, 1329, 1201, 1018, 953 | DMSO : 8.48(1H, s), 8.25-8.00(6H, m), 7.88-7.82(1H, m), 7.79-7.67(2H, m), 7.38(2H, d, J=8Hz), 7.27(2H, d, J=8Hz), 4.10-3.94(2H, m), 3.80-2.95(6H, m), 2.84-2.40(6H, m), 1.98-1.80(2H, m) | Hygroscopic Solid |
| 34 | Liquid Film : 3064, 1674, 1338, 1201, 1153, 723 | $CD_3OD$* : 7.99(1H, s), 8.00-7.93(2H, m), 7.60-7.46(2H, m), 7.41(2H, d, J=8Hz), 7.35(2H, d, J=8Hz), 4.08(2H, s), 3.51(2H, t, J=7Hz), 3.37-3.23(6H, m), 2.82(2H, t, J=7Hz), 2.75(2H, t, J=8Hz), 2.20-2.03(2H, m) | Hygroscopic Solid |
| 35 | KBr : 2875, 1458, 1338, 1155, 760, 621, 552 | DMSO : 10.55-10.40(1H, m), 8.63-8.48(1H, m), 8.45-8.25(3H, m), 8.18-8.01(3H, m), 7.63-7.44(3H, m), 7.24-7.08(2H, m), 4.09-3.98(2H, m), 3.56-2.93(6H, m), 2.75(3H, br.s), 2.69-2.57(2H, m), 2.06-1.87(2H, m) | Hygroscopic Solid |

Table 4 ( Part 7 )

| Example | IR ($cm^{-1}$) | NMR (ppm) (No Mark : 300MHz, * : 270MHz) | Melting Point (℃) |
|---|---|---|---|
| 36 | Liquid Film : 3367, 1622, 1450, 1329, 1161, 1016, 820 | DMSO : 10.30-10.10(1H, m), 8.49(1H, d, J=2Hz), 8.47-8.37(2H, m), 8.37-8.23(3H, m), 8.21-8.09(2H, m), 8.03(1H, d, J=9Hz), 7.88-7.78(2H, m), 7.77-7.69(1H, m), 7.43(1H, d, J=6Hz), 4.22-4.09(2H, m), 3.68-2.95(6H, m), 2.75(3H, d, J=5Hz), 2.69-2.56(2H, m), 2.03-1.87(2H, m) | Hygroscopic Solid |
| 37 | neat : 3400, 1620, 1460, 1335, 1153, 621 | DMSO : 10.53-10.40(1H, m), 8.60-8.53(1H, m), 8.53-8.46(1H, m), 8.45-8.25(3H, m), 8.17-7.98(3H, m), 7.82-7.68(1H, m), 7.61-7.47(2H, m), 7.44(1H, d, J=8Hz), 4.22-4.07(2H, m), 3.64-2.97(6H, m), 2.75(3H, d, J=4Hz), 2.68-2.48(2H, m), 2.03-1.88(2H, m) | Hygroscopic Solid |
| 38 | KBr : 2939, 2792, 1641, 1562, 1543, 1298, 742 | DMSO : 9.20-9.10(1H, m), 9.08-8.80(1H, m), 8.40-8.10(2H, m), 8.20(1H, s), 8.08-8.00(1H, m), 7.97-7.92(1H, m), 7.52-7.41(2H, m), 7.40(2H, d, J=8Hz), 7.28(2H, d, J=8Hz), 3.99(2H, s), 3.68-3.53(2H, m), 3.20-3.04(2H, m), 3.02-2.90(2H, m), 2.76-2.61(2H, m), 2.02-1.85(2H, m) | Hygroscopic Solid |
| 39 | Liquid Film : 3367, 1645, 1541, 1311, 1016, 781 | DMSO : 10.50-10.20(1H, m), 9.12-8.95(1H, m), 8.55(1H, s), 8.40-8.10(3H, m), 8.08-7.93(4H, m), 7.70-7.55(2H, m), 7.38(2H, d, J=8Hz), 7.27(2H, d, J=8Hz), 4.05-3.90(2H, m), 3.83-3.60(2H, m), 3.50-3.00(4H, m), 2.84(3H, br.s), 2.71-2.57(2H, m), 2.10-1.90(2H, m) | Hygroscopic Solid |
| 40 | KBr : 3400, 2951, 1649, 1533, 1458, 1298, 825 | DMSO* : 10.80-10.70(1H, m), 8.95-8.80(1H, m), 8.50-8.15(3H, m), 8.26(1H, d, J=8Hz), 8.05(2H, d, J=8Hz), 7.83-7.64(2H, m), 7.55(1H, d, J=8Hz), 7.43(2H, d, J=8Hz), 7.31(2H, d, J=8Hz), 4.06-3.90(2H, m), 3.80-3.60(2H, m), 3.50-3.00(4H, m), 2.83(3H, s), 2.73-2.60(2H, m), 2.13-1.95(2H, m) | Hygroscopic Solid |

Table 4 ( Part 8 )

| Example | IR ($cm^{-1}$) | NMR (ppm) (No Mark : 300MHz, * : 270MHz) | Melting Point (°C) |
|---|---|---|---|
| 41 | neat : 3400, 1641, 1624, 1545, 1308, 808 | DMSO* : 10.75-10.45(1H, m), 9.21-9.07(1H, m), 8.60(1H, s), 8.46-8.20(3H, m), 8.31(1H, d, J=2Hz), 8.12-7.94(3H, dd, J=2, 9Hz), 7.73(1H, dd, J=2, 9Hz), 7.39(2H, d, J=8Hz), 7.26(2H, d, J=8Hz), 3.96(2H, s), 3.80-3.63(2H, m), 3.50-3.00(4H, m), 2.82(3H, s), 2.64(2H, t, J=7Hz), 2.10-1.92(2H, m) | Hygroscopic Solid |
| 42 | KBr : 3400, 2958, 1630, 1537, 1390, 1219, 1026 | DMSO* : 10.40-10.10(1H, m), 9.03-8.86(1H, m), 8.46(1H, s), 8.35-8.10(3H, m), 8.00-7.82(3H, m), 7.38(2H, d, J=7Hz), 7.27(2H, d, J=7Hz), 7.32-7.17(2H, m), 4.03-3.90(2H, m), 3.91(3H, s), 3.67-3.40(2H, m), 3.42-3.00(4H, m), 2.84(3H, br.s), 2.70-2.58(2H, m), 2.08-1.90(2H, m) | Hygroscopic Solid |
| 43 | neat : 3400, 1680, 1543, 1201, 1138, 723 | DMSO : 13.40-13.13(1H, m), 9.70-9.50(1H, m), 9.08-8.95(1H, m), 8.67(1H, s), 8.53(1H, s), 8.30-7.94(7H, m), 7.37(2H, d, J=8Hz), 7.28(2H, d, J=8Hz), 4.06-3.90(2H, m), 3.80-3.60(2H, m), 3.58-3.00(4H, m), 2.88(3H, s), 2.70-2.55(2H, m), 2.05-1.85(2H, m) | Hygroscopic Solid |
| 44 | KBr : 3400, 1612, 1543, 1510, 1309, 1180, 827 | DMSO : 10.60(1H, br.s), 9.21-9.12(1H, m), 8.61(1H, s), 8.33(3H, br.s), 8.13-7.97(4H, m), 7.60(1H, dd, J=1, 8Hz), 7.39(2H, d, J=8Hz), 7.27(2H, d, J=8Hz), 4.03-3.92(2H, m), 3.80-3.60(2H, m), 3.44-3.05(4H, m), 3.05(3H, br.s), 2.96(3H, br.s), 2.84(3H, d, J=5Hz), 2.71-2.58(2H, m), 2.10-1.93(2H, m) | 147.1-149.1 |
| 45 | Liquid Film : 3392, 1647, 1541, 1508, 1456, 750, 420 | DMSO : 10.76-10.48(1H, m), 9.30-9.00(1H, m), 8.66-8.20(3H, m), 7.91-7.10(9H, m), 4.10-3.85(2H, m), 3.80-3.56(2H, m), 3.54-2.95(4H, m), 2.81(3H, s), 2.79-2.45(2H, m), 2.15-1.85(2H, m) | Hygroscopic Solid |

Table 4 ( Part 9 )

| Ex-ample | IR ($cm^{-1}$) | NMR (ppm) (No Mark : 300MHz, * : 270MHz) | Melting Point (°C) |
|---|---|---|---|
| 46 | neat : 3400, 2925, 1639, 1547, 1464, 1306, 762 | DMSO : 10.36-10.22(1H, m), 8.98-8.84(1H, m), 8.37-8.13(4H, m), 8.10(1H, d, J=2Hz), 7.94-7.84(1H, m), 7.69(1H, d, J=9Hz), 7.39(2H, d, J=8Hz), 7.26(2H, d, J=8Hz), 7.08(1H, d, J=1Hz), 4.03-3.93(2H, m), 3.74-3.62(2H, m), 3.42-2.98(4H, m), 2.82(3H, br.s), 2.67-2.58(2H, m), 2.08-1.89(2H, m) | Hygroscopic Solid |
| 47 | KBr : 3400, 2949, 1630, 1562, 1543, 1300, 762 | DMSO* : 9.36-9.21(1H, m), 8.50-8.15(3H, m), 8.23(1H, s), 8.07-7.87(2H, m), 7.56-7.35(2H, m), 7.38(2H, d, J=8Hz), 7.25(2H, d, J=8Hz), 4.06-3.85(2H, m), 3.80-3.55(2H, m), 3.52-3.00(4H, m), 2.80(3H, s), 2.63(2H, t, J=7Hz), 2.10-1.90(2H, m) | Hygroscopic Solid |
| 48 | KBr : 3400, 1639, 1547, 1331, 1298, 756 | DMSO* : 10.45-10.26(1H, m), 9.04-8.90(1H, m), 8.46(1H, s), 8.36-8.16(3H, m), 8.12(1H, d, J=9Hz), 7.95-7.80(2H, m), 7.56(1H, d, J=6Hz), 7.39(2H, d, J=8Hz), 7.26(2H, d, J=8Hz), 4.02-3.93(2H, m), 3.75-3.63(2H, m), 3.48-2.97(4H, m), 2.82(3H, br.s), 2.69-2.57(2H, m), 2.08-1.89(2H, m) | Hygroscopic Solid |
| 49 | KBr : 3359, 2952, 1639, 1529, 1498, 1311, 748 | DMSO : 10.70-10.50(1H, m), 8.82-8.68(1H, m), 8.40-8.25(3H, m), 8.17-8.07(1H, m), 7.96-7.87(1H, m), 7.67-7.56(2H, m), 7.40(2H, d, J=8Hz), 7.29(2H, d, J=8Hz), 4.03-3.98(2H, m), 3.82-3.66(2H, m), 3.45-2.97(4H, m), 2.82(3H, br.s), 2.65(2H, t, J=8Hz), 2.08-1.93(2H, m) | Hygroscopic Solid |
| 50 | KBr : 3400, 1637, 1556, 1421, 1311, 1261, 752 | DMSO : 11.67(1H, s), 10.50-10.35(1H, m), 9.03-8.90(1H, m), 8.40-8.14(3H, m), 7.62(1H, d, J=8Hz), 7.44(1H, d, J=8Hz), 7.38(2H, d, J=8Hz), 7.26(2H, d, J=8Hz), 7.24-7.16(2H, m), 7.04(1H, t, J=7Hz), 4.05-3.90(2H, m), 3.82-3.58(2H, m), 3.47-3.01(4H, m), 2.82(3H, br.s), 2.69-2.57(2H, m), 2.08-1.92(2H, m) | Hygroscopic Solid |

Table 4 ( Part 10 )

| Ex-ample | IR ($cm^{-1}$) | NMR (ppm) (No Mark : 300MHz, * : 270MHz) | Melting Point (°C) |
|---|---|---|---|
| 51 | KBr : 3400, 1620, 1543, 1456, 1431, 1211, 756 | DMSO* : 11.67(1H, s), 10.50-10.30(1H, m), 8.47-8.20(4H, m), 8.19-8.11(2H, m), 7.48-7.41(1H, m), 7.34(2H, d, J=9Hz), 7.26(2H, d, J=9Hz), 7.22-7.06(2H, m), 4.02-3.93(2H, m), 3.74-3.57(2H, m), 3.48-2.96(4H, m), 2.82(3H, br.s), 2.64(2H, t, J=8Hz), 2.11-1.89(2H, m) | Hygroscopic Solid |
| 52 | KBr : 3400, 2952, 1610, 1539, 1471, 1311, 760 | $CD_3OD$ : 8.19(1H, d, J=2Hz), 7.68(1H, dd, J=2, 9Hz), 7.45(1H, d, J=9Hz), 7.42-7.28(5H, m), 6.57(1H, d, J=3Hz), 4.05(2H, s), 3.78(2H, t, J=6Hz), 3.57-3.43(2H, m), 3.43-3.28(2H, m), 2.98(3H, s), 2.75(2H, t, J=7Hz), 2.18-2.03(2H, m) | Hygroscopic Solid |
| 53 | neat : 3400, 2958, 1620, 1545, 1460, 1317, 779 | DMSO : 11.47(1H, s), 10.27-10.14(1H, m), 8.81-8.68(1H, m), 8.37-8.16(3H, m), 8.00(1H, s), 7.63-7.49(3H, m), 7.38(2H, d, J=8Hz), 7.26(2H, d, J=8Hz), 6.52-6.47(1H, m), 4.03-3.93(2H, m), 3.73-3.58(2H, m), 3.48-3.02(4H, m), 2.84(3H, d, J=5Hz), 2.68-2.57(2H, m), 2.06-1.91(2H, m) | Hygroscopic Solid |
| 54 | KBr : 3400, 1639, 1541, 1464, 1271, 752 | DMSO : 10.47-10.30(1H, m), 8.94-8.78(1H, m), 8.40-8.17(3H, m), 7.65(1H, d, J=8Hz), 7.54(1H, d, J=8Hz), 7.38(2H, d, J=8Hz), 7.33-7.23(3H, m), 7.21(1H, s), 7.11(1H, t, J=8Hz), 4.00(3H, s), 4.04-3.93(2H, m), 3.74-3.58(2H, m), 3.48-3.02(4H, m), 2.82(3H, br.s), 2.70-2.58(2H, m), 2.07-1.93(2H, m) | Hygroscopic Solid |
| 55 | KBr : 3367, 1637, 1554, 1325, 1257, 1126, 806 | DMSO : 11.54(1H, s), 10.50-10.30(1H, m), 9.00-8.81(1H, m), 8.40-8.12(3H, m), 7.42-7.35(1H, m), 7.38(2H, d, J=8Hz), 7.32(1H, d, J=8Hz), 7.26(2H, d, J=8Hz), 7.14-7.09(1H, m), 7.05-6.99(1H, m), 4.05-3.90(2H, m), 3.80-3.55(2H, m), 3.45-3.00(4H, m), 2.83(3H, d, J=5Hz), 2.70-2.55(2H, m), 2.36(3H, s), 2.08-1.91(2H, m) | Hygroscopic Solid |

Table 4 ( Part 11 )

| Ex-ample | IR ($cm^{-1}$) | NMR (ppm) (No Mark : 300MHz, * : 270MHz) | Melting Point (°C) |
|---|---|---|---|
| 56 | KBr : 2956, 1631, 1552, 1255, 818, 748 | DMSO : 11.54(1H, s), 10.80-10.50(1H, m), 9.10-8.85(1H, m), 8.52-8.14(3H, m), 7.43(1H, s), 7.39(2H, d, J=8Hz), 7.35(1H, s), 7.26(2H, d, J=8Hz), 7.20-7.15(1H, m), 7.14-7.07(1H, m), 4.05-3.87(2H, m), 3.80-3.58(2H, m), 3.50-3.00(4H, m), 3.00-2.88(1H, m), 2.88-2.75(3H, m), 2.70-2.57(2H, m), 2.10-1.90(2H, m), 1.24(6H, d, J=7Hz) | 157.9-159.3 |
| 57 | KBr : 2949, 1637, 1552, 1425, 1296, 1261, 766 | DMSO : 10.54-10.34(1H, m), 8.70-8.53(1H, m), 8.50-8.14(3H, m), 7.40(2H, d, J=8Hz), 7.26(2H, d, J=8Hz), 7.43-7.20(3H, m), 7.00-6.86(1H, m), 4.05-3.90(2H, m), 3.80-2.90(6H, m), 2.80(3H, d, J=5Hz), 2.70-2.57(2H, m), 2.05-1.90(2H, m) | Hygroscopic Solid |
| 58 | KBr : 2949, 1639, 1552, 1321, 1254, 808 | DMSO* : 11.89(1H, s), 10.60-10.40(1H, m), 9.10-9.00(1H, m), 8.40-8.20(3H, ms), 7.71(1H, d, J=1Hz), 7.47-7.35(3H, m), 7.31-7.13(4H, m), 4.06-3.90(2H, m), 3.82-3.58(2H, m), 3.47-2.96(4H, m), 2.82(3H, br.s), 2.70-2.57(2H, m), 2.13-1.89(2H, m) | 166.4-170.0 |
| 59 | KBr : 3244, 1639, 1554, 1415, 1263, 916, 802 | $CDCl_3$ : 9.80-9.40(1H, m), 7.59(1H, d, J=2Hz), 7.36(1H, d, J=9Hz), 7.22(1H, dd, J=2, 9Hz), 7.20(2H, d, J=8Hz), 7.13(2H, d, J=8Hz), 7.03-6.93(1H, m), 6.75(1H, s), 3.83(2H, s), 3.58-3.46(2H, m), 2.73-2.56(4H, m), 2.51-2.40(2H, m), 2.29(3H, s), 1.90-1.75(2H, m) | 112.4-113.9 |
| 60 | KBr : 2951, 1639, 1550, 1315, 1248, 1061, 831 | DMSO : 11.82(1H, s), 10.67-10.45(1H, m), 9.14-9.00(1H, m), 8.45-8.18(3H, m), 7.65(1H, d, J=9Hz), 7.45-7.41(1H, m), 7.37(2H, d, J=8Hz), 7.25(2H, d, J=8Hz), 7.05(1H, dd, J=2, 9Hz), 4.03-3.87(2H, m), 3.80-3.56(2H, m), 3.40-3.00(4H, m), 2.85-2.75(3H, m), 2.68-2.55(2H, m), 2.08-1.90(2H, m) | 183.1-186.5 |

Table 4 ( Part 12 )

| Example | IR ($cm^{-1}$) | NMR (ppm) (No Mark : 300MHz, * : 270MHz) | Melting Point (°C) |
|---|---|---|---|
| 61 | Liquid Film : 3392, 1647, 1556, 1543, 1473, 1417, 1319 | DMSO* : 11.90(1H, s), 10.57-10.38(1H, m), 9.12-8.94(1H, m), 8.43-8.17(3H, m), 7.86(1H, d, J=1Hz), 7.46-7.33(3H, m), 7.33-7.15(4H, m), 4.06-3.90(2H, m), 3.82-3.56(2H, m), 3.50-2.95(4H, m), 2.82(3H, br.s), 2.64(2H, t, J=8Hz), 2.12-1.88(2H, m) | Hygroscopic Solid |
| 62 | KBr : 3392, 1637, 1630, 1554, 1259, 1161, 806 | DMSO* : 11.79(1H, s), 10.64-10.49(1H, m), 9.12-8.92(1H, m), 8.45-8.20(3H, m), 7.53-7.38(4H, m), 7.32-7.18(3H, m), 7.16-6.98(1H, m), 4.05-3.91(2H, m), 3.83-3.55(2H, m), 3.47-2.97(4H, m), 2.82(3H, br.s), 2.71-2.56(2H, m), 2.10-1.88(2H, m) | Hygroscopic Solid |
| 63 | KBr : 2952, 1641, 1558, 1338, 1261, 1109, 818 | DMSO* : 12.14(1H, br.s), 10.40-10.20(1H, m), 9.15-9.00(1H, m), 8.40-8.08(3H, m), 8.09(1H, s), 7.62(1H, d, J=9Hz), 7.48(1H, d, J=9Hz), 7.43-7.33(3H, m), 7.27(2H, d, J=8Hz), 4.03-3.93(2H, m), 3.77-3.63(2H, m), 3.45-3.01(4H, m), 2.83(3H, br.s), 2.71-2.52(2H, m), 2.11-1.88(2H, m) | Hygroscopic Solid |
| 64 | Liquid Film : 3257, 2360, 1622, 1556, 1265, 1099, 766 | DMSO : 11.66(1H, s), 10.55-10.40(1H, m), 8.96-8.84(1H, m), 8.40-8.20(3H, m), 7.38(2H, d, J=8Hz), 7.32-7.22(3H, m), 7.11(1H, d, J=8Hz), 7.02(1H, t, J=8Hz), 6.52(1H, d, J=8Hz), 4.03-3.92(2H, m), 3.88(3H, m), 3.76-3.52(2H, m), 3.43-3.00(4H, m), 2.87-2.78(3H, m), 2.69-2.57(2H, m), 2.07-1.93(2H, m) | Hygroscopic Solid |
| 65 | Liquid Film : 3394, 1635, 1556, 1543, 1456, 1259, 1234 | DMSO* : 11.51(1H, s), 10.70-10.50(1H, m), 9.12-8.86(1H, m), 8.46-8.20(3H, m), 7.37(2H, d, J=8Hz), 7.31(1H, d, J=9Hz), 7.24(2H, d, J=8Hz), 7.13(1H, d, J=1Hz), 7.07(1H, d, J=1Hz), 6.83(1H, dd, J=2, 9Hz), 4.00-3.90(2H, m), 3.74(3H, s), 3.73-3.58(2H, m), 3.42-2.98(4H, m), 2.80(3H, br.s), 2.68-2.57(2H, m), 2.09-1.88(2H, m) | Hygroscopic Solid |

Table 4 ( Part 13 )

| Example | IR ($cm^{-1}$) | NMR (ppm) (No Mark : 300MHz, * : 270MHz) | Melting Point (°C) |
|---|---|---|---|
| 66 | KBr : 3400, 2954, 1626, 1549, 1261, 833 | DMSO* : 11.48(1H, s), 10.35-10.20(1H, m), 8.83-8.74(1H, m), 8.36-8.10(3H, m), 7.49(1H, d, J=9Hz), 7.38(2H, d, J=8Hz), 7.26(2H, d, J=8Hz), 7.16-7.10(1H, m), 6.90-6.85(1H, m), 6.70(1H, dd, J=2, 9Hz), 4.05-3.95(2H, m), 3.77(3H, s), 3.74-3.60(2H, m), 3.44-3.00(4H, m), 2.88-2.78(3H, m), 2.69-2.57(2H, m), 2.10-1.88(2H, m) | Hygroscopic Solid |
| 67 | KBr : 2952, 1641, 1554, 1259, 1211, 1159, 812 | DMSO* : 11.96(1H, s), 10.70-10.53(1H, m), 9.18-9.03(1H, m), 8.47-8.23(3H, m), 7.64(1H, s), 7.50(1H, d, J=9Hz), 7.38(2H, d, J=8Hz), 7.29(1H, d, J=1Hz), 7.25(2H, d, J=8Hz), 7.21-7.12(1H, m), 4.02-3.90(2H, m), 3.82-3.59(2H, m), 3.45-2.98(4H, m), 2.80(3H, br.s), 2.70-2.57(2H, m), 2.10-1.91(2H, m) | Hygroscopic Solid |
| 68 | KBr : 3261, 2220, 1643, 1616, 1572, 1342, 810 | DMSO* : 12.25(1H, s), 10.58-10.36(1H, m), 9.25-9.04(1H, m), 8.40-8.15(4H, m), 7.60-7.45(2H, m), 7.40-7.30(1H, m), 7.37(2H, d, J=8Hz), 7.25(2H, d, J=8Hz), 4.05-3.85(2H, m), 3.80-3.58(2H, m), 3.46-2.95(4H, m), 2.82(3H, d, J=4Hz), 2.69-2.55(2H, m), 2.10-1.88(2H, m) | Hygroscopic Solid |
| 69 | KBr : 2954, 1647, 1618, 1554, 1338, 1207, 758 | DMSO : 12.60-12.40(1H, m), 12.05(1H, s), 10.60-10.45(1H, m), 9.18-9.02(1H, m), 8.40-8.20(4H, m), 7.80(1H, d, J=9Hz), 7.48(1H, d, J=9Hz), 7.45-7.32(3H, m), 7.26(2H, d, J=8Hz), 4.03-3.90(2H, m), 3.83-3.60(2H, m), 3.40-3.01(4H, m), 2.89-2.77(3H, m), 2.72-2.58(2H, m), 2.10-1.91(2H, m) | Hygroscopic Solid |
| 70 | KBr : 3435, 2952, 1649, 1614, 1558, 1335, 820 | DMSO : 12.06(1H, s), 10.40-10.22(1H, m), 9.12-9.00(1H, m), 8.38(1H, s), 8.32-8.12(3H, m), 7.80(1H, dd, J=2, 9Hz), 7.48(1H, d, J=9Hz), 7.43-7.31(3H, m), 7.25(2H, d, J=8Hz), 4.02-3.91(2H, m), 3.77-3.60(2H, m), 3.48-2.98(4H, m), 2.82(3H, br.s), 2.60(3H, s), 2.67-2.53(2H, m), 2.08-1.87(2H, m) | Hygroscopic Solid |

Table 4 ( Part 14 )

| Example | IR ($cm^{-1}$) | NMR (ppm) (No Mark : 300MHz, * : 270MHz) | Melting Point (°C) |
|---|---|---|---|
| 71 | KBr : 2952, 1612, 1556, 1290, 1126, 962, 769 | DMSO : 12.28(1H, s), 10.50(1H, br.s), 9.25-9.05(1H, m), 8.28(4H, br.s), 7.71(1H, d, J=9Hz), 7.63(1H, d, J=9Hz), 7.54(1H, s), 7.39(2H, d, J=8Hz), 7.27(2H, d, J=8Hz), 3.96(2H, br.s), 3.83-3.62(2H, m), 3.47-3.02(4H, m), 3.19(3H, s), 2.82(3H, br.s), 2.70-2.57(2H, m), 2.12-1.91(2H, m) | 169.8-182.4 |
| 72 | KBr : 2947, 1626, 1550, 1257, 1234, 1173, 739 | DMSO* : 11.53(1H, s), 10.55-10.40(1H, m), 8.96-8.82(1H, m), 8.40-8.20(3H, m), 7.53-7.07(12H, m), 6.93(1H, dd, J=2, 9Hz), 5.09(2H, s), 4.03-3.93(2H, m), 3.76-3.59(2H, m), 3.43-2.96(4H, m), 2.82(3H, br.s), 2.68-2.58(2H, m), 2.10-1.88(2H, m) | Hygroscopic Solid |
| 73 | KBr : 3400, 2947, 1626, 1610, 1535, 1311, 756 | $CD_3OD$* : 8.30-8.10(1H, m), 7.80-7.52(1H, m), 7.50-7.13(6H, m), 6.62-6.40(1H, m), 4.06(2H, br.s), 3.90-3.60(2H, m), 3.85(3H, s), 3.55-3.10(4H, m), 2.99(3H, br.s), 2.82-2.55(2H, m), 2.20-1.90(2H, m) | Hygroscopic Solid |
| 74 | KBr : 3400, 2949, 1630, 1610, 1535, 1473, 1311 | DMSO* : 11.20(1H, s), 10.27-10.07(1H, m), 8.70-8.53(1H, m), 8.36-8.12(3H, m), 8.02(1H, s), 7.58(1H, d, J=9Hz), 7.38(2H, d, J=8Hz), 7.33-7.20(3H, m), 6.22(1H, s), 4.10-3.88(2H, m), 3.73-3.55(2H, m), 3.47-2.98(4H, m), 2.82(3H, br.s), 2.69-2.57(2H, m), 2.39(3H, s), 2.07-1.88(2H, m) | Hygroscopic Solid |
| 75 | KBr : 3400, 2947, 1630, 1612, 1535, 1477, 1319 | DMSO : 10.98(1H, s), 10.40-10.28(1H, m), 8.73-8.63(1H, m), 8.37-8.18(3H, m), 8.04(1H, s), 7.59(1H, dd, J=2, 9Hz), 7.38(2H, d, J=8Hz), 7.31-7.19(3H, m), 4.05-3.92(2H, m), 3.73-3.58(2H, m), 3.48-3.02(4H, m), 2.83(3H, d, J=5Hz), 2.64(2H, t, J=8Hz), 2.32(3H, s), 2.19(3H, s), 2.07-1.92(2H, m) | Hygroscopic Solid |

Table 4 ( Part 15 )

| Example | IR ($cm^{-1}$) | NMR (ppm) (No Mark : 300MHz, * : 270MHz) | Melting Point (°C) |
|---|---|---|---|
| 76 | KBr : 3435, 1637, 1545, 1462, 1335, 1190, 883 | DMSO : 10.56-10.36(1H, m), 9.05-8.85(1H, m), 8.45-8.15(5H, m), 8.00-7.91(1H, m), 7.85-7.58(5H, m), 7.51(1H, d, J=4Hz), 7.40(2H, d, J=8Hz), 7.28(2H, d, J=8Hz), 6.86(1H, d, J=4Hz), 4.05-3.92(2H, m), 3.80-3.60(2H, m), 3.45-3.00(4H, m), 2.84(3H, d, J=5Hz), 2.70-2.58(2H, m), 2.10-1.91(2H, m) | 137.3-139.8 |
| 77 | neat : 3427, 1657, 1535, 1323, 1296, 1151, 766 | DMSO : 10.13-9.98(1H, m), 9.47-9.35(1H, m), 8.41-8.18(4H, m), 8.18-8.12(1H, m), 7.72-7.56(2H, m), 7.38(2H, d, J=8Hz), 7.27(2H, d, J=8Hz), 4.03-3.93(2H, m), 3.80-3.62(2H, m), 3.53-2.98(4H, m), 2.82(3H, br.s), 2.68-2.56(2H, m), 2.06-1.87(2H, m) | Hygroscopic Solid |
| 78 | Liquid Film: 3369, 2954, 1641, 1539, 1294, 852 | DMSO* : 10.30-10.13(1H, m), 9.56(1H, s), 9.08-8.99(1H, m), 8.78-8.73(1H, m), 8.35-8.10(3H, m), 8.18(1H, d, J=9Hz), 8.12-8.03(1H, m), 7.38(2H, d, J=8Hz), 7.27(2H, d, J=8Hz), 4.04-3.91(2H, m), 3.77-3.62(2H, m), 3.50-3.00(4H, m), 2.84(3H, d, J=5Hz), 2.70-2.56(2H, m), 2.07-1.90(2H, m) | Hygroscopic Solid |
| 79 | KBr : 3400, 1639, 1603, 1487, 1261, 1038, 760 | DMSO* : 10.35-10.10(1H, m), 8.80-8.60(1H, m), 8.50-8.00(3H, m), 7.51(1H, dd, J=2, 8Hz), 7.45(1H, d, J=2Hz), 7.40(2H, d, J=8Hz), 7.27(2H, d, J=8Hz), 7.01(1H, d, J=8Hz), 6.11(2H, s), 4.06-3.90(2H, m), 3.70-3.53(2H, m), 3.40-2.95(4H, m), 2.80(3H, br.s), 2.69-2.56(2H, m), 2.06-1.85(2H, m) | Hygroscopic Solid |
| 80 | KBr : 3400, 2952, 1657, 1535, 1502, 779 | DMSO : 10.53-10.30(1H, m), 9.40-9.23(1H, m), 8.60(1H, d, J=9Hz), 8.50-8.25(3H, m), 8.21-8.07(3H, m), 7.95-7.84(1H, m), 7.80-7.70(1H, m), 7.39(2H, d, J=8Hz), 7.25(2H, d, J=8Hz), 4.03-3.90(2H, m), 3.83-3.70(2H, m), 3.65-3.00(4H, m), 2.83(3H, d, J=5Hz), 2.69-2.55(2H, m), 2.08-1.90(2H, m) | Hygroscopic Solid |

Table 4 ( Part 16 )

| Example | IR ($cm^{-1}$) | NMR (ppm) (No Mark : 300MHz, * : 270MHz) | Melting Point (°C) |
|---|---|---|---|
| 81 | KBr : 3400, 2952, 1657, 1545, 1323, 1209, 808 | DMSO : 10.75-10.56(1H, m), 9.33-9.23(1H, m), 9.15-9.06(1H, m), 8.78-8.70(1H, m), 8.73-8.63(1H, m), 8.48-8.23(4H, m), 8.25-8.15(1H, m), 7.83-7.73(1H, m), 7.40(2H, d, J=8Hz), 7.27(2H, d, J=8Hz), 4.05-3.90(2H, m), 3.83-3.67(2H, m), 3.50-3.00(4H, m), 2.84(3H, d, J=5Hz), 2.71-2.58(2H, m), 2.10-1.94(2H, m) | 143.2-151.8 |
| 82 | KBr : 3369, 1664, 1568, 1535, 1427, 837 | DMSO : 12.01(1H, s), 10.53-10.36(1H, m), 9.00-8.91(1H, m), 8.40-8.17(4H, m), 8.09-8.02(1H, m), 7.95(1H, d, J=9Hz), 7.39(2H, d, J=8Hz), 7.34(1H, d, J=9Hz), 7.26(2H, J=8Hz), 6.60-6.53(1H, m), 4.04-3.93(2H, m), 3.78-3.58(2H, m), 3.50-3.00(4H, m), 2.82(3H, d, J=5Hz), 2.69-2.58(2H, m), 2.09-1.90(2H, m) | Hygroscopic Solid |
| 83 | Liquid Film : 3263, 1651, 1547, 1433, 1302, 1016, 760 | DMSO : 8.97-8.82(1H, m), 8.40-8.10(3H, m), 8.10-7.87(3H, m), 7.52-7.40(2H, m), 7.40-7.32(2H, m), 7.32-7.19(2H, m), 4.04-3.91(2H, m), 3.90-2.20(8H, m), 1.94-1.70(2H, m) | Hygroscopic Solid |
| 84 | KBr : 3400, 2927, 1626, 1543, 1425, 1298, 762 | DMSO : 9.05-8.85(1H, m), 8.30-8.10(3H, m), 8.08-7.98(2H, m), 7.97-7.78(1H, m), 7.52-7.40(2H, m), 7.40-7.31(2H, m), 7.31-7.22(2H, m), 4.00-3.92(2H, m), 3.57-3.22(6H, m), 2.65-2.46(2H, m), 2.04-1.93(3H, m), 1.90-1.70(2H, m) | Hygroscopic Solid |
| 85 | KBr : 3435, 1678, 1637, 1543, 1203, 1138, 723 | DMSO* : 9.10-8.96(1H, m), 8.35-8.00(3H, m), 8.07(1H, s), 8.10-7.90(2H, m), 7.55-7.40(2H, m), 7.34(2H, d, J=8Hz), 7.25(2H, d, J=8Hz), 4.20-3.95(2H, m), 4.07-3.90(2H, m), 3.85-3.05(6H, m), 2.62(2H, t, J=7Hz), 2.05-1.85(2H, m) | Hygroscopic Solid |

Table 4 ( Part 17 )

| Example | IR ($cm^{-1}$) | NMR (ppm) (No Mark : 300MHz, * : 270MHz) | Melting Point (°C) |
|---|---|---|---|
| 86 | KBr : 3269, 1639, 1552, 1419, 1321, 1061, 806 | DMSO : 11.88(1H, s), 9.80-9.60(1H, m), 8.95-8.80(1H, m), 8.40-8.20(1H, m), 7.76-7.72(1H, m), 7.47-7.41(1H, m), 7.23-7.10(6H, m), 4.23-4.15(2H, m), 3.73-3.57(2H, m), 3.45-3.00(4H, m), 2.84(3H, d, J=4Hz), 2.66-2.50(2H, m), 2.03-1.88(2H, m), 1.86(3H, s) | 105.9-112.0 |
| 87 | KBr : 3294, 1604, 1529, 1408, 1178, 800, 766 | DMSO : 11.81(1H, s), 10.55-10.20(1H, m), 8.45-8.17(3H, m), 7.73-7.64(1H, m), 7.46(1H, d, J=9Hz), 7.41(2H, d, J=8Hz), 7.29(2H, d, J=8Hz), 7.21(1H,dd,J=2,9Hz), 7.00-6.94(1H, m), 4.07-3.80(4H, m), 3.50-3.00(7H, m), 2.83(3H, d, J=4Hz), 2.70-2.57(2H, m) 2.12-1.90(2H, m) | 190.2-191.4 |
| 88 | Liquid Film : 3390, 1633, 1564, 1547, 1302, 1014, 762 | DMSO : 10.40-10.25(1H, m), 9.31-9.18(1H, m), 8.56-8.48(1H, m), 8.40-8.25(3H, m), 8.20(1H, s), 8.08-7.99(1H, m), 7.97-7.92(1H, m), 7.81-7.72(1H, m), 7.53-7.37(3H, m), 4.22-4.07(2H, m), 3.74-3.62(2H, m), 3.61-3.04(4H, m), 2.84(3H, d, J=5Hz), 2.76-2.63(2H, m), 2.12-1.94(2H, m) | Hygroscopic Solid |
| 89 | KBr : 3369, 2625, 1626, 1543, 1471, 1311, 762 | DMSO* : 11.41(1H, s), 10.73-10.58(1H, m), 8.83-8.68(1H, m), 8.57-8.37(4H, m), 8.20(1H, s), 7.84-7.65(2H, m), 7.53-7.38(3H, m), 6.56-6.50(1H, m), 4.22-4.09(2H, m), 3.74-3.63(2H, m), 3.43-3.03(4H, m), 2.83(3H, d, J=5Hz), 2.76-2.64(2H, m), 2.13-1.97(2H, m) | Hygroscopic Solid |
| 90 | KBr : 3400, 2954, 1630, 1562, 1543, 1300, 762 | $CD_3OD$* : 8.02(1H, s), 7.97-7.83(2H, m), 7.53-7.37(2H, m), 7.03(1H, d, J=4Hz), 6.87(1H, d, J=4Hz), 4.23(2H, s), 3.78(2H, t, J=6Hz), 3.48-3.19(4H, m), 3.03-2.86(2H, m), 2.97(3H, s), 2.23-2.03(2H, m) | Hygroscopic Solid |
| 91 | KBr : 3369, 2947, 2704, 1479, 1460, 835, 746, 727 | $CD_3OD$ : 7.94-7.82(2H, m), 7.67(1H, s), 7.47-7.32(6H, m), 4.64(2H, s), 4.08(2H, s), 3.70-3.55(4H, m), 3.38-3.23(2H, m), 2.95(3H, s), 2.76(2H, t, J=8Hz), 2.23-2.05(2H, m) | Hygroscopic Solid |

Table 5 ( Part 1 )

| Example Intermediate | NMR (ppm) (No Mark : 300MHz, * : 270MHz) |
|---|---|
| 1-1 | $CDCl_3$ : 7.21(2H, d, J=8Hz), 7.16(2H, d, J=8Hz), 4.80(1H, br.s), 4.28(2H, d, J=6Hz), 3.72-3.63(2H, m), 2.70(2H, t, J=8Hz), 1.95-1.80(2H, m), 1.46(9H, s) |
| 1-2 | $CDCl_3$ : 9.82(1H, t, J=1Hz), 7.21(2H, d, J=8Hz), 7.16(2H, d, J=8Hz), 4.81(1H, br.s), 4.28(2H, d, J=6Hz), 2.95(2H, t, J=8Hz), 2.77(2H, t, J=8Hz), 1.46(9H, s) |
| 1-3 | $CDCl_3$ : 8.44(1H, s), 8.03-7.88(3H, m), 7.83(1H, dd, J=2, 9Hz), 7.70-7.55(2H, m), 7.17(2H, d, J=8Hz), 7.07(2H, d, J=8Hz), 4.85(1H, br.s), 4.27(2H, d, J=6Hz), 3.01(2H, t, J=6Hz), 2.65(2H, t, J=6Hz), 2.53(2H, t, J=7Hz), 2.44(2H, t, J=7Hz), 1.75-1.60(2H, m), 1.46(9H, s) |
| 3-1 | $CDCl_3$ : 7.21(2H, d, J=8Hz), 7.14(2H, d, J=8Hz), 4.83(1H, br.s), 4.28(2H, d, J=6Hz), 3.41(2H, t, J=5Hz), 2.82(2H, t, J=6Hz), 2.70-2.58(4H, m), 1.87-1.74(2H, m), 1.46(9H, s) |
| 3-2 | $CDCl_3$ : 8.12(1H, br.s), 7.21(2H, d, J=8Hz), 7.13(2H, d, J=8Hz), 4.81(1H, br.s), 4.28(2H, d, J=6Hz), 3.45(4H, s), 3.20(2H, t, J=7Hz), 2.58(2H, t, J=8Hz), 1.92-1.77(2H, m), 1.46(9H, s), 1.45(9H, s) |
| 3-3 | $CDCl_3$* : 7.20(2H, d, J=8Hz), 7.13(2H, d, J=8Hz), 4.79(1H, br.s), 4.28(2H, d, J=5Hz), 3.33-3.10(4H, m), 2.80(2H, d, J=7Hz), 2.58(2H, d, J=8Hz), 1.93-1.75(2H, m), 1.46(9H, s), 1.44(9H, s) |
| 3-4 | $CDCl_3$ : 7.88-7.78(2H, m), 7.60-7.40(3H, m), 7.20(2H, d, J=8Hz), 7.09(2H, d, J=8Hz), 5.60(1H, br.s), 4.83(1H, br.s), 4.28(2H, d, J=6Hz), 3.35-3.20(2H, m), 3.15-2.95(4H, m), 2.51(2H, t, J=8Hz), 1.80-1.64(2H, m), 1.46(9H, s), 1.43(9H, s) |

Table 5 ( Part 2 )

| Example Intermediate | NMR (ppm) (No Mark : 300MHz, * : 270MHz) |
|---|---|
| 4-1 | $CDCl_3$ : 8.50-8.40(1H, m), 8.11(1H, d, J=9Hz), 7.98-7.86(1H, m), 7.88(1H, d, J=9Hz), 7.76-7.65(2H, m), 7.15(2H, d, J=8Hz), 7.03(2H, d, J=8Hz), 6.04(1H, br.s), 4.80(1H, br.s), 4.27(2H, d, J=6Hz), 3.40-3.23(2H, m), 3.20-2.95(2H, m), 3.12(2H, t, J=8Hz), 2.46(2H, t, J=8Hz), 1.78-1.64(2H, m), 1.46(9H, s), 1.42(9H, s) |
| 5-1 | $CDCl_3$* : 8.34(1H, d, J=2Hz), 7.97(1H, d, J=8Hz), 7.76(1H, dd, J=2, 8Hz), 7.61(1H, d, J=6Hz), 7.43(1H, d, J=6Hz), 7.17(2H, d, J=8Hz), 7.05(2H, d, J=8Hz), 5.63(1H, br.s), 4.81(1H, br.s), 4.27(2H, d, J=6Hz), 3.28(2H, t, J=6Hz), 3.18-2.98(4H, m), 2.46(2H, t, J=8Hz), 1.80-1.60(2H, m), 1.46(9H, s), 1.41(9H, s) |
| 6-1 | $CDCl_3$* : 8.36(1H, d, J=2Hz), 8.01(1H, d, J=9Hz), 7.87(1H, dd, J=2, 9Hz), 7.18(2H, d, J=8Hz), 7.05(2H, d, J=8Hz), 5.75(1H, br.s), 4.87(1H, br.s), 4.28(2H, d, J=6Hz), 3.29(2H, d, J=6Hz), 3.16-3.00(4H, m), 2.89(3H, s), 2.48(2H, t, J=8Hz), 1.79-1.60(2H, m), 1.46(9H, s), 1.42(9H, s) |
| 7-1 | $CDCl_3$ : 7.69-7.64(1H, m), 7.63-7.58(1H, m), 7.30(1H, d, J=8Hz), 7.19(2H, d, J=8Hz), 7.09(2H, d, J=8Hz), 5.41(1H, br.s), 4.82(1H, br.s), 4.28(2H, d, J=6Hz), 3.35-3.20(2H, m), 3.15-3.00(4H, m), 2.93(4H, t, J=8Hz), 2.51(2H, t, J=8Hz), 2.11(2H, quint, J=8Hz), 1.80-1.65(2H, m), 1.46(9H, s), 1.42(9H, s) |
| 8-1 | $CDCl_3$* : 7.24-7.08(8H, m), 5.13(1H, br.s), 4.80(1H, br.s), 4.27(2H, d, J=6Hz), 4.05-3.90(1H, m), 3.50-3.05(10H, m), 2.57(2H, t, J=8Hz), 1.90-1.73(2H, m), 1.46(9H, s), 1.44(9H, s) |

Table 5 ( Part 3 )

| Example Intermediate | NMR (ppm) (No Mark : 300MHz, * : 270MHz) |
|---|---|
| 9-1 | $CDCl_3$* : 7.75-7.58(2H, m), 7.35-7.22(1H, m), 7.19(2H, d, J=8Hz), 7.10(2H, d, J=8Hz), 5.69(1H, br.s), 4.90-4.70(3H, m), 4.27(2H, d, J=6Hz), 3.95-3.78(2H, m), 3.30(2H, t, J=6Hz), 3.20-2.90(6H, m), 2.52(2H, t, J=8Hz), 1.85-1.60(2H, m), 1.46(9H, s), 1.43(9H, s) |
| 9-2 | $CDCl_3$ : 7.60-7.54(1H, m), 7.50-7.45(1H, m), 7.24-7.16(1H, m), 7.20(2H, d, J=8Hz), 7.10(2H, d, J=8Hz), 5.30(1H, br.s), 5.01(1H, br.s), 4.28(2H, d, J=6Hz), 3.98(2H, s), 3.30(2H, t, J=6Hz), 3.20-2.98(6H, m), 2.82(2H, t, J=6Hz), 2.52(2H, t, J=8Hz), 1.81-1.68(2H, m), 1.46(9H, s), 1.43(9H, s) |
| 10-1 | $CDCl_3$* : 7.88-7.70(2H, m), 7.60-7.35(4H, m), 7.19(2H, d, J=8Hz), 7.12(2H, d, J=8Hz), 4.82(1H, br.s), 4.27(2H, d, J=6Hz), 3.67-3.38(4H, m), 3.35-3.15(2H, m), 2.58(2H, t, J=8Hz), 1.95-1.75(2H, m), 1.46(9H, s), 1.42(9H, s) |
| 11-1 | $CDCl_3$ : 8.35(1H, s), 7.94-7.82(4H, m), 7.77(1H, br.s), 7.58-7.47(2H, m), 7.23-7.07(4H, m), 4.84(1H, br.s), 4.26(2H, d, J=6Hz), 3.67-3.47(4H, m), 3.37-3.15(2H, m), 2.59(2H, t, J=8Hz), 1.95-1.77(2H, m), 1.46(9H, s), 1.44(9H, s) |
| 12-1 | $CDCl_3$* : 8.36(1H, br.s), 7.95-7.79(2H, m), 7.65-7.45(3H, m), 7.41(1H, t, J=8Hz), 7.20(2H, d, J=8Hz), 7.14(2H, d, J=8Hz), 6.96(1H, br.s), 4.81(1H, br.s), 4.27(2H, d, J=6Hz), 3.75-3.60(2H, m), 3.60-3.42(2H, m), 3.40-3.16(2H, m), 2.61(2H, t, J=8Hz), 2.00-1.80(2H, m), 1.46(9H, s), 1.35(9H, s) |

Table 5 ( Part 4 )

| Example Intermediate | NMR (ppm) (No Mark : 300MHz, * : 270MHz) |
|---|---|
| 13-1 | $CDCl_3$ : 9.03-8.93(1H, m), 8.36(1H, s), 8.22(1H, dd, J=2, 8Hz), 8.12(2H, s), 7.88(1H, br.s), 7.45(1H, dd, J=4, 8Hz), 7.20(2H, d, J=8Hz), 7.13(2H, d, J=8Hz), 4.84(1H, br.s), 4.27(2H, d, J=6Hz), 3.72-3.40(4H, m), 3.25(2H, t, J=7Hz), 2.60(2H, t, J=8Hz), 1.98-1.80(2H, m), 1.46(9H, s), 1.44(9H, s) |
| 14-1 | $CDCl_3$ : 7.79(2H, d, J=8Hz), 7.50(1H, br.s), 7.42-7.33(2H, m), 7.24-7.08(5H, m), 7.08-7.01(2H, m), 6.98(2H, d, J=9Hz), 4.84(1H, br.s), 4.27(2H, d, J=6Hz), 3.62-3.41(4H, m), 3.22(2H, t, J=7Hz), 2.58(2H, t, J=8Hz), 1.94-1.78(2H, m), 1.46(9H, s), 1.42(9H, s) |
| 15-1 | $CDCl_3$: 7.46(1H, br.s), 7.31(2H, t, J=8Hz), 7.19(2H, d, J=8Hz), 7.12(2H, d, J=8Hz), 7.01(1H, t, J=8Hz), 6.92(2H, d, J=8Hz), 4.83(1H, br.s), 4.46(2H, br.s), 4.27(2H, d, J=6Hz), 3.50-3.30(2H, m), 3.46(2H, t, J=5Hz), 3.30-3.10(2H, m), 2.57(2H, t, J=8Hz), 1.90-1.75(2H, m), 1.46(9H, s), 1.42(9H, s) |
| 16-1 | $CD_3OD$ : 7.17(2H, d, J=9Hz), 7.15(2H, d, J=9Hz), 4.16(2H, s), 2.87(2H, t, J=8Hz), 2.47-2.35(2H, m), 1.44(9H, s) |
| 16-2 | $CDCl_3$ : 8.35(1H, s), 7.99(2H, d, J=8Hz), 7.96-7.89(1H, m), 7.78-7.59(3H, m), 7.21(2H, d, J=8Hz), 7.20(2H, d, J=8Hz), 5.93(1H, br.s), 4.87(1H, br.s), 4.28(2H, d, J=6Hz), 3.41(2H, q, J=6Hz), 3.50-3.30(2H, m), 2.98(2H, t, J=8Hz), 2.80(3H, s), 2.52(2H, t, J=8Hz), 1.46(9H, s) |
| 17-1 | $CDCl_3$ : 8.47-8.42(1H, m), 8.00-7.87(3H, m), 7.82(1H, dd, J=2, 9Hz), 7.70-7.57(2H, m), 7.18(2H, d, J=8Hz), 7.07(2H, d, J=8Hz), 4.83(1H, br.s), 4.28(2H, d, J=6Hz), 3.01(2H, t, J=6Hz), 2.48(2H, t, J=8Hz), 2.39(2H, t, J=6Hz), 2.25(2H, t, J=8Hz), 2.03(3H, s), 1.75-1.60(2H, m), 1.46(9H, s) |

Table 5 ( Part 5 )

| Example Intermediate | NMR (ppm) (No Mark : 300MHz, * : 270MHz) |
|---|---|
| 18-1 | $CDCl_3$ : 8.44(1H, s), 7.99-7.87(3H, m), 7.86-7.78(1H, m), 7.69-7.57(2H, m), 7.18-7.08(2H, m), 7.03(2H, d, J=8Hz), 4.82(1H, br.s), 4.27(2H, d, J=6Hz), 3.01-2.95(2H, m), 2.53-2.22(4H, m), 2.35(2H, q, J=7Hz), 2.27(2H, t, J=7), 1.70-1.53(2H, m), 1.47(9H, s), 0.86(3H, t, J=7Hz) |
| 19-1 | DMSO : 8.41(1H, s), 8.13-8.06(2H, m), 8.03-7.98(1H, m), 7.81(1H, dd, J=2, 9Hz), 7.72-7.62(2H, m), 7.48(1H, br.s), 7.13(2H, d, J=8Hz), 7.07(2H, d, J=8Hz), 6.81(1H, br.s), 4.10(2H, d, J=6Hz), 3.30(2H, t, J=7Hz), 3.19(2H, t, J=7Hz), 2.99(2H, t, J=7Hz), 2.96(3H, s), 2.46(2H, t, J=8Hz), 1.77-1.64(2H, m), 1.39(9H, s) |
| 20-1 | $CDCl_3$* : 7.76(2H, d, J=7Hz), 7.59(2H, d, J=7Hz), 7.39(2H, t, J=7Hz), 7.28(2H, t, J=7Hz), 7.19(2H, d, J=8Hz), 7.14(2H, d, J=8Hz), 5.28(1H, br.s), 4.80(1H, br.s), 4.38(2H, d, J=7Hz), 4.27(2H, d, J=6Hz), 4.21(1H, t, J=7Hz), 3.35-3.15(2H, m), 2.62(2H, t, J=7Hz), 2.47(2H, t, J=6Hz), 2.38(2H, t, J=7Hz), 2.22(3H, s), 1.85-1.70(2H, m), 1.45(9H, s) |
| 20-2 | $CDCl_3$ : 7.20(2H, d, J=8Hz), 7.15(2H, d, J=8Hz), 4.81(1H, br.s), 4.28(2H, d, J=6Hz), 2.75(2H, t, J=6Hz), 2.62(2H, t, J=8Hz), 2.39(2H, t, J=6Hz), 2.37(2H, t, J=7Hz), 2.21(3H, s), 1.84-1.71(2H, m), 1.46(9H, s) |
| 20-3 | $CDCl_3$ : 8.50-8.42(1H, m), 8.15(1H, d, J=9Hz), 7.94(1H, dd, J=2, 5Hz), 7.90(1H, d, J=9Hz), 7.75-7.65(2H, m), 7.18(2H, d, J=8Hz), 7.10(2H, d, J=8Hz), 6.00(1H, br.s), 4.81(1H, br.s), 4.28(2H, d, J=6Hz), 2.95(2H, t, J=6Hz), 2.53(2H, t, J=8Hz), 2.38(2H, t, J=6Hz), 2.26(2H, t, J=8Hz), 2.00(3H, s), 1.78-1.63(2H, m), 1.47(9H, s) |

Table 5 ( Part 6 )

| Example Intermediate | NMR (ppm) (No Mark : 300MHz, * : 270MHz) |
|---|---|
| 21-1 | $CDCl_3$* : 8.10(1H, d, J=8Hz), 7.55-7.35(3H, m), 7.21(2H, d, J=8Hz), 7.11(2H, d, J=8Hz), 4.84(1H, br.s), 4.29(2H, d, J=6Hz), 2.93(2H, t, J=6Hz), 2.58(2H, t, J=8Hz), 2.40(2H, t, J=6Hz), 2.31(2H, t, J=8Hz), 2.05(3H, s), 1.83-1.65(2H, m), 1.46(9H, s) |
| 22-1 | $CDCl_3$* : 8.14(1H, dd, J=2, 8Hz), 7.72(1H, dd, J=1, 8Hz), 7.47(1H, dt, J=1, 8Hz), 7.40(1H, dt, J=2, 8Hz), 7.20(2H, d, J=8Hz), 7.13(2H, d, J=8Hz), 4.84(1H, br.s), 4.29(2H, d, J=6Hz), 2.92(2H, t, J=6Hz), 2.58(2H, t, J=8Hz), 2.40(2H, t, J=6Hz), 2.32(2H, t, J=8Hz), 2.06(3H, s), 1.82-1.66(2H, m), 1.47(9H, s) |
| 23-1 | $CDCl_3$* : 7.99(1H, d, J=9Hz), 7.55(1H, d, J=9Hz), 7.21(2H, d, J=8Hz), 7.13(2H, d, J=8Hz), 4.83(1H, br.s), 4.29(2H, d, J=6Hz), 2.95(2H, t, J=6Hz), 2.58(2H, d, J=8Hz), 2.41(2H, d, J=6Hz), 2.32(2H, d, J=8Hz), 2.07(3H, s), 1.83-1.66(2H, m), 1.47(9H, s) |
| 24-1 | $CDCl_3$ : 7.70-7.57(2H, m), 7.43-7.32(2H, m), 6.52(1H, d, J=16Hz), 4.29(2H, q, J=7Hz), 1.35(3H, t, J=7Hz) |
| 24-2 | $CDCl_3$* : 7.27-7.17(1H, m), 6.98-6.84(2H, m), 4.95-4.80(1H, m), 4.32(2H, d, J=6Hz), 3.67(2H, t, J=6Hz), 2.69(2H, t, J=8Hz), 1.93-1.81(2H, m), 1.44(9H, s) |
| 24-3 | $CDCl_3$* : 9.81(1H, t, J=1Hz), 7.29-7.18(1H, m), 6.97-6.84(2H, m), 4.95-4.78(1H, m), 4.32(2H, d, J=6Hz), 2.93(2H, t, J=7Hz), 2.83-2.73(2H, m), 1.43(9H, s) |
| 24-4 | $CDCl_3$* : 7.76(2H, d, J=7Hz), 7.59(2H, d, J=7Hz), 7.39(2H, t, J=7Hz), 7.34-7.14(3H, m), 6.96-6.83(2H, m), 5.24(1H, br.s), 4.85(1H, br.s), 4.39(2H, d, J=7Hz), 4.31(2H, d, J=6Hz), 4.21(1H, t, J=7Hz), 3.34-3.18(2H, m), 2.61(2H, t, J=8Hz), 2.53-2.38(2H, m), 2.38(2H, t, J=7Hz), 2.22(3H, s), 1.84-1.68(2H, m), 1.44(9H, s) |

Table 5 ( Part 7 )

| Example Intermediate | NMR (ppm) (No Mark : 300MHz, * : 270MHz) |
|---|---|
| 24-5 | $CDCl_3$* : 7.27-7.17(1H, m), 6.96-6.83(2H, m), 4.87(1H, br.s), 4.32(2H, d, J=6Hz), 2.75(2H, t, J=6Hz), 2.61(2H, t, J=8Hz), 2.39(2H, t, J=6Hz), 2.36(2H, t, J=8Hz), 2.22(3H, s), 1.85-1.69(2H, m), 1.44(9H, s) |
| 24-6 | $CDCl_3$* : 8.47-8.41(1H, m), 8.02-7.86(3H, m), 7.82(1H, dd, J=2, 9Hz), 7.71-7.56(2H, m), 7.21(1H, t, J=8Hz), 6.88-6.73(2H, m), 5.26(1H, br.s), 4.88(1H, br.s), 4.32(2H, d, J=6Hz), 3.00(2H, t, J=6Hz), 2.45(2H, t, J=8Hz), 2.36(2H, t, J=6Hz), 2.21(2H, t, J=8Hz), 2.00(3H, s), 1.73-1.48(2H, m), 1.45(9H, s) |
| 25-1 | $CDCl_3$* : 7.70-7.62(1H, m), 7.43-7.27(3H, m), 7.16(2H, d, J=8Hz), 7.10(2H, d, J=8Hz), 6.87(1H, br.s), 4.81(1H, br.s), 4.27(2H, d, J=6Hz), 3.60-3.45(2H, m), 2.60(2H, d, J=7Hz), 2.57(2H, t, J=6Hz), 2.41(2H, t, J=7Hz), 2.24(3H, s), 1.85-1.66(2H, m), 1.46(9H, s) |
| 26-1 | $CDCl_3$* : 7.58(1H, d, J=7Hz), 7.53(1H, dd, J=2, 7Hz), 7.35(1H, t, J=7Hz), 7.26(1H, dt, J=2, 7Hz), 7.16(2H, d, J=8Hz), 7.09(2H, d, J=8Hz), 6.64(1H, br.s), 4.81(1H, br.s), 4.27(2H, d, J=6Hz), 3.60-3.43(2H, m), 2.65-2.53(4H, m), 2.41(2H, t, J=7Hz), 2.24(3H, s), 1.85-1.67(2H, m), 1.46(9H, s) |
| 27-1 | $CDCl_3$ : 7.87-7.70(4H, m), 7.53-7.39(3H, m), 7.16(2H, d, J=8Hz), 7.09(2H, d, J=8Hz), 4.78(1H, br.s), 4.26(2H, d, J=6Hz), 3.96(2H, s), 3.45-3.07(4H, m), 2.81(2H, t, J=7Hz), 2.54(2H, t, J=8Hz), 1.88-1.73(2H, m), 1.46(9H, s), 1.42(9H, s) |
| 28-1 | DMSO : 8.50-8.30(1H, m), 8.16-8.10(1H, m), 8.10-7.83(4H, m), 8.05(1H, s), 7.61-7.49(2H, m), 3.20-3.05(2H, m), 2.97-2.82(2H, m) |

Table 5 ( Part 8 )

| Example Intermediate | NMR (ppm) (No Mark : 300MHz, * : 270MHz) |
|---|---|
| 28-2 | $CDCl_3$ : 7.92-7.83(2H, m), 7.84(1H, s), 7.54-7.42(2H, m), 7.21(2H, d, J=8Hz), 7.14(2H, d, J=8Hz), 5.90-5.65(1H, m), 5.45-5.25(1H, m), 5.15-4.90(1H, m), 4.31(2H, d, J=6Hz), 3.33-3.17(2H, m), 3.15-2.99(2H, m), 2.92(2H, t, J=7Hz), 2.43(2H, t, J=7Hz), 1.45(9H, s) |
| 28-3 | $CDCl_3$ : 7.91-7.82(2H, m), 7.86(1H, s), 7.55-7.42(2H, m), 7.17(2H, d, J=8Hz), 7.08(2H, d, J=8Hz), 4.90-4.75(1H, m), 4.27(2H, d, J=6Hz), 3.15-3.06(2H, m), 2.77-2.68(2H, m), 2.63-2.40(4H, m), 1.77-1.63(2H, m), 1.46(9H, s) |
| 29-1 | $CDCl_3$ : 7.72-7.66(1H, m), 7.53-7.42(2H, m), 7.41-7.32(2H, m), 7.19(2H, d, J=8Hz), 7.09(2H, d, J=8Hz), 4.90-4.74(1H, m), 4.28(2H, d, J=6Hz), 3.13(2H, t, J=6Hz), 2.56-2.47(2H, m), 2.45-2.38(2H, m), 2.33-2.25(2H, m), 2.07(3H, s), 1.76-1.63(2H, m), 1.46(9H, s) |
| 30-1 | $CDCl_3$* : 7.91-7.80(2H, m), 7.86(1H, s), 7.53-7.41(2H, m), 7.18(2H, d, J=8Hz), 7.07(2H, d, J=8Hz), 4.90-4.70(1H, m), 4.28(2H, d, J=6Hz), 3.10(2H, t, J=6Hz), 2.55-2.37(4H, m), 2.32-2.22(2H, m), 2.06(3H, s), 1.75-1.60(2H, m), 1.47(9H, s) |
| 31-1 | $CDCl_3$* : 7.87-7.76(2H, m), 7.54-7.42(2H, m), 7.18(2H, d, J=8Hz), 7.08(2H, d, J=8Hz), 4.90-4.70(1H, m), 4.28(2H, d, J=6Hz), 3.15-3.05(2H, m), 2.69(3H, s), 2.55-2.46(2H, m), 2.45-2.37(2H, m), 2.33-2.22(2H, m), 2.05(3H, s), 1.77-1.60(2H, m), 1.47(9H, s) |
| 32-1 | $CDCl_3$ : 7.97-7.80(2H, m), 7.62-7.50(2H, m), 7.19(2H, d, J=8Hz), 7.10(2H, d, J=8Hz), 4.91-4.72(1H, m), 4.28(2H, d, J=6Hz), 3.10(2H, t, J=6Hz), 2.58-2.50(2H, m), 2.47-2.40(2H, m), 2.35-2.27(2H, m), 2.07(3H, s), 1.80-1.65(2H, m), 1.47(9H, s) |

Table 5 ( Part 9 )

| Example Intermediate | NMR (ppm) (No Mark : 300MHz, * : 270MHz) |
|---|---|
| 33-1 | $CDCl_3$ : 8.47(1H, s), 8.00-7.84(4H, m), 7.70-7.55(2H, m), 7.14(2H, d, J=8Hz), 7.02(2H, d, J=8Hz), 5.75-5.55(1H, m), 4.90-4.70(1H, m), 4.27(2H, d, J=6Hz), 3.06-2.93(2H, m), 2.62-2.50(2H, m), 2.52-2.35(4H, m), 2.33-2.16(4H, m), 1.70-1.52(2H, m), 1.47(18H, s) |
| 34-1 | $CDCl_3$ : 7.89(1H, s), 7.89-7.80(2H, m), 7.53-7.40(2H, m), 7.14(2H, d, J=8Hz), 7.03(2H, d, J=8Hz), 5.95-5.60(1H, m), 4.90-4.70(1H, m), 4.27(2H, d, J=6Hz), 3.15-3.10(2H, m), 2.60(2H, t, J=6Hz), 2.53(2H, t, J=6Hz), 2.44(2H, t, J=8Hz), 2.32(2H, t, J=8Hz), 2.27(2H, t, J=6Hz), 1.70-1.55(2H, m), 1.47(9H, s), 1.45(9H, s) |
| 35-1 | $CDCl_3$ : 7.91-7.83(3H, m), 7.53-7.42(2H, m), 7.26-7.16(1H, m), 6.87-6.74(2H, m), 4.95-4.80(1H, m), 4.32(2H, d, J=6Hz), 3.11(2H, t, J=6Hz), 2.54-2.39(4H, m), 2.26(2H, t, J=7Hz), 2.06(3H, s), 1.73-1.59(2H, m), 1.45(9H, s) |
| 36-1 | $CD_3OD$ : 8.62-8.57(1H, m), 7.87-7.79(1H, m), 7.43(1H, d, J=8Hz), 4.67(2H, s), 4.21(2H, s) |
| 36-2 | $CDCl_3$ : 10.10(1H, s), 8.99(1H, d, J=2Hz), 8.15(1H, dd, J=2, 8Hz), 7.46(1H, d, J=8Hz), 5.60-5.50(1H, m), 4.54(2H, d, J=5Hz), 1.47(9H, s) |
| 36-3 | $CDCl_3$ : 8.65(1H, d, J=2Hz), 7.82(1H, dd, J=2, 8Hz), 7.66(1H, d, J=16Hz), 7.31(1H, d, J=8Hz), 6.49(1H, d, J=16Hz), 5.56-5.46(1H, m), 4.47(2H, d, J=5Hz), 4.28(2H, q, J=7Hz), 1.47(9H, s), 1.35(3H, t, J=7Hz) |
| 36-4 | $CDCl_3$* : 8.38(1H, d, J=2Hz), 7.51(1H, dd, J=2, 8Hz), 7.20(1H, d, J=8Hz), 5.57-5.42(1H, m), 4.41(2H, d, J=6Hz), 3.67(3H, s), 2.94(2H, t, J=8Hz), 2.63(2H, t, J=8Hz), 1.53(9H, s) |
| 36-5 | $CDCl_3$ : 9.82(1H, t, J=1Hz), 8.40(1H, d, J=2Hz), 7.50(1H, dd, J=2, 8Hz), 7.21(1H, d, J=8Hz), 5.56-5.43(1H, m), 4.41(2H, d, J=5Hz), 3.02-2.90(2H, m), 2.86-2.74(2H, m), 1.46(9H, s) |

Table 5 ( Part 10 )

| Example Intermediate | NMR (ppm) (No Mark : 300MHz, * : 270MHz) |
|---|---|
| 36-6 | $CDCl_3$* : 8.36(1H, d, J=2Hz), 7.76(2H, d, J=8Hz), 7.59(2H, d, J=8Hz), 7.47(1H, dd, J=2, 8Hz), 7.39(2H, t, J=7Hz), 7.28(2H, t, J=7Hz), 7.17(1H, d, J=8Hz), 5.58-5.42(1H, m), 5.32-5.16(1H, m), 4.48-4.29(4H, m), 4.26-4.15(1H, m), 3.37-3.13(2H, m), 2.62(2H, t, J=8Hz), 2.53-2.28(4H, m), 2.22(3H, s), 1.85-1.71(2H, m), 1.46(9H, s) |
| 36-7 | $CDCl_3$ : 8.36(1H, d, J=2Hz), 7.49(1H, dd, J=2, 8Hz), 7.20(1H, d, J=8Hz), 5.60-5.45(1H, m), 4.41(2H, d, J=5Hz), 2.76(2H, t, J=6Hz), 2.63(2H, t, J=8Hz), 2.45-2.32(4H, m), 2.21(3H, s), 1.91-1.70(2H, m), 1.46(9H, s) |
| 36-8 | $CDCl_3$* : 8.47-8.41(1H, m), 8.31(1H, d, J=2Hz), 8.15(1H, d, J=8Hz), 7.97-7.89(1H, m), 7.90(1H, d, J=8Hz), 7.74-7.64(2H, m), 7.43(1H, dd, J=2, 8Hz), 7.18(1H, d, J=9Hz), 5.58-5.44(1H, m), 4.42(2H, d, J=5Hz), 2.97(2H, t, J=6Hz), 2.54(2H, t, J=7Hz), 2.40(2H, t, J=6Hz), 2.28(2H, t, J=7Hz), 2.02(3H, s), 1.78-1.58(2H, m), 1.47(9H, s) |
| 37-1 | $CDCl_3$ : 8.36(1H, d, J=2Hz), 7.89-7.77(3H, m), 7.48-7.35(3H, m), 7.15(1H, d, J=8Hz), 7.08-6.97(1H, m), 5.55-5.45(1H, m), 4.39(2H, d, J=5Hz), 3.62-3.52(2H, m), 2.72-2.60(4H, m), 2.49(2H, t, J=6Hz), 2.32(3H, s), 1.95-1.74(2H, m), 1.46(9H, s) |
| 38-1 | DMSO : 9.10-8.95(1H, m), 8.15(1H, s), 8.10-7.90(2H, m), 8.03-7.78(3H, m), 7.53-7.40(2H, m), 3.60-3.47(2H, m), 3.06-2.95(2H, m) |
| 38-2 | $CDCl_3$* : 7.88(1H, s), 7.90-7.77(2H, m), 7.46-7.33(2H, m), 7.15(2H, d, J=8Hz), 7.11(2H, d, J=8Hz), 4.93-4.72(1H, m), 4.25(2H, d, J=6Hz), 3.67-3.53(2H, m), 3.03-2.85(2H, m), 2.82-2.57(4H, m), 2.00-1.82(2H, m), 1.46(9H, s) |

Table 5 ( Part 11 )

| Example Intermediate | NMR (ppm) (No Mark : 300MHz, * : 270MHz) |
|---|---|
| 39-1 | $CDCl_3$ : 8.31(1H, s), 7.90-7.80(4H, m), 7.58-7.45(2H, m), 7.24-7.13(1H, m), 7.15(2H, d, J=8Hz), 7.11(2H, d, J=8Hz), 4.88-4.73(1H, m), 4.25(2H, d, J=6Hz), 3.68-3.53(2H, m), 2.72-2.60(4H, m), 2.54-2.46(2H, m), 2.33(3H, s), 1.92-1.77(2H, m), 1.46(9H, s) |
| 40-1 | $CDCl_3$* : 8.35(1H, d, J=8Hz), 7.88-7.78(2H, m), 7.70-7.52(2H, m), 7.51(1H, d, J=8Hz), 7.12(2H, d, J=8Hz), 7.07(2H, d, J=8Hz), 6.70-6.57(1H, m), 4.90-4.67(1H, m), 4.24(2H, d, J=6Hz), 3.63-3.51(2H, m), 2.67-2.52(4H, m), 2.47-2.37(2H, m), 2.25(3H, s), 1.85-1.67(2H, m), 1.45(9H, s) |
| 41-1 | $CDCl_3$ : 8.26(1H, s), 8.07-8.00(1H, m), 7.90-7.68(3H, m), 7.64-7.53(1H, m), 7.16(2H, d, J=8Hz), 7.11(2H, d, J=8Hz), 7.13-7.00(1H, m), 4.90-4.70(1H, m), 4.26(2H, d, J=8Hz), 3.65-3.50(2H, m), 2.70-2.59(4H, m), 2.53-2.43(2H, m), 2.30(3H, s), 1.90-1.75(2H, m), 1.46(9H, s) |
| 42-1 | $CDCl_3$ : 8.23(1H, s), 7.88-7.71(3H, s), 7.23-7.09(6H, m), 7.08-6.98(1H, m), 4.93-4.73(1H, m), 4.35-4.17(2H, m), 3.93(3H, s), 3.65-3.51(2H, m), 2.73-2.60(4H, m), 2.54-2.43(2H, m), 2.30(3H, s), 1.91-1.76(2H, m), 1.46(9H, s) |
| 43-1 | $CDCl_3$ : 8.54(1H, s), 8.31(1H, s), 8.06(1H, dd, J=2, 9Hz), 7.99(1H, d, J=9Hz), 7.90(1H, d, J=9Hz), 7.87(1H, dd, J=2, 9Hz), 7.16(2H, d, J=8Hz), 7.11(2H, d, J=8Hz), 7.06-6.99(1H, m), 4.90-4.73(1H, m), 4.26(2H, d, J=6Hz), 3.63-3.52(2H, m), 2.69-2.58(4H, m), 2.51-2.42(2H, m), 2.29(3H, s), 1.90-1.75(2H, m), 1.65(9H, s), 1.45(9H, s) |
| 44-1 | $CDCl_3$ : 8.31(1H, s), 7.97-7.83(4H, m), 7.59-7.51(1H, m), 7.15(2H, d, J=8Hz), 7.10(2H, d, J=8Hz), 7.08-6.98(1H, m), 4.91-4.78(1H, m), 4.26(2H, d, J=5Hz), 3.65-3.50(2H, m), 3.17(3H, s), 3.03(3H, s), 2.70-2.58(4H, m), 2.54-2.41(2H, m), 2.29(3H, s), 1.90-1.72(2H, m), 1.46(9H, s) |

Table 5 ( Part 12 )

| Example Intermediate | NMR (ppm) (No Mark : 300MHz, * : 270MHz) |
|---|---|
| 45-1 | $CDCl_3$ : 7.69-7.64(1H, m), 7.47-7.45(1H, m), 7.44-7.33(2H, m), 7.31-7.23(1H, m), 7.15(4H, s), 4.87-4.70(1H, m), 4.26(2H, d, J=6Hz), 3.60-3.47(2H, m), 2.75-2.65(2H, m), 2.64-2.56(2H, m), 2.48-2.40(2H, m), 2.29(3H, s), 1.88-1.74(2H, m), 1.46(9H, s) |
| 46-1 | $CDCl_3$* : 8.06(1H, d, J=2Hz), 7.73(1H, dd, J=2, 9Hz), 7.68(1H, d, J=2Hz), 7.52(1H, d, J=9Hz), 7.20-7.07(4H, m), 6.90-6.75(2H, m), 4.86-4.72(1H, m), 4.24(2H, d, J=6Hz), 3.60-3.47(2H, m), 2.70-2.53(4H, m), 2.44(2H, t, J=6Hz), 2.28(3H, s), 1.88-1.72(2H, m), 1.46(9H, s) |
| 47-1 | $CDCl_3$ : 7.88-7.77(2H, m), 7.79(1H, s), 7.46-7.34(2H, m), 7.25-7.17(1H, m), 7.16(2H, d, J=8Hz), 7.13(2H, d, J=8Hz), 4.90-4.75(1H, m), 4.26(2H, d, J=6Hz), 3.62-3.50(2H, m), 2.70-2.60(4H, m), 2.53-2.45(2H, m), 2.32(3H, s), 1.90-1.77(2H, m), 1.46(9H, s) |
| 48-1 | $CDCl_3$* : 8.30-8.22(1H, m), 7.91(1H, d, J=8Hz), 7.73(1H, dd, J=2, 8Hz), 7.51(1H, d, J=6Hz), 7.36(1H, d, J=6Hz), 7.16(2H, d, J=8Hz), 7.10(2H, d, J=8Hz), 6.98-6.85(1H, m), 4.90-4.70(1H, m), 4.26(2H, d, J=6Hz), 3.60-3.49(2H, m), 2.70-2.55(4H, m), 2.50-2.38(2H, m), 2.28(3H, s), 1.99-1.72(2H, m), 1.46(9H, s) |
| 49-1 | $CDCl_3$* : 7.91-7.79(3H, m), 7.53-7.44(2H, m), 7.17(2H, d, J=8Hz), 7.13(2H, d, J=8Hz), 4.86-4.72(1H, m), 4.26(2H, d, J=6Hz), 3.63-3.53(2H, m), 2.71-2.54(4H, m), 2.46(2H, t, J=7Hz), 2.29(3H, s), 1.89-1.75(2H, m), 1.46(9H, s) |
| 50-1 | $CDCl_3$ : 9.20-9.09(1H, m), 7.67-7.60(1H, m), 7.44(1H, d, J=8Hz), 7.33-7.26(1H, m), 7.18-7.05(5H, m), 6.94-6.80(2H, m), 4.92-4.77(1H, m), 4.26(2H, d, J=5Hz), 3.56-3.43(2H, m), 2.65(2H, t, J=7Hz), 2.62-2.53(2H, m), 2.47-2.37(2H, m), 2.29(3H, s), 1.85-1.73(2H, m), 1.47(9H, s) |

Table 5 ( Part 13 )

| Example Intermediate | NMR (ppm) (No Mark : 300MHz, * : 270MHz) |
|---|---|
| 51-1 | $CDCl_3$ : 8.81-8.65(1H, m), 8.06-7.95(1H, m), 7.71-7.61(1H, m), 7.46-7.40(1H, m), 7.30-7.16(2H, m), 7.13(2H, d, J=8Hz), 7.08(2H, d, J=8Hz), 6.85-6.70(1H, m), 4.98-4.80(1H, m), 4.27(2H, d, J=6Hz), 3.63-3.52(2H, m), 2.70-2.57(4H, m), 2.54-2.44(2H, m), 2.32(3H, s), 1.90-1.75(2H, m), 1.47(9H, s) |
| 52-1 | $CDCl_3$ : 8.80-8.55(1H, m), 8.16-8.06(1H, m), 7.66-7.55(1H, m), 7.34(1H, d, J=8Hz), 7.30-7.22(1H, m), 7.14(2H, d, J=8Hz), 7.10(2H, d, J=8Hz), 6.97-6.84(1H, m), 6.63-6.55(1H, m), 4.95-4.77(1H, m), 4.26(2H, d, J=6Hz), 3.60-3.50(2H, m), 2.72-2.55(4H, m), 2.51-2.39(2H, m), 2.28(3H, s), 1.94-1.72(2H, m), 1.46(9H, s) |
| 53-1 | $CDCl_3$ : 8.84-8.65(1H, m), 7.99(1H, s), 7.63(1H, d, J=8Hz), 7.45(1H, dd, J=2, 8Hz), 7.36-7.30(1H, m), 7.14(2H, d, J=8Hz), 7.09(2H, d, J=8Hz), 7.00-6.84(1H, m), 6.62-6.53(1H, m), 4.95-4.75(1H, m), 4.26(2H, d, J=5Hz), 3.62-3.47(2H, m), 2.73-2.54(4H, m), 2.50-2.35(2H, m), 2.28(3H, s), 1.90-1.70(2H, m), 1.46(9H, s) |
| 54-1 | $CDCl_3$ : 7.64-7.57(1H, m), 7.42-7.33(1H, m), 7.35-7.28(1H, m), 7.19-7.08(5H, m), 6.92-6.85(1H, m), 6.83(1H, d, J=1Hz), 4.86-4.75(1H, m), 4.26(1H, d, J=6Hz), 4.06(3H, s), 3.54-3.46(2H, m), 2.64(2H, t, J=8Hz), 2.59(2H, t, J=6Hz), 2.44(2H, t, J=8Hz), 2.27(3H, s), 1.84-1.72(2H, m), 1.46(9H, s) |
| 55-1 | $CDCl_3$ : 9.18-9.00(1H, m), 7.43-7.38(1H, m), 7.36-7.29(1H, m), 7.20-7.07(5H, m), 6.97-6.80(1H, m), 6.78-6.72(1H, m), 4.95-4.77(1H, m), 4.26(2H, d, J=5Hz), 3.57-3.37(2H, m), 2.70-2.55(4H, m), 2.44(3H, s), 2.50-2.38(2H, m), 2.30(3H, s), 1.90-1.75(2H, m), 1.47(9H, s) |

Table 5 ( Part 14 )

| Example Intermediate | NMR (ppm) (No Mark : 300MHz, * : 270MHz) |
|---|---|
| 56-1 | $CDCl_3$ : 9.72-9.44(1H, m), 7.45(1H, s), 7.37(1H, d, J=8Hz), 7.22-7.05(5H, m), 7.00-6.83(1H, m), 6.80-6.73(1H, m), 5.00-4.80(1H, m), 4.26(2H, d, J=5Hz), 3.62-3.35(2H, m), 3.07-2.92(1H, m), 2.70-2.53(4H, m), 2.48-2.37(2H, m), 2.28(3H, s), 1.88-1.73(2H, m), 1.46(9H, s), 1.29(6H, d, J=7Hz) |
| 57-1 | $CDCl_3$ : 10.20-9.90(1H, m), 7.36(1H, d, J=8Hz), 7.18(1H, t, J=8Hz), 7.19-7.08(5H, m), 7.07-6.96(1H, m), 6.94-6.89(1H, m), 4.95-4.77(1H, m), 4.25(2H, d, J=5Hz), 3.67-3.35(2H, m), 2.70-2.55(4H, m), 2.50-2.38(2H, m), 2.30(3H, s), 1.88-1.75(2H, m), 1.46(9H, s) |
| 58-1 | $CDCl_3$ : 9.30-9.25(1H, m), 7.59(1H, d, J=2H, s), 7.36(1H, d, J=9Hz), 7.23(1H, dd, J=2, 9Hz), 7.16(2H, d, J=8Hz), 7.11(2H, d, J=8Hz), 6.95-6.85(1H, m), 6.75(1H, d, J=2Hz), 4.93-4.81(1H, m), 4.27(2H, d, J=6Hz), 3.57-3.42(2H, m), 2.70-2.52(4H, m), 2.49-2.37(2H, m), 2.29(3H, s), 1.88-1.72(2H, m), 1.47(9H, s) |
| 60-1 | $CDCl_3$* : 9.50-9.30(1H, m), 7.53(1H, d, J=9Hz), 7.44(1H, s), 7.20-7.05(5H, m), 6.95-6.83(1H, m), 6.82-6.75(1H, m), 4.95-4.75(1H, m), 4.26(2H, d, J=6Hz), 3.60-3.30(2H, m), 2.70-2.53(4H, m), 2.50-2.35(2H, m), 2.28(3H, s), 1.89-1.72(2H, m), 1.47(9H, s) |
| 61-1 | $CDCl_3$ : 9.55-9.35(1H, m), 7.75(1H, s), 7.80-7.60(2H, m), 7.40-7.20(4H, m), 6.90-6.80(1H, ms), 6.73(1H, s), 4.93-4.80(1H, m), 4.27(2H, d, J=5Hz), 3.58-3.42(2H, m), 2.70-2.52(4H, m), 2.48-2.36(2H, m), 2.29(3H, s), 1.89-1.72(2H, m), 1.47(9H, s) |
| 62-1 | $CDCl_3$ : 9.22-9.07(1H, m), 7.42-7.31(1H, m), 7.30-7.20(1H, m), 7.20-7.10(4H, m), 7.10-7.00(1H, m), 6.91-6.76(1H, m), 6.80-6.72(1H, m), 4.93-4.77(1H, m), 4.27(2H, d, J=6Hz), 3.58-3.42(2H, m), 2.70-2.52(4H, m), 2.48-2.32(2H, m), 2.28(3H, s), 1.89-1.72(2H, m), 1.47(9H, s) |

Table 5 ( Part 15 )

| Example Intermediate | NMR (ppm) (No Mark : 300MHz, * : 270MHz) |
|---|---|
| 63-1 | $CDCl_3$ : 9.80-9.65(1H, m), 7.93(1H, s), 7.60-7.44(2H, m), 7.20-7.05(4H, m), 7.04-6.94(1H, m), 6.93-6.84(1H, ms), 4.95-4.76(1H, m), 4.26(2H, d, J=5Hz), 3.62-3.42(2H, m), 2.72-2.36(6H, m), 2.30(3H, s), 1.90-1.74(2H, m), 1.47(9H, s) |
| 64-1 | $CDCl_3$ : 9.45-9.24(1H, m), 7.20(1H, t, J=8Hz), 7.20-7.10(4H, m), 7.05(1H, d, J=8Hz), 7.00-6.92 (1H, m), 6.92-6.81(1H, m), 6.51(1H, d, J=8Hz), 4.95-4.78(1H, m), 4.26(2H, d, J=5Hz), 3.95(3H, s), 3.57-3.37(2H, m), 2.72-2.50(4H, m), 2.48-2.33(2H, m), 2.27(3H, s), 1.90-1.68(2H, m), 1.47(9H, s) |
| 65-1 | $CDCl_3$ : 9.25-9.05(1H, m), 7.33(1H, d, J=9Hz), 7.16(2H, d, J=8Hz), 7.13(2H, d, J=8Hz), 7.03(1H, d, J=2Hz), 6.95(1H, dd, J=2, 9Hz), 6.90-6.80(1H, m), 6.76-6.72(1H, m), 4.90-4.60(1H, m), 4.27(2H, d, J=6Hz), 3.84(3H, s), 3.58-3.40(2H, m), 2.72-2.54(4H, m), 2.48-2.36(2H, m), 2.28(3H, s), 1.90-1.73(2H, m), 1.47(9H, s) |
| 66-1 | $CDCl_3$ : 9.34-9.12(1H, m), 7.48(1H, d, J=9Hz), 7.16(2H, d, J=8Hz), 7.12(2H, d, J=8Hz), 6.92-6.83(1H, m), 6.80(1H, dd, J=2, 9Hz), 6.85-6.72(2H, m), 4.97-4.75(1H, m), 4.26(2H, d, J=5Hz), 3.85(3H, s), 3.60-3.35(2H, m), 2.70-2.53(4H, m), 2.48-2.35(2H, m), 2.28(3H, s), 1.87-1.74(2H, m), 1.47(9H, s) |
| 67-1 | $CDCl_3$* : 9.40-9.30(1H, m), 7.53-7.38(2H, m), 7.21-7.07(5H, m), 6.98-6.83(1H, m), 6.85-6.77(1H, m), 4.95-4.75(1H, m), 4.26(2H, d, J=6Hz), 3.60-3.40(2H, m), 2.71-2.52(4H, m), 2.50-2.35(2H, m), 2.29(3H, s), 1.90-1.71(2H, m), 1.47(9H, s) |
| 68-1 | $CDCl_3$* : 10.65-10.15(1H, m), 7.99(1H, s), 7.60-7.40(2H, m), 7.20-6.97(5H, m), 6.88(1H, s), 5.00-4.75(1H, m), 4.26(2H, d, J=6Hz), 3.70-3.20(2H, m), 2.70-2.53(4H, m), 2.51-2.34(2H, m), 2.30(3H, s), 1.90-1.72(2H, m), 1.47(9H, s) |

Table 5 ( Part 16 )

| Example Intermediate | NMR (ppm) (No Mark : 300MHz, * : 270MHz) |
|---|---|
| 69-1 | $CDCl_3$ : 8.36-8.30(1H, m), 7.96-7.87(1H, m), 7.48-7.39(1H, m), 7.21-7.06(4H, m), 7.04-6.94(1H, m), 6.93-6.86(2H, m), 5.03-4.80(1H, m), 4.26(2H, d, J=5Hz), 3.65-3.40(2H, m), 2.72-2.53(4H, m), 2.50-2.35(2H, m), 2.29(3H, s), 1.93-1.72(2H, m), 1.62(9H, s), 1.46(9H, s) |
| 70-1 | $CDCl_3$* : 9.40-9.29(1H, m), 8.34-8.28(1H, m), 7.95(1H, dd, J=2, 8Hz), 7.47(1H, d, J=8Hz), 7.22-7.08(4H, m), 6.97-6.86(2H, m), 4.96-4.82(1H, ms), 4.27(2H, d, J=6Hz), 3.58-3.42(2H, m), 2.72-2.52(4H, m), 2.66(3H, s), 2.48-2.36(2H, m), 2.30(3H, s), 1.90-1.73(2H, m), 1.47(9H, s) |
| 71-1 | $CDCl_3$ : 9.67-9.62(1H, m), 8.32-8.26(1H, m), 7.84-7.77(1H, m), 7.59(1H, d, J=9Hz), 7.21-7.05(4H, m), 7.06-6.89(1H, m), 6.93(1H, s), 5.00-4.85(1H, m), 4.27(2H, d, J=5Hz), 3.60-3.43(2H, m), 3.09(3H, s), 2.72-2.53(4H, m), 2.50-2.38(2H, m), 2.30(3H, s), 1.89-1.74(2H, m), 1.47(9H, s) |
| 72-1 | $CDCl_3$ : 9.30-9.20(1H, m), 7.51-7.28(6H, m), 7.20-7.08(5H, m), 7.07-7.00(1H, m), 6.93-6.80(1H, m), 6.76-6.70(1H, m), 5.09(2H, s), 4.96-4.80(1H, m), 4.26(2H, d, J=5Hz), 3.60-3.40(2H, m), 2.72-2.51(4H, m), 2.50-2.34(2H, m), 2.28(3H, s), 1.92-1.72(2H, m), 1.46(9H, s) |
| 73-1 | $CDCl_3$* : 8.08(1H, d, J=2Hz), 7.69(1H, dd, J=2, 9Hz), 7.32(1H, d, J=9Hz), 7.16(2H, d, J=8Hz), 7.12(2H, d, J=8Hz), 7.10(1H, d, J=3Hz), 6.90-6.80(1H, m), 6.53(1H, d, J=3Hz), 4.90-4.70(1H, m), 4.26(2H, d, J=6Hz), 3.81(3H, s), 3.60-3.47(2H, m), 2.70-2.55(4H, m), 2.50-2.37(2H, m), 2.27(3H, s), 1.88-1.73(2H, m), 1.46(9H, s) |
| 74-1 | $CDCl_3$ : 8.15-8.05(1H, m), 7.99-7.95(1H, m), 7.57-7.48(1H, m), 7.27-7.20(1H, m), 7.17-7.07(4H, m), 6.90-6.76(1H, m), 6.27-6.24(1H, m), 4.89-4.75(1H, m), 4.26(2H, d, J=5Hz), 3.58-3.47(2H, m), 2.70-2.55(4H, m), 2.50-2.40(2H, m), 2.46(3H, s), 2.28(3H, s), 1.87-1.74(2H, m), 1.46(9H, s) |

Table 5 ( Part 17 )

| Example Intermediate | NMR (ppm) (No Mark : 300MHz, * : 270MHz) |
|---|---|
| 75-1 | $CDCl_3$* : 8.00-7.88(2H, m), 7.55-7.45(1H, m), 7.19(1H, d, J=9Hz), 7.17-7.07(4H, m), 6.93-6.78(1H, m), 4.90-4.69(1H, m), 4.32-4.18(2H, m), 3.61-3.48(2H, m), 2.71-2.52(4H, m), 2.50-2.40(2H, m), 2.37(3H, s), 2.28(3H, s), 2.20(3H, s), 1.89-1.71(2H, m), 1.46(9H, s) |
| 76-1 | CDCl3* : 8.39(1H, d, J=9Hz), 8.10(1H, d, J=1Hz), 7.80-7.70(3H, m), 7.68-7.50(3H, m), 7.37(1H, d, J=4Hz), 7.18(2H, d, J=8Hz), 7.13(2H, d, J=8Hz), 6.96-6.85(1H, m), 6.67-6.63(1H, m), 4.95-4.75(1H, m), 4.27(2H, d, J=6Hz), 3.62-3.57(2H, m), 2.70-2.56(4H, m), 2.50-2.40(2H, m), 2.29(3H, s), 1.88-1.75(2H, m), 1.45(9H, s) |
| 77-1 | $CDCl_3$ : 8.04-7.91(3H, m), 7.56-7.44(2H, m), 7.20(2H, d, J=9Hz), 7.14(2H, d, J=9Hz), 4.88-4.72(1H, m), 4.25(2H, d, J=5Hz), 3.63-3.52(2H, m), 2.70(2H, t, J=7Hz), 2.62(2H, t, J=6Hz), 2.44(2H, t, J=7Hz), 2.30(3H, s), 1.89-1.73(2H, m), 1.45(9H, s) |
| 78-1 | $CDCl_3$ : 9.11(1H, s), 8.51-8.45(1H, m), 8.18-8.12(1H, m), 7.90-7.83(1H, m), 7.17(2H, d, J=8Hz), 7.11(2H, d, J=8Hz), 7.02-6.90(1H, m), 4.90-4.73(1H, m), 4.27(2H, d, J=6Hz), 3.62-3.47(2H, m), 2.68-2.57(4H, m), 2.52-2.40(2H, m), 2.29(3H, s), 1.88-1.73(2H, m), 1.46(9H, s) |
| 79-1 | $CDCl_3$* : 7.33-7.25(1H, m), 7.28(1H, s), 7.18(2H, d, J=8Hz), 7.11(2H, d, J=8Hz), 6.85-6.77(1H, m), 6.75-6.65(1H, m), 6.02(2H, s), 4.90-4.73(1H, m), 4.27(2H, d, J=6Hz), 3.55-3.40(2H, m), 2.67-2.51(4H, m), 2.47-2.37(2H, m), 2.25(3H, s), 1.86-1.71(2H, m), 1.46(9H, s) |
| 80-1 | $CDCl_3$ : 8.73-8.62(1H, m), 8.31(2H, s), 8.07-8.01(1H, m), 7.90-7.84(1H, m), 7.73-7.65(1H, m), 7.63-7.55(1H, m), 7.16(2H, d, J=8Hz), 7.12(2H, d, J=8Hz), 4.85-4.70(1H, m), 4.25(2H, d, J=7Hz), 3.67-3.55(2H, m), 2.80-2.60(4H, m), 2.46(2H, t, J=7Hz), 2.33(3H, s), 1.90-1.77(2H, m), 1.46(9H, s) |

Table 5 ( Part 18 )

| Example Intermediate | NMR (ppm) (No Mark : 300MHz, * : 270MHz) |
|---|---|
| 81-1 | $CDCl_3$ : 8.98(1H, dd, J=2, 4Hz), 8.32(1H, d, J=2Hz), 8.23-8.16(1H, m), 8.13(1H, d, J=9Hz), 8.04(1H, dd, J=2, 9Hz), 7.46(1H, dd, J=4, 8Hz), 7.16(2H, d, J=8Hz), 7.11(2H, d, J=8Hz), 7.10-7.02(1H, m), 4.95-4.80(1H, m), 4.26(2H, d, J=6Hz), 3.65-3.50(2H, m), 2.70-2.58(4H, m), 2.52-2.42(2H, m), 2.30(3H, s), 1.93-1.75(2H, m), 1.46(9H, s) |
| 82-1 | $CDCl_3$ : 8.05(1H, d, J=2Hz), 7.86-7.76(1H, m), 7.80(1H, d, J=10Hz), 7.33(1H, d, J=8Hz), 7.18(2H, d, J=8Hz), 7.12(2H, d, J=8Hz), 6.97-6.85(1H, m), 6.72(1H, d, J=10Hz), 5.05-4.85(1H, m), 4.28(2H, d, J=6Hz), 3.62-3.47(2H, m), 2.70-2.57(4H, m), 2.53-2.41(2H, m), 2.29(3H, s), 1.90-1.72(2H, m), 1.46(9H, s) |
| 83-1 | $CDCl_3$ : 8.11(1H, s), 7.88-7.82(2H, m), 7.75(1H, s), 7.46-7.37(3H, m), 7.20(2H, d, J=8Hz), 7.12(2H, d, J=8Hz), 4.90-4.75(1H, m), 4.27(2H, d, J=7Hz), 3.71-3.59(4H, m), 3.32(2H, t, J=7Hz), 2.63(2H, t, J=7Hz), 2.01-1.88(2H, m), 1.46(9H, s) |
| 84-1 | $CDCl_3$ : 7.88-7.80(2H, m), 7.80-7.73(1H, m), 7.73(1H, s), 7.45-7.34(2H, m), 7.21(2H, d, J=8Hz), 7.13(2H, d, J=8Hz), 4.92-4.78(1H, m), 4.28(2H, d, J=6Hz), 3.68-3.56(4H, m), 3.35-3.25(2H, m), 2.64(2H, t, J=8Hz), 2.05(3H, s), 2.02-1.87(2H, m), 1.46(9H, s) |
| 85-1 | $CDCl_3$ : 8.07-8.00(1H, m), 7.98(1H, s), 7.90-7.76(2H, m), 7.44-7.33(2H, m), 7.13(2H, d, J=8Hz), 7.08(2H, d, J=8Hz), 4.90-4.73(1H, m), 4.25(2H, d, J=5Hz), 3.47-3.37(2H, m), 3.26(2H, s), 2.78-2.70(2H, m), 2.67-2.57(4H, m), 1.84-1.70(2H, m), 1.50(9H, s), 1.46(9H, s) |

Table 5 ( Part 19 )

| Example Intermediate | NMR (ppm) (No Mark : 300MHz, * : 270MHz) |
|---|---|
| 87-1 | $CDCl_3$ : 7.80-7.68(2H, m), 7.63-7.53(2H, m), 7.43-7.23(4H, m), 7.20-7.05(4H, m), 4.85-4.70(1H, m), 4.55-4.15(5H, m), 3.45-3.33(1H, m), 3.25-3.13(1H, m), 2.96(1.5H, s), 2.91(1.5H, s), 2.65-2.20(6H, m), 2.27(1.5H, s), 2.11(1.5H, s), 1.84-1.60(2H, s), 1.46(9H, s) |
| 87-2 | $CDCl_3$ : 7.19(2H, d, J=8Hz), 7.14(2H, d, J=8Hz), 4.95-4.75(1H, m), 4.28(2H, d, J=6Hz), 2.66-2.56(4H, m), 2.50-2.41(2H, m), 2.43(3H, s), 2.40-2.32(2H, m), 2.20(3H, s), 1.85-1.68(2H, m), 1.46(9H, s) |
| 87-3 | $CDCl_3$ : 7.63-7.59(1H, m), 7.35-6.90(3H, m), 7.16(2H, d, J=8Hz), 7.09(2H, d, J=8Hz), 4.27(2H, d, J=6Hz), 3.73-3.55(2H, m), 3.20-2.94(2H, m), 2.80-2.20(10H, m), 1.98-1.63(2H, m), 1.47(9H, s) |
| 88-1 | $CDCl_3$ : 8.29(1H, d, J=2Hz), 7.89-7.82(3H, m), 7.52-7.44(2H, m), 7.40(1H, dd, J=2, 8Hz), 7.17(1H, d, J=8Hz), 5.60-5.45(1H, m), 4.41(2H, d, J=5Hz), 3.12(2H, t, J=6Hz), 2.54-2.42(4H, m), 2.28(2H, t, J=7Hz), 2.07(3H, s), 1.75-1.63(2H, m), 1.47(9H, s) |
| 89-1 | $CDCl_3$* : 8.60-8.45(1H, m), 8.38-8.28(1H, m), 8.11(1H, br.s), 7.61(1H, dd, J=2, 9Hz), 7.44-7.34(1H, m), 7.35(1H, d, J=9Hz), 7.30-7.24(1H, m), 7.09(1H, d, J=8Hz), 6.92-6.77(1H, m), 6.63-6.56(1H, m), 5.60-5.40(1H, m), 4.39(2H, d, J=5Hz), 3.62-3.49(2H, m), 2.71-2.57(4H, m), 2.53-2.40(2H, m), 2.30(3H, s), 1.90-1.75(2H, m), 1.47(9H, s) |
| 90-1 | $CDCl_3$ : 9.82(1H, t, J=1Hz), 6.74(1H, d, J=3Hz), 6.63(1H, d, J=3Hz), 4.92-4.75(1H, m), 4.39(2H, d, J=6Hz), 3.16-3.05(2H, m), 2.87-2.77(2H, m), 1.46(9H, s) |
| 90-2 | $CDCl_3$* : 7.90-7.77(2H, m), 7.75(1H, s), 7.60-7.43(1H, m), 7.45-7.33(2H, m), 3.70-3.45(4H, m), 2.93(3H, s), 1.46(9H, s) |

Table 5 ( Part 20 )

| Example Intermediate | NMR (ppm) (No Mark : 300MHz, * : 270MHz) |
|---|---|
| 90-3 | $CD_3OD$* : 8.00(1H, s), 7.96-7.87(2H, m), 7.51-7.38(2H, m), 3.77-3.67(2H, m), 3.30-3.22(2H, m), 2.76(3H, s) |
| 90-4 | $CDCl_3$ : 7.87-7.78(2H, m), 7.76(1H, s), 7.46-7.35(2H, m), 6.93-6.83(1H, m), 6.71(1H, d, J=3Hz), 6.59(1H, d, J=3Hz), 4.92-4.77(1H, m), 4.37(2H, d, J=5Hz), 3.58-3.47(2H, m), 2.84(2H, t, J=7Hz), 2.60(2H, t, J=6Hz), 2.47(2H, t, J=7Hz), 2.28(3H, s), 1.93-1.79(2H, m), 1.45(9H, s) |
| 91-1 | $CDCl_3$* : 7.83-7.75(1H, m), 7.72-7.65(1H, m), 7.36-7.23(2H, m), 7.17(2H, d, J=8Hz), 7.12(2H, d, J=8Hz), 7.20-7.08(1H, m), 4.87-4.70(1H, m), 4.27(2H, d, J=6Hz), 4.10-4.05(2H, m), 2.79-2.68(2H, m), 2.64-2.45(4H, m), 2.42-2.30(2H, m), 2.19(3H, s), 1.88-1.67(2H, m), 1.46(9H, s) |

<<Pharmacological Example>>

1. β-tryptase inhibition test

Sixty μL of a tris-hydrochloric acid buffer (pH 7.5, containing 200 ng/mL of heparin) was added to 20 μL of β-tryptase (manufactured by Promega Corporation) adjusted to 100 ng/mL with the same buffer. Then, 1 μL of a dimethyl sulfoxide (hereinafter, abbreviated to DMSO) solution as a test compound adjusted to a concentration 100 times as high as the final concentration was added to the mixture. After that, 20 μL of a 0.5 mM synthetic substrate Boc-Phe-Ser-Arg-MCA (manufactured by Peptide Institute, Inc) was added to the mixture, and the whole was reacted at 25°C for 1 hour, followed by the addition of 100 μL of 20% acetic acid for stopping the reaction. Measured was fluorescence of free MCA generated by laser light with an excitation wavelength of 355 nm (fluorescent wavelength=460 nm, hereinafter abbreviated to F 460 nm).

Δ fluorescence count value (a fluorescence count value after an enzyme reaction - a fluorescence count value before the enzyme reaction) was used for a measure of an enzyme inhibition reaction of a drug, and the enzyme inhibition reaction was evaluated in terms of a suppression rate calculated by the following equation. As a result, in each of Example Compounds 4, 53, 55, 61, 73, and 87, the β-tryptase inhibitory activity was 0.01 μM or less in terms of $IC_{50}$, which means that each of the compounds exhibited a high activity (Table 6).

Here, $IC_{50}$ represents an inhibitor concentration that shows a suppression rate of 50%.

A=Δ F 460 nm of a mixture (control) of a substrate containing 1% DMSO and an enzyme
B=Δ F 460 nm of a mixture of a substrate containing a drug dissolved in DMSO and an enzyme
C=Δ F 460 nm of a mixture (blank) containing 1% DMSO and a substrate

$$\text{Suppression rate (\%)} = (A - B) \times 100 / (A - C)$$

Table 6

| Example Compound | Tryptase Inhibiting Activity $IC_{50}$ (μM) |
|---|---|
| 1 | 0.086 |
| 4 | 0.005 |
| 5 | 0.21 |
| 7 | 0.20 |
| 9 | 0.19 |
| 11 | 0.15 |
| 13 | 0.19 |
| 17 | 0.12 |
| 18 | 0.14 |
| 20 | 0.015 |
| 33 | 0.44 |
| 45 | 0.020 |
| 46 | 0.064 |
| 49 | 0.015 |
| 51 | 0.10 |
| 53 | 0.005 |
| 55 | 0.005 |
| 61 | 0.006 |
| 62 | 0.014 |
| 63 | 0.032 |
| 69 | 0.055 |
| 73 | 0.001 |
| 77 | 0.073 |
| 78 | 0.020 |
| 87 | 0.001 |
| 89 | 0.14 |
| 90 | 0.20 |
| 91 | 0.31 |

2. β-tryptase inhibition selectivity test

Selectivities of β-tryptase, trypsin, and FXa inhibiting activities were compared by the following test method. As a result, in each of Example Compounds 4, 41, 55, 58, 61, and 70, the β-tryptase inhibitory activity was 0.01 μM or less in terms of $IC_{50}$, whereas trypsin and FXa inhibiting activities were 100 μM or more in terms of $IC_{50}$. This means that each of the compounds exhibited high selectivity to β-tryptase (Table 7).

<β-tryptase inhibition test>

The β-tryptase inhibitory activity was measured by the method described above.

<Trypsin inhibition test>

Added to 158 µL of a 20 mM tris-hydrochloric acid buffer (pH 7.5, containing 130 mM NaCl, 3.75 mM $CaCl_2$, and 0.1% BSA) was 20 µL of a trypsin solution (manufactured by Sigma, a stock solution of 100 µg/mL was adjusted to 100 ng/mL with 0.001 N HCl containing 0.1% BSA). After that, 2 µL of a DMSO solution as a test compound adjusted to a concentration 100 times as high as the final concentration was added to the mixture, and the whole was reacted at 37°C for 3 minutes. Then, 20 µL of a 2 mM synthetic substrate S-2222 (manufactured by Daiichi Pure Chemicals Co., Ltd.) was added to the mixture, and the whole was reacted at 37°C for an additional 20 minutes, followed by addition of 100 µL of 20% acetic acid for stopping the reaction. After that, absorbance at 405 nm (hereinafter, abbreviated to OD 405 nm) was measured with a microplate reader.

<FXa inhibition test>

158 µL of a 20 mM tris-hydrochloric acid buffer (pH 7.5, containing 130 mM NaCl, 3.75 mM $CaCl_2$, and 0.1% BSA) was placed on a 96 well microplate, and 20 µL of FXa (manufactured by Enzyme Research Ltd.) adjusted to 0.1 U/mL with the buffer was added to the buffer. Then, 2 µL of a DMSO solution as a test compound adjusted to a concentration 100 times as high as the final concentration was added to the mixture, and the whole was reacted at 37°C for 3 minutes. After that, 20 µL of an S-2222 substrate solution adjusted to 2 mM with the buffer was added to the mixture, and the whole was reacted at 37°C for 30 minutes, followed by addition of 100 µL of 20% acetic acid for stopping the reaction. After that, OD 405 nm was measured with a microplate reader.

Table 7

| Example Compound | Enzyme | Inhibition | $IC_{50}$ (µM) |
|---|---|---|---|
| | Tryptase | Trypsin | FXa |
| 1 | 0. 086 | >100 | >100 |
| 4 | 0. 005 | >100 | >100 |
| 11 | 0. 15 | >30 | >30 |
| 17 | 0. 12 | >30 | >30 |
| 41 | 0. 008 | >100 | >100 |
| 55 | 0. 005 | >100 | >100 |
| 58 | 0. 006 | >100 | >100 |
| 61 | 0. 006 | >100 | >100 |
| 70 | 0. 008 | >100 | >100 |
| 72 | 0. 015 | >100 | >100 |

3. Toxicity test

Eight male BALB/c mice at 9 weeks old (each having a body weight of about 25 g) was gathered in one group. The amine derivative of Example Compound 1 dissolved in 0.1 M tartaric acid to be adjusted to 1 mg/mL was administered to a caudal vein of each of the mice at a dose of 10 mg/kg (the administration was repeated 4 times at an interval of 2 hours), and general symptoms of the mice were observed for 24 hours. There was no fatal example, and no particularly problematic symptom was recognized.

<<Preparation Example>>

Hereinafter, examples of the medicinal composition of the present invention are given. Here, a compound M refers to the compound of the present invention of the formula (I) or the pharmaceutically acceptable salt thereof (pharamaceutically acceptable salt). More specifically, the compound M refers to any compound selected from Example Compounds.

(a) Tablet (active ingredient content 1 mg)

1.0 g of the compound M, 90.0 g of lactose, 5.0 g of sodium carboxymethylcellulose, 1.0 g of corn starch paste (5% W/V paste), and 1.0 g of magnesium stearate were weighted out, respectively. The products were tableted to prepare tablets each weighing 100 mg.

(b) Tablet (active ingredient content 10 mg)

10 g of the compound M, 150 g of lactose, 6.0 g of croscarmellose sodium, 28.5 g of corn starch paste (5% W/V paste), 2.5 g of polyvinyl pyrrolidone, and 3 g of magnesium stearate were weighted out, respectively. The products were tableted to prepare tablets each weighing 200 mg. Then, each of the tablets were coated with cellulose acetate phthalate to obtain an enteric agent.

(c) Tablet (active ingredient content 100 mg)

100 g of the compound M, 180 g of lactose, 13 g of croscarmellose sodium, 4 g of corn starch paste (5% W/V paste), and 3 g of magnesium stearate were weighted out, respectively. The products were tableted to prepare tablets each weighing 300 mg.

(d) Capsule (active ingredient content 50 mg)

100 g of the compound M, 395.5 g of lactose, and 4.5 g of magnesium stearate were weighted out, respectively, followed by uniform mixing. 250 mg of the mixed powder was encapsulated in a hard capsule of Japanese Pharmacopoeia No. 1.

Industrial Applicability

According to the present invention, there is provided a low-molecular amine derivative that is absorbed well, has low toxicity, and has a strong and highly selective inhibitory activity against a human mast cell β-tryptase.

Furthermore, a medicine containing the compound of the present invention as an active ingredient has an excellent property as a prophylactic/therapeutic agent for diseases the crisis and evolution of which are considered to be attributed to β-tryptase, such as: respiratory failures such as bronchial asthma, lung fibrosis, and chronic obstructive pulmonary disease (COPD); and allergic diseases such as anaphylaxis, allergic rhinitis, allergic conjunctivitis, atopic dermatitis, and urticaria. In addition, the medicine containing the compound of the present invention as an active ingredient has an excellent property as a prophylactic/therapeutic agent for diseases the crisis and evolution of which are considered to be attributed to β-tryptase, such as: an inflammatory bowel disease; a hyperproliferative skin disease; vascular edema; and rheumatoid arthritis. Particularly, the medicine containing the compound of the present invention as an active ingredient has an excellent property as a prophylactic/therapeutic agent for bronchial asthma and an allergic disease such as atopic dermatitis.

## Claims

**1.** An amine derivative represented by the following formula (I) or a pharmaceutically acceptable salt thereof:

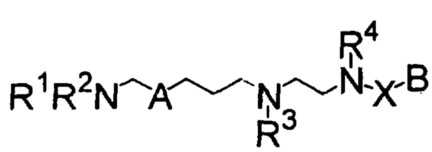

(I)

wherein $R^1$, $R^2$, and $R^3$ each independently is hydrogen atom, a lower alkyl group, a lower alkenyl group, a lower alkynyl group, or a lower acyl group; $R^1$ and $R^2$ may be bonded to each other to form one of a 5-membered ring and a 6-membered ring each containing nitrogen atom to which $R^1$ and $R^2$ are bonded; and 1 to 5 hydrogen atoms in each of the lower alkyl group, the lower alkenyl group, the lower alkynyl group, and the lower acyl group may be substituted by a hydroxyl group, an amino group, a carboxyl group, a lower alkoxyl group, or a lower alkoxycarbonyl group;

$R^4$ is hydrogen atom or a lower alkyl group;

A is an aromatic ring which is a 5-membered aromatic ring or 6-membered aromatic ring, wherein the aromatic ring may contain 1 to 4 hetero atoms selected from the group consisting of nitrogen atom, sulfur atom, and oxygen atom, and A may be substituted;

B is a saturated or unsaturated hydrocarbon group which is a 5-to-7-membered monocyclic hydrocarbon group or 6-to-12-membered condensed bicyclic hydrocarbon group, wherein the hydrocarbon group may contain 1 to 3 hetero atoms selected from the group consisting of nitrogen atom, sulfur atom, and oxygen atom, and each of these groups may be substituted with any of substituents excluding an amino group, an amidino group, and a guanidino group, with the proviso that B is a benzene ring the substituent is any one of substituents excluding a carboxyl group, an alkoxycarbonyl group, and a carbamoyl group; and

X is a carbonyl group (-CO-), a sulfonyl group ($-SO_2-$), a methylene group ($-CH_2-$), a vinylenecarbonyl group (-CO-CH=CH-), a vinylenesulfonyl group ($-SO_2-CH=CH-$), an oxymethylenecarbonyl group ($-CO-CH_2O-$), or an oxymethylenesulfonyl group ($-SO_2-CH_2O-$).

**2.** The amine derivative or the pharmaceutically acceptable salt thereof according to claim 1, wherein each of $R^1$ and $R^2$ is hydrogen atom.

**3.** The amine derivative or the pharmaceutically acceptable salt thereof according to claim 1 or 2, wherein the aromatic ring A is a benzene ring.

**4.** The amine derivative or the pharmaceutically acceptable salt thereof according to any one of claims 1 to 3, wherein X is a carbonyl group (-CO-), a sulfonyl group ($-SO_2-$), or a methylene group ($-CH_2-$).

**5.** The amine derivative or the pharmaceutically acceptable salt thereof according to any one of claims 1 to 4, wherein B is a saturated or unsaturated 6-to-12-membered condensed bicyclic hydrocarbon group which may contain 1 to 3 hetero atoms selected from the group consisting of nitrogen atom, sulfur atom, and oxygen atom.

**6.** A medicine **characterized by** containing the amine derivative or the pharmaceutically acceptable salt thereof according to any one of claims 1 to 5.

**7.** The medicine according to claim 6 **characterized in that** the medicine is a β-tryptase inhibitor.

**8.** A prophylactic/therapeutic agent for an allergic disease comprising the medicine according to claim 6 or 7.

International application No.
PCT/JP02/07843

**A. CLASSIFICATION OF SUBJECT MATTER**

Int.Cl[7] C07C211/30, 311/14, 311/18, 233/78, 235/50, C07C235/10, 235/66, A61K31/137, 31/166, 31/18, A61K31/381, 31/428, 31/47, 31/472, A61P1/00, A61P9/00, 11/00, 11/06, 17/00,

According to International Patent Classification (IPC) or to both national classification and IPC

**B. FIELDS SEARCHED**

Minimum documentation searched (classification system followed by classification symbols)

Int.Cl[7] C07C211/30, 311/14, 311/18, 233/78, 235/50, C07C235/10, 235/66, A61K31/137, 31/166, 31/18, A61K31/381, 31/428, 31/47, 31/472, A61P1/00, A61P9/00, 11/00, 11/06, 17/00,

Documentation searched other than minimum documentation to the extent that such documents are included in the fields searched

Electronic data base consulted during the international search (name of data base and, where practicable, search terms used)

REGISTRY(STN), CA(STN)

**C. DOCUMENTS CONSIDERED TO BE RELEVANT**

| Category* | Citation of document, with indication, where appropriate, of the relevant passages | Relevant to claim No. |
|---|---|---|
| A | WO 99/40083 A2 (Max-Planck-Gesellschaft zur Forderung Der Wissenschaften E.V.), 12 August, 1999 (12.08.99), & JP 2002-502850 A | 1-8 |
| A | WO 99/40073 A2 (Max-Planck-Gesellschaft zur Forderung Der Wissenschaften E.V.), 12 August, 1999 (12.08.99), & JP 2002-502845 A | 1-8 |
| A | EP 893437 A1 (Ono Pharmaceutical Co.), 27 January, 1999 (27.01.99), & WO 97/37969 A1 & US 6388122 B1 | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 November, 2002 (07.11.02) | 26 November, 2002 (26.11.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/07843

Continuation of A. CLASSIFICATION OF SUBJECT MATTER
(International Patent Classification (IPC))

Int.Cl$^7$ A61P27/14, 27/16, 29/00, 37/08, A61P43/00, C07D215/48, 217/02, C07D277/64, 333/62, 333/70, C07D409/12, A61K31/4436, C07D209/08, 209/42, A61K31/404, C07D333/54, 401/12, A61K31/4439, C07D333/58, 277/62, C07D277/68, 307/66, A61K31/341, C07D213/38, A61K31/44

(According to International Patent Classification (IPC) or to both national classification and IPC)

Continuation of B. FIELDS SEARCHED
Minimum Documentation Searched(International Patent Classification (IPC))

Int.Cl$^7$ A61P27/14, 27/16, 29/00, 37/08, A61P43/00, C07D215/48, 217/02, C07D277/64, 333/62, 333/70, C07D409/12, A61K31/4436, C07D209/08, 209/42, A61K31/404, C07D333/54, 401/12, A61K31/4439, C07D333/58, 277/62, C07D277/68, 307/66, A61K31/341, C07D213/38, A61K31/44

Minimum documentation searched (classification system followed by classification symbols)

Form PCT/ISA/210 (extra sheet) (July 1998)